(19) Europäisches Patentamt
European Patent Office
Office européen des brevets

(11) EP 1 275 713 A1

(12) **EUROPEAN PATENT APPLICATION**
published in accordance with Art. 158(3) EPC

(43) Date of publication:
**15.01.2003 Bulletin 2003/03**

(21) Application number: **01917790.6**

(22) Date of filing: **02.04.2001**

(51) Int Cl.[7]: **C12N 15/00**, C07K 7/08, C07K 14/705, C07K 16/28, A61K 45/00, A61P 31/04, A61K 38/02, A61K 39/395, A61P 43/00, G01N 33/15, G01N 33/50, G01N 33/53, G01N 33/577

(86) International application number:
**PCT/JP01/02869**

(87) International publication number:
**WO 01/072993 (04.10.2001 Gazette 2001/40)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **31.03.2000 JP 2000099617**
**22.11.2000 JP 2000356719**
**28.03.2001 US 806158**

(71) Applicant: **MOCHIDA PHARMACEUTICAL CO., LTD.**
**Shinjuku-ku Tokyo 160-8515 (JP)**

(72) Inventors:
- **FURUSAKO, Shoji, c/o Mochida Pharmaceut. Co.,Ltd Tokyo 160-8515 (JP)**
- **MORI, Sadao, c/o Mochida Pharmaceutical Co., Ltd Tokyo 160-8515 (JP)**
- **SHIRAKAWA, Kamon, c/o Mochida Pharmaceut. Co., Ltd Tokyo 160-8515 (JP)**
- **TAKAHASHI, Tomohiro, c/o Mochida Pharm. Co., Ltd Tokyo 160-8515 (JP)**

(74) Representative: **Grünecker, Kinkeldey, Stockmair & Schwanhäusser Anwaltssozietät**
**Maximilianstrasse 58**
**80538 München (DE)**

# (54) TLR/CD14 BINDING INHIBITOR

(57) The present invention provides an anti-CD14 antibody and fragments thereof that have a function of inhibiting the binding between CD14 and Toll Like Receptor (TLR). And also hybridomas producing the antibody or fragments thereof, peptides, method of preparing antibodies, CD14 mutant polypeptide, method of screening medicaments for treating sepsis and pharmaceutical compositions for sepsis were provided.

EP 1 275 713 A1

SEQUENCE LISTING

<110> MOCHIDA PHARMACEUTICAL CO., LTD.

<120> Anti-CD14 antibody

<130> MD0580

<140>

<141>

<160> 6

<170> PatentIn Ver. 2.1

<210> 1

<211> 356

<212> PRT

<213> human

<400> 1

Thr Thr Pro Glu Pro Cys Glu Leu Asp Asp Glu Asp Phe Arg Cys Val

1 5 10 15

Cys Asn Phe Ser Glu Pro Gln Pro Asp Trp Ser Glu Ala Phe Gln Cys

20 25 30

Val Ser Ala Val Glu Val Glu Ile His Ala Gly Gly Leu Asn Leu Glu

35 40 45

Pro Phe Leu Lys Arg Val Asp Ala Asp Ala Asp Pro Arg Gln Tyr Ala

50 55 60

Asp Thr Val Lys Ala Leu Arg Val Arg Arg Leu Thr Val Gly Ala Ala

65 70 75 80

Gln Val Pro Ala Gln Leu Leu Val Gly Ala Leu Arg Val Leu Ala Tyr

85 90 95

Ser Arg Leu Lys Glu Leu Thr Leu Glu Asp Leu Lys Ile Thr Gly Thr

100 105 110

Met Pro Pro Leu Pro Leu Glu Ala Thr Gly Leu Ala Leu Ser Ser Leu

115 120 125

Arg Leu Arg Asn Val Ser Trp Ala Thr Gly Arg Ser Trp Leu Ala Glu

130 135 140

Leu Gln Gln Trp Leu Lys Pro Gly Leu Lys Val Leu Ser Ile Ala Gln

145 150 155 160

Ala His Ser Pro Ala Phe Ser Cys Glu Gln Val Arg Ala Phe Pro Ala

165 170 175

Leu Thr Ser Leu Asp Leu Ser Asp Asn Pro Gly Leu Gly Glu Arg Gly

180 185 190

Leu Met Ala Ala Leu Cys Pro His Lys Phe Pro Ala Ile Gln Asn Leu

195 200 205

Ala Leu Arg Asn Thr Gly Met Glu Thr Pro Thr Gly Val Cys Ala Ala

210 215 220

Leu Ala Ala Ala Gly Val Gln Pro His Ser Leu Asp Leu Ser His Asn

225 230 235 240

Ser Leu Arg Ala Thr Val Asn Pro Ser Ala Pro Arg Cys Met Trp Ser

245 250 255

Ser Ala Leu Asn Ser Leu Asn Leu Ser Phe Ala Gly Leu Glu Gln Val

260 265 270

Pro Lys Gly Leu Pro Ala Lys Leu Arg Val Leu Asp Leu Ser Cys Asn

275 280 285

Arg Leu Asn Arg Ala Pro Gln Pro Asp Glu Leu Pro Glu Val Asp Asn

290 295 300

Leu Thr Leu Asp Gly Asn Pro Phe Leu Val Pro Gly Thr Ala Leu Pro

305 310 315 320

His Glu Gly Ser Met Asn Ser Gly Val Val Pro Ala Cys Ala Arg Ser

325 330 335

Thr Leu Ser Val Gly Val Ser Gly Thr Leu Val Leu Leu Gln Gly Ala

340 345 350

Arg Gly Phe Ala

355

<210> 2

<211> 47

<212> PRT

<213> human

<400> 2

Leu Glu Gln Val Pro Lys Gly Leu Pro Ala Lys Leu Arg Val Leu Asp
1 5 10 15

Leu Ser Cys Asn Arg Leu Asn Arg Ala Pro Gln Pro Asp Glu Leu Pro
20 25 30

Glu Val Asp Asn Leu Thr Leu Asp Gly Asn Pro Phe Leu Val Pro
35 40 45

<210> 3

<211> 1360

<212> DNA

<213> Human

<220>

<221> CDS

<222> (117)..(1244)

<400> 3

gtcgacgagt tcacaagtgt gaagcctgga agccggcggg tgccgctgtg taggaaagaa 60

gctaaagcac ttccagagcc tgtccggagc tcagaggttc ggaagactta tcgacc atg 119
Met
1

gag cgc gcg tcc tgc ttg ttg ctg ctg ctg ctg ccg ctg gtg cac gtc 167
Glu Arg Ala Ser Cys Leu Leu Leu Leu Leu Leu Pro Leu Val His Val

5 10 15

tct gcg acc acg cca gaa cct tgt gag ctg gac gat gaa gat ttc cgc 215

Ser Ala Thr Thr Pro Glu Pro Cys Glu Leu Asp Asp Glu Asp Phe Arg

20 25 30

tgc gtc tgc aac ttc tcc gaa cct cag ccc gac tgg tcc gaa gcc ttc 263

Cys Val Cys Asn Phe Ser Glu Pro Gln Pro Asp Trp Ser Glu Ala Phe

35 40 45

cag tgt gtg tct gca gta gag gtg gag atc cat gcc ggc ggt ctc aac 311

Gln Cys Val Ser Ala Val Glu Val Glu Ile His Ala Gly Gly Leu Asn

50 55 60 65

cta gag ccg ttt cta aag cgc gtc gat gcg gac gcc gac ccg cgg cag 359

Leu Glu Pro Phe Leu Lys Arg Val Asp Ala Asp Ala Asp Pro Arg Gln

70 75 80

tat gct gac acg gtc aag gct ctc cgc gtg cgg cgg ctc aca gtg gga 407

Tyr Ala Asp Thr Val Lys Ala Leu Arg Val Arg Arg Leu Thr Val Gly

85 90 95

gcc gca cag gtt cct gct cag cta ctg gta ggc gcc ctg cgt gtg cta 455

Ala Ala Gln Val Pro Ala Gln Leu Leu Val Gly Ala Leu Arg Val Leu

100 105 110

gcg tac tcc cgc ctc aag gaa ctg acg ctc gag gac cta aag ata acc 503

Ala Tyr Ser Arg Leu Lys Glu Leu Thr Leu Glu Asp Leu Lys Ile Thr

115 120 125

ggc acc atg cct ccg ctg cct ctg gaa gcc aca gga ctt gca ctt tcc 551

Gly Thr Met Pro Pro Leu Pro Leu Glu Ala Thr Gly Leu Ala Leu Ser

130 135 140 145

agc ttg cgc cta cgc aac gtg tcg tgg gcg aca ggg cgt tct tgg ctc 599

Ser Leu Arg Leu Arg Asn Val Ser Trp Ala Thr Gly Arg Ser Trp Leu

150 155 160

gcc gag ctg cag cag tgg ctc aag cca ggc ctc aag gta ctg agc att 647

Ala Glu Leu Gln Gln Trp Leu Lys Pro Gly Leu Lys Val Leu Ser Ile

165 170 175

gcc caa gca cac tcg cct gcc ttt tcc tgc gaa cag gtt cgc gcc ttc 695

Ala Gln Ala His Ser Pro Ala Phe Ser Cys Glu Gln Val Arg Ala Phe

180 185 190

ccg gcc ctt acc agc cta gac ctg tct gac aat cct gga ctg ggc gaa 743

Pro Ala Leu Thr Ser Leu Asp Leu Ser Asp Asn Pro Gly Leu Gly Glu

195 200 205

cgc gga ctg atg gcg gct ctc tgt ccc cac aag ttc ccg gcc atc cag 791

Arg Gly Leu Met Ala Ala Leu Cys Pro His Lys Phe Pro Ala Ile Gln

210 215 220 225

aat cta gcg ctg cgc aac aca gga atg gag acg ccc aca ggc gtg tgc 839

Asn Leu Ala Leu Arg Asn Thr Gly Met Glu Thr Pro Thr Gly Val Cys

230 235 240

gcc gca ctg gcg gcg gca ggt gtg cag ccc cac agc cta gac ctc agc 887

Ala Ala Leu Ala Ala Ala Gly Val Gln Pro His Ser Leu Asp Leu Ser

245 250 255

cac aac tcg ctg cgc gcc acc gta aac cct agc gct ccg aga tgc atg 935

His Asn Ser Leu Arg Ala Thr Val Asn Pro Ser Ala Pro Arg Cys Met

260 265 270

tgg tcc agc gcc ctg aac tcc ctc aat ctg tcg ttc gct ggg ctg gaa 983

Trp Ser Ser Ala Leu Asn Ser Leu Asn Leu Ser Phe Ala Gly Leu Glu

275 280 285

cag gtg cct aaa gga ctg cca gcc aag ctc aga gtg ctc gat ctc agc 1031

Gln Val Pro Lys Gly Leu Pro Ala Lys Leu Arg Val Leu Asp Leu Ser

290 295 300 305

tgc aac aga ctg aac agg gcg ccg cag cct gac gag ctg ccc gag gtg 1079

Cys Asn Arg Leu Asn Arg Ala Pro Gln Pro Asp Glu Leu Pro Glu Val

310 315 320

gat aac ctg aca ctg gac ggg aat ccc ttc ctg gtc cct gga act gcc 1127

Asp Asn Leu Thr Leu Asp Gly Asn Pro Phe Leu Val Pro Gly Thr Ala

325 330 335

ctc ccc cac gag ggc tca atg aac tcc ggc gtg gtc cca gcc tgt gca 1175

Leu Pro His Glu Gly Ser Met Asn Ser Gly Val Val Pro Ala Cys Ala

340 345 350

cgt tcg acc ctg tcg gtg ggg gtg tcg gga acc ctg gtg ctg ctc caa 1223

Arg Ser Thr Leu Ser Val Gly Val Ser Gly Thr Leu Val Leu Leu Gln

355 360 365

ggg gcc cgg ggc ttt gcc taa gatccaagac agaataatga atggactcaa 1274

Gly Ala Arg Gly Phe Ala

370 375

actgccttgg cttcagggga gtcccgtcag gacgttgagg acttttcgac caattcaacc 1334

ctttgcccca cctttattaa gcatgc 1360

<210> 4

<211> 855

<212> DNA

<213> Artificial Sequence

<400> 4

ACC ACG CCA GAA CCT TGT GAG CTG GAC GAT GAA GAT TTC CGC TGC GTC 48

Thr Thr Pro Glu Pro Cys Glu Leu Asp Asp Glu Asp Phe Arg Cys Val

1 5 10 15

TGC AAC TTC TCC GAA CCT CAG CCC GAC TGG TCC GAA GCC TTC CAG TGT 96

Cys Asn Phe Ser Glu Pro Gln Pro Asp Trp Ser Glu Ala Phe Gln Cys

20 25 30

GTG TCT GCA GTA GAG GTG GAG ATC CAT GCC GGC GGT CTC AAC CTA GAG 144

Val Ser Ala Val Glu Val Glu Ile His Ala Gly Gly Leu Asn Leu Glu

35 40 45

CCG TTT CTA AAG CGC GTC GAT GCG GAC GCC GAC CCG CGG CAG TAT GCT 192

Pro Phe Leu Lys Arg Val Asp Ala Asp Ala Asp Pro Arg Gln Tyr Ala

50 55 60

GAC ACG GTC AAG GCT CTC CGC GTG CGG CGG CTC ACA GTG GGA GCC GCA 240

Asp Thr Val Lys Ala Leu Arg Val Arg Arg Leu Thr Val Gly Ala Ala

65 70 75 80

CAG GTT CCT GCT CAG CTA CTG GTA GGC GCC CTG CGT GTG CTA GCG TAC 288

Gln Val Pro Ala Gln Leu Leu Val Gly Ala Leu Arg Val Leu Ala Tyr

85 90 95

TCC CGC CTC AAG GAA CTG ACG CTC GAG GAC CTA AAG ATA ACC GGC ACC 336

Ser Arg Leu Lys Glu Leu Thr Leu Glu Asp Leu Lys Ile Thr Gly Thr

100 105 110

ATG CCT CCG CTG CCT CTG GAA GCC ACA GGA CTT GCA CTT TCC AGC TTG 384

Met Pro Pro Leu Pro Leu Glu Ala Thr Gly Leu Ala Leu Ser Ser Leu

115 120 125

CGC CTA CGC AAC GTG TCG TGG GCG ACA GGG CGT TCT TGG CTC GCC GAG 432

Arg Leu Arg Asn Val Ser Trp Ala Thr Gly Arg Ser Trp Leu Ala Glu

130 135 140

CTG CAG CAG TGG CTC AAG CCA GGC CTC AAG GTA CTG AGC ATT GCC CAA 480

Leu Gln Gln Trp Leu Lys Pro Gly Leu Lys Val Leu Ser Ile Ala Gln

145 150 155 160

GCA CAC TCG CCT GCC TTT TCC TGC GAA CAG GTT CGC GCC TTC CCG GCC 528

Ala His Ser Pro Ala Phe Ser Cys Glu Gln Val Arg Ala Phe Pro Ala

165 170 175

CTT ACC AGC CTA GAC CTG TCT GAC AAT CCT GGA CTG GGC GAA CGC GGA 576

Leu Thr Ser Leu Asp Leu Ser Asp Asn Pro Gly Leu Gly Glu Arg Gly

180 185 190

CTG ATG GCG GCT CTC TGT CCC CAC AAG TTC CCG GCC ATC CAG AAT CTA 624

Leu Met Ala Ala Leu Cys Pro His Lys Phe Pro Ala Ile Gln Asn Leu

195 200 205

GCG CTG CGC AAC ACA GGA ATG GAG ACG CCC ACA GGC GTG TGC GCC GCA 672

Ala Leu Arg Asn Thr Gly Met Glu Thr Pro Thr Gly Val Cys Ala Ala

210 215 220

CTG GCG GCG GCA GGT GTG CAG CCC CAC AGC CTA GAC CTC AGC CAC AAC 720

Leu Ala Ala Ala Gly Val Gln Pro His Ser Leu Asp Leu Ser His Asn

225 230 235 240

TCG CTG CGC GCC ACC GTA AAC CCT AGC GCT CCG AGA TGC ATG TGG TCC 768

Ser Leu Arg Ala Thr Val Asn Pro Ser Ala Pro Arg Cys Met Trp Ser

245 250 255

AGC GCC CTG AAC TCC CTC AAT CTG TCG TTC GCT GGG CTG GAA CAG GTG 816

Ser Ala Leu Asn Ser Leu Asn Leu Ser Phe Ala Gly Leu Glu Gln Val

260 265 270

CCT AAA GGA CTG CCA GCC AAG CTC AGA GTG CTC GAT CTC 855

Pro Lys Gly Leu Pro Ala Lys Leu Arg Val Leu Asp Leu

275 280 285

<210> 5

<211> 921

<212> DNA

<213> Artificial Sequence

<400> 5

ACC ACG CCA GAA CCT TGT GAG CTG GAC GAT GAA GAT TTC CGC TGC GTC 48

Thr Thr Pro Glu Pro Cys Glu Leu Asp Asp Glu Asp Phe Arg Cys Val

1 5 10 15

TGC AAC TTC TCC GAA CCT CAG CCC GAC TGG TCC GAA GCC TTC CAG TGT 96

Cys Asn Phe Ser Glu Pro Gln Pro Asp Trp Ser Glu Ala Phe Gln Cys

20 25 30

GTG TCT GCA GTA GAG GTG GAG ATC CAT GCC GGC GGT CTC AAC CTA GAG 144

Val Ser Ala Val Glu Val Glu Ile His Ala Gly Gly Leu Asn Leu Glu

35 40 45

CCG TTT CTA AAG CGC GTC GAT GCG GAC GCC GAC CCG CGG CAG TAT GCT 192

Pro Phe Leu Lys Arg Val Asp Ala Asp Ala Asp Pro Arg Gln Tyr Ala

50 55 60

GAC ACG GTC AAG GCT CTC CGC GTG CGG CGG CTC ACA GTG GGA GCC GCA 240

Asp Thr Val Lys Ala Leu Arg Val Arg Arg Leu Thr Val Gly Ala Ala

65 70 75 80

CAG GTT CCT GCT CAG CTA CTG GTA GGC GCC CTG CGT GTG CTA GCG TAC 288

Gln Val Pro Ala Gln Leu Leu Val Gly Ala Leu Arg Val Leu Ala Tyr

85 90 95

TCC CGC CTC AAG GAA CTG ACG CTC GAG GAC CTA AAG ATA ACC GGC ACC 336

Ser Arg Leu Lys Glu Leu Thr Leu Glu Asp Leu Lys Ile Thr Gly Thr

100 105 110

ATG CCT CCG CTG CCT CTG GAA GCC ACA GGA CTT GCA CTT TCC AGC TTG 384

Met Pro Pro Leu Pro Leu Glu Ala Thr Gly Leu Ala Leu Ser Ser Leu

115 120 125

CGC CTA CGC AAC GTG TCG TGG GCG ACA GGG CGT TCT TGG CTC GCC GAG 432

Arg Leu Arg Asn Val Ser Trp Ala Thr Gly Arg Ser Trp Leu Ala Glu

130 135 140

CTG CAG CAG TGG CTC AAG CCA GGC CTC AAG GTA CTG AGC ATT GCC CAA 480

Leu Gln Gln Trp Leu Lys Pro Gly Leu Lys Val Leu Ser Ile Ala Gln

145 150 155 160

GCA CAC TCG CCT GCC TTT TCC TGC GAA CAG GTT CGC GCC TTC CCG GCC 528

Ala His Ser Pro Ala Phe Ser Cys Glu Gln Val Arg Ala Phe Pro Ala

165 170 175

CTT ACC AGC CTA GAC CTG TCT GAC AAT CCT GGA CTG GGC GAA CGC GGA 576

Leu Thr Ser Leu Asp Leu Ser Asp Asn Pro Gly Leu Gly Glu Arg Gly

180 185 190

CTG ATG GCG GCT CTC TGT CCC CAC AAG TTC CCG GCC ATC CAG AAT CTA 624

Leu Met Ala Ala Leu Cys Pro His Lys Phe Pro Ala Ile Gln Asn Leu

```
        195                 200                 205
GCG CTG CGC AAC ACA GGA ATG GAG ACG CCC ACA GGC GTG TGC GCC GCA   672
Ala Leu Arg Asn Thr Gly Met Glu Thr Pro Thr Gly Val Cys Ala Ala
    210                 215                 220
CTG GCG GCG GCA GGT GTG CAG CCC CAC AGC CTA GAC CTC AGC CAC AAC   720
Leu Ala Ala Ala Gly Val Gln Pro His Ser Leu Asp Leu Ser His Asn
225                 230                 235                 240
TCG CTG CGC GCC ACC GTA AAC CCT AGC GCT CCG AGA TGC ATG TGG TCC   768
Ser Leu Arg Ala Thr Val Asn Pro Ser Ala Pro Arg Cys Met Trp Ser
                245                 250                 255
AGC GCC CTG AAC TCC CTC AAT CTG TCG TTC GCT GGG CTG GAA CAG GTG   816
Ser Ala Leu Asn Ser Leu Asn Leu Ser Phe Ala Gly Leu Glu Gln Val
            260                 265                 270
CCT AAA GGA CTG CCA GCC AAG CTC AGA GTG CTC GAT CTC GCT TGC AAC   864
Pro Lys Gly Leu Pro Ala Lys Leu Arg Val Leu Asp Leu Ala Cys Asn
        275                 280                 285
AGA CTG AAC AGG GCG CCG CAG CCT GAC GAG CTG CCC GAG GTG GAT AAC   912
Arg Leu Asn Arg Ala Pro Gln Pro Asp Glu Leu Pro Glu Val Asp Asn
    290                 295                 300
CTG ACA CTG   921
Leu Thr Leu
```

<210> 6

<211> 921

<212> DNA

<213> Artificial Sequence

<400> 6

ACC ACG CCA GAA CCT TGT GAG CTG GAC GAT GAA GAT TTC CGC TGC GTC 48
Thr Thr Pro Glu Pro Cys Glu Leu Asp Asp Glu Asp Phe Arg Cys Val
1 5 10 15

TGC AAC TTC TCC GAA CCT CAG CCC GAC TGG TCC GAA GCC TTC CAG TGT 96
Cys Asn Phe Ser Glu Pro Gln Pro Asp Trp Ser Glu Ala Phe Gln Cys
20 25 30

GTG TCT GCA GTA GAG GTG GAG ATC CAT GCC GGC GGT CTC AAC CTA GAG 144
Val Ser Ala Val Glu Val Glu Ile His Ala Gly Gly Leu Asn Leu Glu
35 40 45

CCG TTT CTA AAG CGC GTC GAT GCG GAC GCC GAC CCG CGG CAG TAT GCT 192
Pro Phe Leu Lys Arg Val Asp Ala Asp Ala Asp Pro Arg Gln Tyr Ala
50 55 60

GAC ACG GTC AAG GCT CTC CGC GTG CGG CGG CTC ACA GTG GGA GCC GCA 240
Asp Thr Val Lys Ala Leu Arg Val Arg Arg Leu Thr Val Gly Ala Ala
65 70 75 80

CAG GTT CCT GCT CAG CTA CTG GTA GGC GCC CTG CGT GTG CTA GCG TAC 288
Gln Val Pro Ala Gln Leu Leu Val Gly Ala Leu Arg Val Leu Ala Tyr

85 90 95

TCC CGC CTC AAG GAA CTG ACG CTC GAG GAC CTA AAG ATA ACC GGC ACC 336

Ser Arg Leu Lys Glu Leu Thr Leu Glu Asp Leu Lys Ile Thr Gly Thr

100 105 110

ATG CCT CCG CTG CCT CTG GAA GCC ACA GGA CTT GCA CTT TCC AGC TTG 384

Met Pro Pro Leu Pro Leu Glu Ala Thr Gly Leu Ala Leu Ser Ser Leu

115 120 125

CGC CTA CGC AAC GTG TCG TGG GCG ACA GGG CGT TCT TGG CTC GCC GAG 432

Arg Leu Arg Asn Val Ser Trp Ala Thr Gly Arg Ser Trp Leu Ala Glu

130 135 140

CTG CAG CAG TGG CTC AAG CCA GGC CTC AAG GTA CTG AGC ATT GCC CAA 480

Leu Gln Gln Trp Leu Lys Pro Gly Leu Lys Val Leu Ser Ile Ala Gln

145 150 155 160

GCA CAC TCG CCT GCC TTT TCC TGC GAA CAG GTT CGC GCC TTC CCG GCC 528

Ala His Ser Pro Ala Phe Ser Cys Glu Gln Val Arg Ala Phe Pro Ala

165 170 175

CTT ACC AGC CTA GAC CTG TCT GAC AAT CCT GGA CTG GGC GAA CGC GGA 576

Leu Thr Ser Leu Asp Leu Ser Asp Asn Pro Gly Leu Gly Glu Arg Gly

180 185 190

CTG ATG GCG GCT CTC TGT CCC CAC AAG TTC CCG GCC ATC CAG AAT CTA 624

Leu Met Ala Ala Leu Cys Pro His Lys Phe Pro Ala Ile Gln Asn Leu

195 200 205

GCG CTG CGC AAC ACA GGA ATG GAG ACG CCC ACA GGC GTG TGC GCC GCA 672

Ala Leu Arg Asn Thr Gly Met Glu Thr Pro Thr Gly Val Cys Ala Ala

210 215 220

CTG GCG GCG GCA GGT GTG CAG CCC CAC AGC CTA GAC CTC AGC CAC AAC 720

Leu Ala Ala Ala Gly Val Gln Pro His Ser Leu Asp Leu Ser His Asn

225 230 235 240

TCG CTG CGC GCC ACC GTA AAC CCT AGC GCT CCG AGA TGC ATG TGG TCC 768

Ser Leu Arg Ala Thr Val Asn Pro Ser Ala Pro Arg Cys Met Trp Ser

245 250 255

AGC GCC CTG AAC TCC CTC AAT CTG TCG TTC GCT GGG CTG GAA CAG GTG 816

Ser Ala Leu Asn Ser Leu Asn Leu Ser Phe Ala Gly Leu Glu Gln Val

260 265 270

CCT AAA GGA CTG CCA GCC AAG CTC AGA GTG CTC GAT CTC TGT TGC AAC 864

Pro Lys Gly Leu Pro Ala Lys Leu Arg Val Leu Asp Leu Cys Cys Asn

275 280 285

AGA CTG AAC AGG GCG CCG CAG CCT GAC GAG CTG CCC GAG GTG GAT AAC 912

Arg Leu Asn Arg Ala Pro Gln Pro Asp Glu Leu Pro Glu Val Asp Asn

290 295 300

CTG ACA CTG 921

Leu Thr Leu

305

<210> 7

<211> 20

<212> DNA

<213> Artificial Sequence

<220>

<221> sense primer 1

<400> 7

tcgaggaaga gaagacacca

<210> 8

<211> 20

<212> DNA

<213> Artificial Sequence

<220>

<221> sense primer 2

<400> 8

cccatccaga gtttagccct

<210> 9

<211> 24

<212> DNA

<213> Artificial Sequence

<220>

<221> antisense primer 1

<400> 9

ccatccgaaa ttataagaaa agtc

<210> 10

<211> 29

<212> DNA

<213> Artificial Sequence

<220>

<221> antisense primer 2

<400> 10

cccaagcttt ggaattactc acccttagc

<210> 11

<211> 24

<212> PRT

<213> human

<400> 11

Leu Asp Leu Ser Cys Asn Arg Leu Asn Arg Ala Pro Gln Pro Asp Glu

1 5 10 15

Leu Pro Glu Val Asp Asn Leu Thr

20

<210> 12

<211> 18

<212> PRT

<213> human

<400> 12

Glu Val Asp Asn Leu Thr Leu Asp Gly Asn Pro Phe Leu Val Pro Gly

1 5 10 15

Thr Ala

<210> 13

<211> 12

<212> PRT

<213> human

<400> 13

Thr Ala Leu Pro His Glu Gly Ser Met Asn Ser Gly

1 5 10

<210> 14

<211> 15

<212> PRT

<213> human

<400> 14

Pro Ala Cys Ala Arg Ser Thr Leu Ser Val Gly Val Ser Gly Thr
1 5 10 15

<210> 15

<211> 15

<212> PRT

<213> Artificial Sequence

<400> 15

Gln Gly Pro Cys Arg Ala Phe Ile Lys Leu Trp Ala Phe Asp Cys
1 5 10 15

<210> 16

<211> 20

<212> DNA

<213> Artificial Sequence

<220>

<221> sense primer 3

<400> 16

ctctggctaa ctagagaacc

<210> 17

<211> 21

<212> DNA

<213> Artificial Sequence

<220>

<221> antisense primer 3

<400> 17

gcggaaatca caaggttctg g

<210> 18

<211> 21

<212> DNA

<213> Artificial Sequence

<220>

<221> sense primer 4

<400> 18

gaaccttgtg atttccgctg c

<210> 19

<211> 20

<212> DNA

<213> Artificial Sequence

<220>

<221> antisense primer 4

<400> 19

ttattaggaa aggacagtgg

<210> 20

<211> 30

<212> DNA

<213> Artificial Sequence

<220>

<221> antisense primer 5

<400> 20

agccttgacc gtgtccgcat cgacgcgctt

<210> 21

<211> 30

<212> DNA

<213> Artificial Sequence

<220>

<221> sense primer 5

<400> 21

aagcgcgtcg atgcggacac ggtcaaggct

<210> 22

<211> 39

<212> DNA

<213> Artificial Sequence

<220>

<221> antisense primer 6

<400> 22

gttgtgagac aggtctaggc tggtaagggc cgggaaggc

<210> 23

<211> 39

<212> DNA

<213> Artificial Sequence

<220>

<221> sense primer 6

<400> 23

gcccttacca gcctagacct gtctcacaac tcgctgcgc

<210> 24

<211> 39

<212> DNA

<213> Artificial Sequence

<220>

<221> antisense primer 7

<400> 24

cagtctgttg caagacaggt ctaggctgtg gggctgcac

<210> 25

<211> 39

<212> DNA

<213> Artificial Sequence

<220>

<221> sense primer 7

<400> 25

cacagcctag acctgtcttg caacagactg aacagggcg

<210> 26

<211> 39

<212> DNA

<213> Artificial Sequence

<220>

<221> antisense primer 8

<400> 26

cagtctgttg caagacaggt ctaggctggt aagggccgg

<210> 27

<211> 39

<212> DNA

<213> Artificial Sequence

<220>

<221> sense primer 8

<400> 27

accagcctag acctgtcttg caacagactg aacagggcg

<210> 28

<211> 20

<212> DNA

<213> Artificial Sequence

<220>

<221> sense primer 9

<400> 28

cacgccagaa ccttgtgagc

<210> 29

<211> 38

<212> DNA

<213> Artificial Sequence

<220>

<221> antisense primer 9

<400> 29

gtcagtgcac aggctgggac cacaacggat tgcattga

<210> 30

<211> 30

<212> DNA

<213> Artificial Sequence

<220>

<221> antisense primer 10

<400> 30

cccaagcttc tattacagtg tcaggttatc

<210> 31

<211> 30

<212> DNA

<213> Artificial Sequence

<220>

<221> antisense primer 11

<400> 31

cccaagcttc tattagggca gctcgtcagg

<210> 32

<211> 30

<212> DNA

<213> Artificial Sequence

<220>

<221> antisense primer 12

<400> 32

cccaagcttc tattactgcg gcgccctgtt

<210> 33

<211> 30

<212> DNA

<213> Artificial Sequence

<220>

<221> antisense primer 13

<400> 33

cccaagcttc tattacagtc tgttgcagct

<210> 34

<211> 30

<212> DNA

<213> Artificial Sequence

<220>

<221> antisense primer 14

<400> 34

cccaagcttc tattagagat cgagcactct

<210> 35

<211> 30

<212> DNA

<213> Artificial Sequence

<220>

<221> antisense primer 15

<400> 35

cccaagcttc tattatacgg tggcgcgcag

<210> 36

<211> 30

<212> DNA

<213> Artificial Sequence

<220>

<221> antisense primer 16

<400> 36

cccaagcttc tattaagaca ggtctaggct

<210> 37

<211> 30

<212> DNA

<213> Artificial Sequence

<220>

<221> antisense primer 17

<400> 37

cccaagcttc tattagcctg gcttgagcca

<210> 38

<211> 23

<212> DNA

<213> Artificial Sequence

<220>

<221> antisense primer 18

<400> 38

actctgagag cggctggcag tcc

<210> 39

<211> 23

<212> DNA

<213> Artificial Sequence

<220>

<221> sense primer 10

<400> 39

tgccagccgc tctcagagtg ctc

<210> 40

<211> 23

<212> DNA

<213> Artificial Sequence

<220>

<221> antisense primer 19

<400> 40

agatcgagag ctctgagctt ggc

<210> 41

<211> 20

<212> DNA

<213> Artificial Sequence

<220>

<221> antisense primer 20

<400> 41

gagatcagcc actctgagct

<210> 42

<211> 20

<212> DNA

<213> Artificial Sequence

<220>

<221> antisense primer 21

<400> 42

gctgagagcg agcactctga

<210> 43

<211> 20

<212> DNA

<213> Artificial Sequence

<220>

<221> antisense primer 22

<400> 43

gcagctagca tcgagcactc

<210> 44

<211> 20

<212> DNA

<213> Artificial Sequence

<220>

<221> antisense primer 23

<400> 44

gttgcaagcg agatcgagca

<210> 45

<211> 20

<212> DNA

<213> Artificial Sequence

<220>

<221> antisense primer 24

<400> 45

tctgttagcg ctgagatcga

<210> 46

<211> 20

<212> DNA

<213> Artificial Sequence

<220>

<221> antisense primer 25

<400> 46

gttcagagcg ttgcagctga

<210> 47

<211> 21

<212> DNA

<213> Artificial Sequence

<220>

<221> antisense primer 26

<400> 47

tcaggctgcg ccgccctgtt c

<210> 48

<211> 21

<212> DNA

<213> Artificial Sequence

<220>

<221> antisense primer 27

<400> 48

agctcgtcag cctgcggcgc c

<210> 49

<211> 21

<212> DNA

<213> Artificial Sequence

<220>

<221> antisense primer 28

<400> 49

tcgtcagcct gcgccgccct g

<210> 50

<211> 21

<212> DNA

<213> Artificial Sequence

<220>

<221> antisense primer 29

<400> 50

tccacctcgg ccagctcgtc a

<210> 51

<211> 23

<212> DNA

<213> Artificial Sequence

<220>

<221> sense primer 11

<400> 51

agctcagagc tctcgatctc agc

<210> 52

<211> 20

<212> DNA

<213> Artificial Sequence

<220>

<221> sense primer 12

<400> 52

agagtggctg atctcagctg

<210> 53

<211> 20

<212> DNA

<213> Artificial Sequence

<220>

<221> sense primer 13

<400> 53

gtgctcgctc tcagctgcaa

<210> 54

<211> 20

<212> DNA

<213> Artificial Sequence

<220>

<221> sense primer 14

<400> 54

ctcgatgcta gctgcaacag

<210> 55

<211> 20

<212> DNA

<213> Artificial Sequence

<220>

<221> sense primer 15

<400> 55

gatctcgctt gcaacagact

<210> 56

<211> 20

<212> DNA

<213> Artificial Sequence

<220>

<221> sense primer 16

<400> 56

ctcagcgcta acagactgaa

<210> 57

<211> 20

<212> DNA

<213> Artificial Sequence

<220>

<221> sense primer 17

<400> 57

tgcaacgctc tgaacagggc

<210> 58

<211> 21

<212> DNA

<213> Artificial Sequence

<220>

<221> sense primer 18

<400> 58

gaacagggcg gcgcagcctg a

<210> 59

<211> 21

<212> DNA

<213> Artificial Sequence

<220>

<221> sense primer 19

<400> 59

ggcgccgcag gctgacgagc t

<210> 60

<211> 21

<212> DNA

<213> Artificial Sequence

<220>

<221> sense primer 20

<400> 60

cagggcggcg caggctgacg a

<210> 61

<211> 21

<212> DNA

<213> Artificial Sequence

<220>

<221> sense primer 21

<400> 61

tgacgagctg gccgaggtgg a

<210> 62

<211> 20

<212> DNA

<213> Artificial Sequence

<220>

<221> sense primer 22

<400> 62

tgcaacgatc tgaacagggc

<210> 63

<211> 20

<212> DNA

<213> Artificial Sequence

<220>

<221> antisense primer 30

<400> 63

gttcagatcg ttgcagctga

<210> 64

<211> 20

<212> DNA

<213> Artificial Sequence

<220>

<221> sense primer 23

<400> 64

ctcgatctct gttgcaacag

<210> 65

<211> 20

<212> DNA

<213> Artificial Sequence

<220>

<221> antisense primer 31

<400> 65

tctgttgcaa cagagatcga

<210> 66

<211> 20

<212> DNA

<213> Artificial Sequence

<220>

<221> sense primer 24

<400> 66

ctcgatctcg gttgcaacag

<210> 67

<211> 20

<212> DNA

<213> Artificial Sequence

<220>

<221> antisense primer 32

<400> 67

tctgttgcaa ccgagatcga

<210> 68

<211> 20

<212> DNA

<213> Artificial Sequence

<220>

<221> sense primer 25

<400> 68

ctcgatctca cttgcaacag

<210> 69

<211> 20

<212> DNA

<213> Artificial Sequence

<220>

<221> antisense primer 33

<400> 69

tctgttgcaa gtgagatcga

<210> 70

<211> 20

<212> DNA

<213> Artificial Sequence

<220>

<221> sense primer 26

<400> 70

ctcgatctcc tttgcaacag

<210> 71

<211> 20

<212> DNA

<213> Artificial Sequence

<220>

<221> antisense primer 34

<400> 71

tctgttgcaa aggagatcga

<210> 72

<211> 21

<212> DNA

<213> Artificial Sequence

<220>

<221> sense primer27

<400> 72

accacgccag aaccttgtga g

<210> 73

<211> 22

<212> DNA

<213> Artificial Sequence

<220>

<221> antisense primer35

<400> 73

ctgaaaatct tctctcatcc gc

## Description

TECHNICAL FIELD

The present invention relates to an anti-CD14 antibody and fragment thereof having a function of inhibiting binding of CD14 and Toll Like Receptor (hereinafter, referred to as "TLR"), to a hybridoma producing the antibody or fragments thereof, to a peptide, to a method of preparing the antibody, to a CD14 mutant polypeptide, to a method of screening a medicament for treating sepsis, and to a pharmaceutical composition for sepsis.

BACKGROUND ART

CD14 is a glycoprotein composed of 356 amino acids and exists on membranes of macrophages, monocytes, Kupffer cells, neutrophiles, and partly B cells as anchored thereto through glycosylphosphatidylinositol (GPI).

Human CD14 includes besides membrane-bound type CD14 (hereinafter, also referred to as "mCD14"), soluble type CD14 (hereinafter, also referred to as "sCD14" or "soluble CD14"). Furthermore, it has been reported that in blood there are a plurality of sCD14s having different'molecular weights (Labeta MO: Eur. J. Immunol., 23:2144, 1993).

Human CD14 is known as an LPS receptor for endotoxins of gram-negative bacilli (Wright et al.: Science, 249:1431, 1990), which receives LPS from LBP (LPS binding protein) in blood to form a complex.

Macrophage and the like that express mCD14 are activated by a complex of LPS and sCD14 to induce production of inflammatory cytokines (Hailmann E, et al.: J. Immnol., 156:4384, 1996).

In vascular endothelial cells and vascular smooth muscle cells that do not express mCD14, production of inflammatory cytokines is induced by a complex of sCD14 and LPS (hereinafter, also referred to as "sCD14/LPS") (Loppnow H, et al.: Infection & Immunity, 63:1020, 1995).

In addition, it reacts with bacterial cell components not only of gram-negative bacteria but also of gram-positive bacteria and with mycobacteria, and it has functions as a receptor for such as lipoteichoic acid (LTA) and peptide glycans (PepG), and reaction with above induces production of inflammatory cytokines of cells (Cleveland MG, et al.: Infect immunity, 64:1906, 1996).

Production of cytokines in cells through CD14 such as LPS or LTA exerts harmful effects on a living body and causes sepsis. Generally, in an early stage of sepsis, such symptoms as chill, hidrosis, fever, and hyposthenia are observed, and subsequently serious clinical symptoms that involve a shock are caused such as hypotension, neutropenia, disseminated intravascular coagulation syndrome, adult respiration distress syndrome, respiratory insufficiency, and multiple organ insufficiency.

As for the function of human CD14 to transduce the signal of LPS to cells, a part of the functioning region has been elucidated. The positions from 1 to 152 of the N-terminal are a region essential for expressing the function of CD14 (Juan TS, et al.: J. Biol. Chem., 270:1382, 1995) and the positions from 7 to 14 and the positions from 57 to 64 are portions essential for binding to LPS (Juan TS: J. Biol. Chem., 270, 29:17237 (1995) and Juan TS: J. Biol. Chem., 270, 10:5219 (1995)).

However, nothing has been elucidated on the function that regions from 153 to C-terminal of human CD14.

In addition, the participation of toll like receptor (TLR) in the signal transduction of CD14/LPS to cells has been studied in recent years. To date in total 10 kinds of TLR family genes composed of human TLR1, TLR2, TLR3, TLR4 and TLR5 (Fernand R: Proc. Natl. Acad. Sci. USA, 95:588, 1998), TLR6 (Takeuchi O: Gene, 231:59, 1999), TLR7, TLR8 and TLR9 and pseudogene (Chuang TH: Eur. Cytokine Netw., 11:372, 2000) have been cloned.

Based on studies on TLR2- or TLR4-deficient. mice, a possibility that TLR4 is required for signal transduction of cell components of gram-negative bacteria into cells and TLR2 is required for signal transduction of cell components of gram-positive bacteria into cells has been reported (Takeuchi O, et al.: Immunity, 11:443, 1999). Further, it has been reported that TLR4. participates in signal transduction of LPS into cells, TLR4 directly reacts with LPS on cell surfaces, and the reaction is enhanced in the presence of CD14 (Ruey-Bing Y: Nature, 395:284, 1998). Furthermore, recently MD2 that binds to TLR4 has been found (Rintaro Shimazu: J. Exp. Med., 189, 1777, 1999). It has been reported that in mouse cells, TLR4 performs LPS signal transduction only in the presence of MD2, which is a secretory protein composed of 160 amino acids. Although, exact role of MD2 in the LPS signal transduction remains to be clarified, it may contribute to stabilization of the structure of TLR4 or dimer thereof (Alan Aderem, Nature, 406, 782, 2000). It has also been reported that the antibody that recognizes the complex of mouse TLR4 and MD2 specifically suppresses production of TNF-$\alpha$ and nitrogen monoxide by LPS (Akasi S., J. Immunol, 164, 3471, 1999).

Also in mice, soluble TLR4, which is a splice variant of TLR4, has been reported. The soluble TLR4, which is composed of 86 amino acid residues, has been reported to exhibit an activity of suppressing activation of cells by LPS (Iwami KI, J. Immunol., 165, 6682, 2000).

However, in the mutual action between CD14 and TLR, it has not been clarified if CD14 directly binds to TLR or a complex of TLR with accessory molecule, which is a low molecular substance whose function has not yet clarified.

Further, the binding region of TLR and CD14 is quite unknown.

As the human anti-CD14 antibody that controls signal transduction of LPS through human CD14, there have been known 3C10 antibody that binds to 7th to 14th amino acids of human CD14 (Steinman: J. Exp. Med., 158: 126 (1983) and Juan TS: J. Biol. Chem., 270:29, 17237 (1995)), and MEM-18 antibody that binds to 57th to 64th amino acids of CD14 (Bazil: Eur. J. Immunol., 16:1583 (1986) and Juan TS: J. Biol. Chem., 270, 10, 5219 (1995)) are known and their application to medicaments for treating sepsis is disclosed.

Furthermore, 28C5 antibody and 23G4 antibody that inhibits binding of LPS and suppresses release of cytokines as well as 18E12 antibody that inhibits binding of LPS only partly and suppresses release cytokines have been disclosed (JP 8-510909 A).

Also, anti-CD14 antibody that is against the action of gram-positive bacteria and mycobacteria has been disclosed (JP 10-505839 A).

However, when used as a medicament for treating sepsis, an antibody that recognizes the binding region of LPS or an antibody that suppresses the binding of LPS is not expected to have an effect of suppressing signal transduction of already formed LPS/CD14. Further, it is not expected to have an effect of suppressing signal transduction by cell components of gram-positive bacteria, mycoplasma or the like, since the binding of LPS to CD14 is specifically inhibited. Further, the 18E12 antibody is not clarified with respect to the recognition site of CD14 and the mechanism of suppressing release of cytokines is also unclear.

As an anti-inflammatory peptide focused on the binding site between human CD14 and LPS, an anti-inflammatory peptides based on amino acids at positions 7 to 10, 11 to 14, and 57 to 64, respectively, of CD14 from its N-terminal have been disclosed (JP 10-511954). These peptides have bindability to LPS and remove LPS but do not suppress signal transduction of already formed CD14/LPS into cells.

Further, as an anti-inflammatory peptide having a modified binding site between human CD14 and LPS, an anti-inflammatory polypeptide including a soluble CD14-related polypeptide that has a different amino acid sequence than that of natural sequence at positions from 7 to 10 of CD14 from the N-terminal or that has no amino acids at positions 1 to 14 of CD14 from the N-terminal thereof has been disclosed (JP 10-512142 A). These peptides have a modified binding site to LPS so that they suppress transduction of signal of LPS into cells. However, they do not suppress signal transduction of already formed CD14/LPS into cells.

That is, conventionally, what suppresses the function of CD14 includes substances that inhibit binding of a specified bacterial cell component to CD14. These substances are difficult to inhibit binding to other bacterial cell components and it is not expectable that they have the effect of suppressing signal transduction of already formed CD14/LPS.

As described above, participation of TLR in signal transduction of CD14/LPS into cells has been known. However, it is not certain as to whether or not the substance that inhibits this participation controls the signal transduction and no such inhibitory substance has been known.

An object of the present invention is to provide a substance having a function of inhibiting the binding between CD14 and TLR, which can even control the signal transduction of already formed CD14/LPS into cells, a method of preparing the same, a method of screening a medicament for treating sepsis, and a pharmaceutical composition for sepsis.

## DISCLOSURE OF THE INVENTION

The inventors of the present invention have made extensive studies in order to overcome the conventional problems as described above and provide an anti-CD14 antibody that inhibits the binding between CD14 and TLR. Also, by specifying in human CD14 a recognition region that is necessary for an anti-CD14 antibody to perform that action, they provide a method of preparing an antibody by using such a specified region.

Furthermore, they provide a CD14 mutant polypeptide that inhibits the binding between CD14 and TLR. Also they provide a method of preparing the CD14 mutant polypeptide.

Also, they provide a method of screening novel medicaments for treating sepsis by performing contact of CD14 with a test substance in the presence of TLR and a pharmaceutical composition for sepsis containing as an active ingredient a substance that inhibits the binding between CD14 and TLR.

That is, the present invention provides anti-CD14 antibodies as described in (1) to (11) below.

(1) An anti-CD14 antibody, which specifically recognizes an epitope comprising a part of the region from 269th to 315th amino acids of human CD14 described in SEQ ID NO:1 and has a function of inhibiting the binding between CD14 and Toll Like Receptor (TLR).

(2) An antibody according to (1), which is a monoclonal antibody.

(3) Fragment of an antibody according to (2) above, from Fab, Fab' or $(Fab')_2$.

(4) F1024-1-3 Monoclonal antibody produced by hybridoma F1024-1-3 (Accession No. P-18061).

(5) A hybridoma producing an antibody or fragment of antibody according to any one of (2) to (4) above.
(6) A peptide comprising 8 or more consecutive amino acids out of the region from 269th to 315th amino acids of human CD14 described in SEQ ID NO:1.
(7) A method of preparing an antibody, comprising using a peptide according to (6) above as an immunogen.
(8) A CD14 mutant polypeptide having a function of inhibiting the binding between CD14 and TLR, and comprising an amino acid sequence selected from the group consisting of [1] and [2] below.

[1] having an amino acid sequence corresponding to the amino acid sequence described in SEQ ID NO:1 with its N-terminal being any one of 1st to 6th amino acids thereof and its C-terminal being any one of 246th to 306th amino acids thereof.
[2] having an amino acid sequence corresponding to the amino acid sequence described in SEQ ID NO:1 with its N-terminal being any one of 1st to 6th amino acids thereof and its C-terminal being any one of 269th to 356th amino acids thereof and with any one or more out of 269th to 307th amino acids thereof being substituted by one or more other amino acids.

Needless to say, the CD14 mutant polypeptide described above may be a modified product of CD14 polypeptide, a chemically synthesized product, or artificially expressed product by genetic engineering and the method of producing it is not particularly limited.
(9) A CD14 mutant polypeptide according to (8), in which in (8) [1], the C-terminal is any one of 246th to 285th amino acids thereof.
(10) A CD14 mutant polypeptide according to (8), in which in (8) [2], the C-terminal is any one of 269th to 356th amino acids thereof and any one or more of 269th to 307th amino acids thereof are substituted by Leu, Ala, Cys or Gly.
(11) A CD14 mutant polypeptide according to any one of (8) to (10) above, in which one or more amino acids are deleted, substituted, inserted or added and the polypeptide has a function of inhibiting the binding between CD14 and TLR.
(12) A gene encoding a CD14 mutant polypeptide according to any one of (8) to (11) above.
(13) A gene comprising a DNA of [1] or [2] below

[1] DNA described in any one of SEQ ID NOs:4 to 6,
[2] DNA encoding a polypeptide, in which the DNA hybridizes with DNA described in any one of SEQ ID NOs: 4 to 6 under stringent conditions, and the polypeptide has a function of inhibiting the binding between CD14 and TLR.

(14) A recombinant vector containing a gene according to (12) or (13) above.
(15) A transformant containing recombinant a vector according to (14) above.
(16) A method of producing a CD14 mutant polypeptide according to any one of (8) to (11) above, characterized by comprising the step of culturing a transformant according to (15) above.
(17) A CD14 low molecular weight form having a molecular weight of 36±5 kDa obtainable from plasma.
(18) A binding inhibitor for inhibiting binding between CD14 and TLR, comprising an anti-CD 114 antibody, an antibody fragment, a polypeptide or a CD14 low molecular weight form.
(19) A method of screening a medicament for treating sepsis, comprising the steps of contacting a cell expressing TLR prepared by a genetic engineering method with CD14 and a test substance, detecting a change in a specified index, and judging whether or not the test substance can be a candidate of a medicament for treating sepsis.
(20) An anti-CD14 antibody, CD14 mutant or low molecular weight compound obtained by a screening method according to (18) or (19) above.
(21) A pharmaceutical composition for sepsis comprising a binding inhibitor for inhibiting binding between CD14 and TLR as an active ingredient.

BRIEF DESCRIPTION OF THE DRAWINGS

Fig. 1 is a graph illustrating luciferase induction activity by LPS/sCD14 in a human TLR4 expressing cell.
HEK293 is a cell that does not express TLR4 and HEKT4-14 is a recombinant HEK293 cell that is made to constitutively express human TLR4.
Fig. 2 is a graph illustrating inhibition by F1024-1-3 antibody to activation of NF-κB by LPS/CD14 through TLR4.
Note that inhibitory activity is calculated as described below.

(Luciferase activity when no antibody is added -

Luciferase activity when an antibody is added)/Luciferase

activity when no antibody is added $\times$ 100

Fig. 3 is a graph illustrating the effect of F1024-1-3 antibody to suppress IL-6 production in endothelial cells through LPS/CD14. ConA indicates a rat IgG for control.
Fig. 4 is a graph illustrating the effect of F1024-1-3 antibody to suppress IL-6 production in endothelial cells after the formation of LPS/CD14 complex.
Fig. 5 is a chart illustrating F1024-1-3 antibody not inhibiting the binding of FITC-LPS to CD14 expressing cells.
Fig. 6 is a diagram illustrating a peptide having activity of preventing binding of F1024-1-3 antibody to CD14(1-356).
Fig. 7 is a diagram illustrating binding activity of F1024-1-3 antibody to various kinds of CD14-deficient modified products.
Fig. 8 is a diagram illustrating binding activity of F1024-1-3 antibody to various kinds of CD14 amino acid substituted modified products.
Fig. 9 is a diagram illustrating results of Western Blotting conducted by using human CD14 antibody (3C10 and MEM-18) to determine the molecular weight of human soluble type CD14 C-terminal deleted modified polypeptide. Lanes 1 to 6 indicate preparations obtained by purifying sCD14(1-356), sCD14(1-307), sCD14(1-285), sCD14 (1-246), sCD14(1-183), and sCD14(1-152), respectively, by means of a 3C10 column.
Fig. 10 is a graph illustrating inhibitory activities to IL-6 production induced by LPS/CD14 in endothelial cells of a CD14 mutant polypeptide deleting C-terminal amino acids of human CD14 and of soluble type CD14 low molecular weight form derived from serum.

sCD14(1-356) indicates a full-length type human soluble CD14.

sCD14(1-307), sCD14(1-285), sCD14(1-246), sCD14(1-183) and sCD14(1-152) indicate CD14 mutant polypeptides having the positions from 1 to 307, from 1 to 285, from 1 to 246, from 1 to 183 and from 1 to 152, respectively, from the N-terminal of human CD14, and soluble type CD14 low molecular weight form derived from serum indicate preparations obtained by further purifying and concentrating a 3C10 column-purified preparation of serum from a normal person by means of F1033-3-1 column and F1025-3-1 column.

Fig. 11 is a graph illustrating the inhibitory activity of CD14 mutant polypeptide with sCD14(1-307) being substituted to IL-6 production in endothelial cells through LPS/CD14.

sCD14(1-307)D284A, sCD14(1-307)S286A and sCD14(1-307)C287A indicate CD14 mutant polypeptides obtained by substituting 284th amino acid from the N-terminal thereof, Asp, by Ala, 286th amino acid from the N-terminal thereof, Ser, by Ala, and 287th amino acid from the N-terminal thereof, Cys, by Ala, respectively.

Fig. 12 is a graph illustrating the inhibitory activity of CD14 mutant polypeptide obtained by substituting 286th amino acid of sCD14(1-307) from the N-terminal thereof, Ser to IL-6 production in endothelial cells through LPS/CD14.

sCD14(1-307)S286A, sCD14(1-307)S286C and sCD14(1-307)S286G indicate CD14 mutant polypeptides obtained by substituting 286th amino acid of sCD14 (1-307) from the N-terminal thereof, Ser, by Ala, Cys and Gly, respectively.

Fig. 13 is a graph illustrating the activity of CD14 mutant polypeptide to inhibit production of TNF$\alpha$ of peripheral macrophages through LPS.
Fig. 14 is a graph illustrating the suppressive effect of sCD14(1-307)S286C, which is a CD14 mutant polypeptide, against activation of NF-kB. The method of calculating inhibitory activity is the same as that for Fig. 2.
Fig. 15 is a diagram illustrating the binding amount when forming LPS/CD14/TLR4-COS complex by using BIACORE 3000 and the binding suppressing effect of TLR4-COS when binding F1024-1-3 antibody. Response is an index by BIACORE Co., with a graph of 1,000 RU indicating a binding amount of 1.2 ng.
Fig. 16 is a diagram illustrating the binding amount when forming LPS/CD14/TLR4-COS complex by using BIACORE 3000, and the binding suppressing effect when reacting CD14(s286C) with TLR4-COS cells. Response is an index by BIACORE Co., with a graph of 1,000 RU indicating a binding amount of 1.2 ng.
Fig. 17 is a diagram illustrating results of the bindability of CD14 mutant polypeptide (sCD14(1-307)S286A) to LPS.
Fig. 18 is a diagram illustrating results of Western Blotting conducted by using anti-human CD14 antibody (3C10 and MEM-18) to determine the molecular weight of CD14 polypeptide derived from human serum.

Lanel indicates results of a preparation purified from serum of a normal person by means of 3C10 column, and Lane2 indicates results of further purifying and concentrating a 3C10 column-purified preparation of serum from a normal person by means of F1033-3-1 column and F1025-3-1 column.

Fig. 19 is a graph illustrating the activity of CD14 polypeptide derived from human serum to suppress cytotoxicity through LPS/CD14.

Soluble type CD14 low molecular weight form derived from serum indicate preparations obtained by further purifying and concentrating a 3C10 column-purified preparation of serum from a normal person by means of F1033-3-1 column and F1025-3-1 column.

Fig. 20 is a graph illustrating the suppressing effect of F1024-1-3 and sCD14(1-307)S286C on induction production of IL-8 in HEKT4-14.

Note that the inhibitory activity is calculated as described below.

(IL-8 Production amount when adding no antibody or recombinant - IL-8 Production amount when adding antibody or recombinant)/IL-8 Production amount when adding no antibody or recombinant $\times$ 100

Fig. 21 is a graphical representation of IL-6 production inhibitory activity of U-373MG cell line by a CD14 mutant polypeptide with sCD14(1-307) being substituted an amino acid or amino acids, 3C10 antibody and F1024-1-3 antibody through Staphylococcus aureus cell suspension/CD14.

Fig. 22 is a graph illustrating cross reactivity of F1024-1-3 antibody with soluble type CD14 of dog, rhesus monkey, crab-eating monkey, rabbit or human:

Fig. 23 is a chart illustrating results of study on binding of F1024-1-3 antibody to rabbit CD14 by means of a fluorocytometry method.

Fig. 24 is a graph illustrating suppression of production of TNF by pre-administration of F1024-13 antibody in an LPS-loaded rabbit sepsis model.

Fig. 25 is a graph illustrating improvement of leukocytopenia by pre-administration of F1024-1-3 antibody in an LTA/PepG-administered rabbit sepsis model.

Fig. 26 is a graph illustrating improvements of values of ALT and creatinine by post-administration of F1024-1-3 antibody in an LPS-loaded rabbit sepsis model. Black circles indicate an antibody administered group and white circles indicate saline-administered group.

Fig. 27 is a graph illustrating results of the effect of CD14 mutant polypeptide in a mouse endotoxin shock model.

## BEST MODE FOR CARRYING OUT THE INVENTION

Hereinafter, the present invention will be described in more detail.

A first embodiment of the present invention relates to an anti-CD14 antibody that specifically recognizes an epitope including a part of the region from the positions 269 to 315 of. human CD14 described in SEQ ID NO:1 and has a function of inhibiting the binding of CD14 with TLR.

The amino acid sequence described in SEQ ID NO:1 is an amino acid sequence of human CD14. The protein composed of 356 amino acids described in SEQ ID NO:1 equals to a full-length of human CD14.

The term "epitope" used herein means an antigenic determinant, which indicates a structure site that specifically binds to an antibody.

The region that an antibody recognizes is not limited to one that recognizes amino acid residues continuously arranged in a primary sequence and some antibodies have an epitope that recognizes the three-dimensional structure of a protein (Protein Engineering of Antibodies, pages 1 to 4, ed. by Keizaburo Miki et al.: "Kodansha Scientific" 1991).

For example, amino acid residues discontinuously arranged exist in a position sufficiently close to be recognized by an antibody in a manner of the three-dimensional structure, resulting in that there are some cases where an antibody that recognizes amino acid residues discontinuously arranged in a primary sequence employs the amino acid residues discontinuously arranged as an epitope.

The "epitope including a part of the region that exists from the positions 269 to 315 of human CD14" refers to an epitope that exists from the positions 269 to 315 of the amino acid sequence of human CD14. For example, the epitope includes consecutive amino acids in a part of the region. However, it also includes amino acids that are discontinuously arranged in that region as described above. That is, the "part of the region that exists from the positions 269 to 315 of human CD14" includes a set of amino acids that are arranged continuously or discontinuously and also a set of amino acids which includes two or more interspersed of continuous or discontinuous amino acids as far as they exist in the region from the positions 269 to 315.

Also, the antibody of the present invention includes an antibody that recognizes an epitope including the region other than the positions 269 to 315. For example, it includes an antibody that specifically recognizes an epitope formed by one or more amino acid residues in the region from the positions 269 to 315 of human CD14 and several amino acid residues in a region other than that from the positions 269 to 315 of human CD14.

There is a reason thereof in that even when the epitope that the antibody of the present invention recognizes is a single amino acid residue in the region from the positions 269 to 315 of human CD14, it is recognized and the binding is conducted so that it can change the specified function that positions 269 to 315 of human CD14 has.

The antibody of the present invention specifically recognizes an epitope including a part of the region from the 269th to 315th of human CD14 and binds to human CD14. Human CD14 may be either sCD14 or mCD14. Also, there is included an antibody that specifically recognizes an epitope comprising a part of the 269th to 315th of either one of the CD14s. For example, sCD14 exists in blood while mCD14 exists in macrophages.

The fact that an epitope including a part of the region at positions 269 to 315 of human CD14 is specifically recognized, can be judged by binding to a soluble polypeptide having amino acids at positions 1 to 315 from the N-terminal of human CD14 (sCD14(1-315)) but not binding to sCD14(1-268) in the same manner as, for example, Example 6 which will be described hereinbelow.

The antibody of the present invention has a function of inhibiting the binding of CD14 with TLR by specifically recognizing the epitope including a part of the region from the 269th to 315th of human CD14.

CD14 binds to bacterial cell components and the CD14/bacterial cell components complex binds to TLR, thus transducing signal to cells to activate the cells. When cells are activated through the signal transduction mechanism, inflammatory cytokines are released from the cells to cause cytotoxicity, inflammation such as sepsis.

The antibody of the present invention suppresses activation of cells with the bacterial cell components by having the function of inhibiting the binding of CD14 with TLR, thus suppressing cytotoxicity and inflammation such as sepsis.

Explaining the "binding of CD14 with TLR" in detail, it means binding between a CD14/bacterial cell components complex, which is obtained by binding CD14 to bacterial cell components, and TLR. The bacterial cell components include LPS, LTA, peptide glycan (PGN), lipoarabinomannan or the like, mycoplasma, and the like. For example, LPS/CD14, which is a complex of LPS and CD14, transduces signal to cells by associating with TLR, in particular TLR2 or TLR4. When a signal is transduced into cells by TLR, activation of cells occurs through activation of NF-κB via MyD88, IRAK and NIK and the like. The antibody of the present invention inhibits binding of LPS/CD14 with TLR in this process.

"Having a function of inhibiting the binding of CD14 with TLR" is not particularly limited as far as the function of inhibiting the binding of CD14 with TLR is provided. This can be measured and judged by means of systems described in, for example, Examples 2, 13, 14 and 20, which will be described hereinbelow.

Also, as for the antibody of the present invention, it is preferred that an antibody suppresses activation of cells of TLR expressing cells by 30% or more is preferred.

In a more specific assay system, it is preferred that an antibody has a concentration of antibody of 10 μg/mL or less and suppresses the activation of NF-κB or IL-8 production of TLR expressing cells in the presence of 1 μg/mL of LPS and 0.5 μg/mL of exogenous CD14 by 30% or more. More preferably, the antibody is one that suppresses by 40% or more. Further preferably, the antibody is one that suppresses by 50% or more. Preferably in particular, the antibody is one that suppresses by 70% or more.

Further, as for the antibody of the present invention, it is preferred that an antibody suppresses production of cytokines in endothelial cells through exogenous LPS/CD14 to 60% or less. More specifically, it is preferred an antibody has an antibody concentration of 1 μg/mL or more and suppresses the IL-6 production of endothelial cells in the presence of 10 ng/mL of LPS and 300 ng/mL of exogenous CD14 to 60% or less. More preferably, the antibody is one that suppresses to 40% or less.

The binding of CD14 with TLR includes direct binding, binding through other factors, and binding activated by other factors. In blood on in vitro, there is included a case where the binding is performed in the pressure of serum derived from an animal. For example, there is included a case where the binding is performed at the time when the structure of TLR or of a dimer of TLR4 is stabilized in the presence of MD2. The antibody of the present invention may have a function of inhibiting any one of such bindings. For example, there is included an anti-CD14 antibody that has a function of inhibiting the binding of CD14 with TLR in blood or in vitro in the presence of serum derived from an animal.

The reason that the antibody of the present invention has a function of inhibiting the binding of CD14 with TLR consists in that the region is a region that is associated with binding of CD14 with TLR. AS a result, an antibody that specifically recognizes an epitope including a part of the region from the 269th to 315th of human CD14 has a function of inhibiting the binding of CD14 with TLR by changing the function of that region.

As for the region associated with the binding of CD14 with TLR, it is preferred that an antibody that recognizes an epitope region that functions as a region that is associated with binding of CD14 with TLR is completely included by the region from the positions 269 to 315 of human CD14. A more preferred epitope region is the region from the positions 285 to 315 of human CD14, and also, preferably it is the region from the positions 269 to 307 of human CD14 from the standpoint of having epitopes similar to that of F1024-1-3 antibody in the examples.

From the standpoint of changing the function of the region, the antibody of the present invention is preferably an antibody that recognizes a part of the region of antigen composed of 6 amino acids or more as an epitope. More preferably that part is composed of 8 amino acids or more. Further preferably, an antibody that specifically recognizes consecutive amino acids as an epitope.

The antibody of the present innovation is preferably an antibody that specifically recognizes an epitope comprising a part of the region from the positions 285 to 315 of human CD14 from the standpoint of having an epitope similar to the antibody of F1024-1-3 of Example. The antibody recognizes an epitope that resides from the positions 285 to 315 of a three-dimensional structure that can be generated by the fact that the amino acid at 294th position of CD14 is Pro.

Unlike other amino acids, Pro has an N atom bound to its α-carbon atom, which is incorporated into the cyclic structure to form an >NH group and therefore the polypeptide is restricted in its primary structure due to a Pro skeleton thereof, thus giving an influence on the three-dimensional structure of the protein. That is, Pro has no NH group and therefore it cannot form hydrogen bonds, thus failing to form an α-helix ("Protein Biotechnology," ed. by F. Frank, Baifukan). Although the antibody does not bind to CD14 which was carried out point mutation of Pro at position 294 of CD14, this is because three-dimensional structure of CD14 changed. That is, the antibody recognizes an epitope that resides from the positions 285 to 315 of the three-dimensional structure that can be generated by the fact that the amino acid at position 294 is Pro.

Further, the antibody of the present invention is an anti-human CD14 antibody. However, the present invention also includes antibodies against regions of CD14 of mammalians other than humans that are identical with the region concerned as far as it is an antibody that exhibits effects identical with those of the anti-human CD antibody described hereinbelow.

The amino acid sequence described in SEQ ID NO:2 is an amino acid sequence from the positions 269 to 315 of human CD14.

An antibody prepared by using a peptide that includes a part or whole region of the amino acid sequence described in SEQ ID NO:2 and has consecutive 6 or more amino acids as an immunogen is included in the antibody of the present invention. Preferably, it is an antibody prepared by using a peptide that includes a part or whole amino acid sequence of the region from the positions 285 to 307 of human CD14 and has consecutive 6 or more amino acids as an immunogen. Further, in consideration of the fact that a peptide assumes a three-dimensional structure, an antigen prepared by using a peptide having consecutive 8 or more amino acids is preferred. More preferably, it is an antibody prepared by using a peptide having consecutive 10 or more, more preferably consecutive 15 or more amino acids as an immunogen.

The antibody of the present invention has a function of inhibiting the binding of CD14 with TLR by specifically binding to CD14. CD14 may be either sCD14 or mCD14. Also, the antibody of the present invention includes antibodies having a function of inhibiting the binding between either one of CD14s and TLR. As the examples, there can be referred to antibodies having a function of inhibiting the binding between sCD14 and TLR that exists in blood or antibodies having a function of inhibiting the binding between mCD14 and TLR that exists on macrophage and the like.

The antibody of the present invention may be either a polyclonal antibody or monoclonal antibody. To clarify the function of the antibody or exhibit it clearly, a monoclonal antibody is preferred.

The species of animal from which the antibody of the present invention is derived is not particularly limited. In consideration of ease of preparing antibodies, rat is preferred. In a case where it is used as a constituent of a pharmaceutical composition, the antibody of the present invention is preferably a human antibody. The human antibody also includes human antibodies prepared by immunizing human antibody producing mice. Additionally, the antibody of the present invention includes humanized antibodies, phage antibodies, chimera antibodies or the like. The humanized antibody is an antibody that includes a constant region and a framework region derived from a human, and a complementarity determining region derived from a nonhuman. The phage antibody is an antibody prepared by fusing an antibody to the coat protein of filamentous phage to present the antibody on the surface of the phage particle, in which single chain Fv (scFv) form or Fab form is mainly used. The chimera antibody is an antibody that includes a variable region of monoclonal antibody from a nonhuman mammal, for example, mouse and a constant region from a human antibody.

The antibody of the present invention is not particularly limited to the molecular species thereof. Even when Immunoglobulins are classified into any class, subclass or isotype, they may be applied. Further, the present invention also includes biologically active antibody fragments, such as Fab, Fab', and $(Fab')_2$.

The antibody of the present invention can be prepared by using known technologies. For example, the monoclonal antibodies can be prepared by the following method.

The antibody of this invention can be prepared from the clones selected by using screening method of the twelfth embodiment of this invention described below from the hybridomas produced by fusing the myeloma cells and the immunized cells of the mammal which is immunized with a protein having the whole amino acid sequence shown in SEQ No:1 or a peptide including consecutive 6 or more amino acids of the region from positions 269 to 315 as an immunogen. Also, the antibody of the present invention may be prepared by selecting a clone that binds to sCD14 (1-315) whereas not binds to sCD14(1-268). Preferably a peptide composed of consecutive 6 or more amino acids in the region from the positions 285 to 307 is used as an immunogen. A peptide composed of preferably consecutive 8 or more amino acids, more preferably consecutive 10 or more, and particularly preferably consecutive 15 or more amino acids is used as an immunogen.

The mammal to be immunized is not particularly limited. However, it is preferred that it is selected in consideration of compatibility with myeloma cells used in cell fusion and mice, rats, hamsters or the like preferred. As the myeloma cell, various known cells can be used. They include myeloma cells such as P3, P3U1, SP2/O, NS-1, YB2/0 and Y3-Ag1, 2, 3 and so on.

The immunization may be performed by a known method. For example, it is performed by administering an antigen intraperitoneally, subcutaneously, intravenously or into a footpad. In a case of administering the antigen, an adjuvant may be used and it is preferred that the antigen is administered in plural times. In a case of administering the antigen, an adjuvant may be used in combination and it is preferred that the antigen is administered in plural times. The immunized cells are preferably spleen cells or cells originated from Lymph nodes, in which they are extracted at the time when several days passed after the final administration of antigen for example, 3 days.

The fusion between the immunized cells and myeloma cells can be performed by a known method such as the method of Milstein (Methods in Enzymol., Vol. 73, page 3). Examples of the known method includes a method of using polyethylene glycol (PEG) as a fusing agent, or an electrofusion method.

The mixing ratio between immunized cells and myeloma cells is not particularly limited as far as they can be fused each other. It is preferred to use myeloma cells in an amount 1/10 time that of the immunized cell or in an equivalent amount.

In a method in which cell fusion is performed by using PEG (average molecular weight 1,000 to 4,000), the concentration of PEG is not particularly limited, however it is preferred to perform the cell fusion at a concentration of 50%. Further, an auxiliary such as dimethyl sulfoxide (DMSO) may be added as a fusion accelerator.

The fusion is started by adding a PEG solution warmed to 37°C to mixed cells and terminated by addition of the medium after reaction for 1 to 5 minutes.

The hybridomas formed by this fusion are cultured in a selection medium containing hypoxanthine, thymidine, and aminopterine (HAT medium) for 1 to 7 days, to thereby separate from non-fused cells. The obtained hybridomas are further selected by the antibodies they produce. The selected hybridoma is monoclonized by a known limiting dilution method to establish it as a monoclonal antibody producing hybridoma.

As for the method of detecting the activity of antibody that is produced by the hybridoma, a known method may be used. Examples thereof include an ELISA method, a coagulation reaction method, and a radioimmunoassay method.

The established hybridoma is cultured by a known method and from its supernatant monoclonal antibody can be obtained. Also, the hybridoma may be administered to a mammal having compatibility therewith to proliferate it and a monoclonal antibody may be obtained from the ascites of the mammal.

The purification of antibody can be performed by using known purification means such as a salting out method, a gel permeation method, ion exchange chromatography, or affinity chromatography.

Furthermore, the human antibody may be prepared by using the method developed by Ishida et al. (PNAS, 97:722, 2000). That is, first a trans-chromosome (Tc) mouse is prepared as follows. According to the method of Ishida et al., human No. 2 chromosome fragment (Ig light chain κ) and No. 14 chromosome fragment (Ig heavy chain) are introduced into murine ES cells with a microcell fusion method, and chimera mice having respective chromosome fragments are prepared by the method of Joyner et al. ("Gene Targeting," Experimental Methods Series, Medical Science International). Then, the prepared two kinds of chimera mice are mated, to thereby prepare a chimera mouse having both human No. 2 chromosome fragment (Ig light chain κ) and No. 14 chromosome fragment (Ig heavy chain). In order to have the productivity of endogenous murine antibody originated from a mouse, a double KO mouse with endogenous Ig heavy chain and κ chain being knocked out is prepared by the method of Capecchi et al. (Mol. Cell. Biol. 12:2919-2923, 1992), and mated with a chimera mouse having introduced therein a human chromosome fragment, to thereby prepare a transchromosome mouse including human No. 2 chromosome fragment (Ig light chain κ) and No. 14 chromosome fragment (Ig heavy chain) with endogenous Ig heavy chain and κ chain being knocked out. The prepared Tc mouse produces human originated antibodies in blood, which include an antibody having murine γ chain as a part thereof. However no murine Ig(κ) is detected therein. Even after several generations, the obtained Tc mouse retains the chromosome and its off springs can be used for preparing an anti-CD14 human monoclonal antibody as described hereinbelow.

50 μg of purified CD14 antigen after being mixed with Titer Max Gold (Cytrex Co.) is subcutaneously administered to a Tc mouse and an additional administration is performed after 3 weeks in the same manner. An increase in antibody titer is determined by reacting diluted antiserum with a plate having immobilized an antigen thereto, then detecting bound human antibody in the serum with anti-human IgG antibody. Cell fusion is performed 3 days after

administering 50 μg of an antigen to the abdominal cavity of the mouse whose antibody titer has increased. Specifically, harvested spleen cells are mixed with murine myeloma cells (SP2/O-Ag14) and then fused with PEG4000 (Merck), followed by selecting hybridomas in HAT medium containing G418 (1 mg/mL). The hybridomas that is brought into emergence are screened by using anti-human IgG κ antibody, anti-IgG antibody, anti-IgG2 antibody, anti-IgG3 antibody or anti-IgG4 antibody as a secondary antibody, to thereby select a hybridoma that produces human antibodies binding to CD14. Furthermore, by the screening method according to the twelfth embodiment of the present invention which will be described later, the human CD14 antibody of the present invention can be obtained.

Further, the humanized antibody obtained by a CDR graft method can be prepared by using the method described in Nature, 321:522, 1986. Also, the humanized antibody can be prepared by using a mouse having introduced a part of human genes and the method described in J. Immunol. Methods, 231:11, 1999. The human/murine chimera antibody can be prepared by using the method described in Proc. Natl. Acad. Sci., 81:6851, 1984. The antibody obtained by a phage presenting method can be prepared by using the method described in Annu Rev. Immunol, 12:433, 1994. Diabody, tribody and tetrabody can be prepared by using di- tri- and tetramer of ScFv, respectively, and the methods described in FEBS Letter 454:90, 1999, Protein eg. 10:423, 1997 and FEBS Letter 453:164, 1999, respectively.

Fab, Fab', $(Fab')_2$ and the like, which are fragments of the antibody of the present invention, can be prepared by a known method (Eiji Ishikawa, Ultra-High Sensitive Enzyme Immunoassay, Japan Scientific Societies Press (JSSP)).

The method of producing antibodies includes a method of producing antibodies in plants (Nature 342:76, 1989), a method of producing antibodies in mammalian cells such as CHO cells, murine myeloma cells, etc. (Biotechnology 36:35, 1994), a method of producing antibodies in milk (Nat. Biotechnol 17:456, 1999) and a method of producing antibodies in Escherichia coli (Science 240:1038, 1988) and so on.

Polypeptides can be prepared by purifying soluble type CD14 in human serum with a known method. Also, a method of using a generally employed peptide synthesizer (Peptide Synthesizer 432A Type, Perkin-Elmer, Japan), or the like, a genetic recombination method ("New Cell Engineering Experiments Protocols," Ed. Department of Carcinostatic Research, The Institute of Medical Science, The University of Tokyo, Shujunsha) and the like may be used.

For example, a peptide having consecutive 6 or more amino acids that reside in the region from the positions 269 to 315 can be synthesized by an Fmoc method with using 432A Type peptide synthesizer. After deprotection with TFA and cutting out from the resin, it is purified by using C18 HPLC column (Capcell-pak, Shiseido Co., Ltd.), to thereby prepare the target peptide.

One preferred example of the antibody of the present invention is F1024-1-3 antibody produced by hybridoma F1024-1-3 obtained by cell fusion between immunized cells, which is prepared by immunizing a rat with CD14 protein purified from human serum as an antigen, and myeloma cells. The hybridoma F1024-1-3 has been deposited at National Institute of Bioscience and Human-Technology, Agency of Industrial Science and Technology, Ministry of International Trade and Industry, Japan at 1-3, Higashi 1-chome, Tsukuba-shi, Ibaraki-ken, Japan (Hereinafter, referred to as "National Institute of Bioscience and Human-Technology") on September 29, 2000) (Accession No. P-18061) and transferred from the original deposit to an international deposit (Accession No. FERM BP-7511) on March 16, 2001).

The antibody of the present invention has a function of inhibiting the binding between CD14 and TLR. That is, it is a binding inhibitor for CD14 and TLR. This indicates that it has a function of suppressing human CD14-mediated cell activation, so that it can be used in the therapy or prevention of diseases associated with bacterial infection. For example, it is useful in the therapy or prevention of symptoms accompanying an increase in concentration of inflammatory cytokines, in particular blood TNF, associated with diseases such as sepsis. More specifically, it is useful in the therapy or prevention of fever, hypotension, leukocytopenia, thrombocytepenia, a shock, and multiple organ insufficiency. Further, the causative agent of cell activities that the antibody of the present invention suppresses is not limited to LPS alone. The antibody of the present invention also suppresses the cell activation mediated by CD14 that has formed a complex with, for example, LTA, PGN, mycoplasma or the like. That is, in diseases caused by these substances, the antibody of the present invention is useful in the therapy or prevention thereof as described above.

Since the antibody of the present invention has a function of specifically recognizing an epitope comprising a part of the region from the positions 269 to 315 of human CD14, it is useful as a tool for qualitative or quantitative determination of human CD14. For example, there is a measurement method in which an analyte and the antibody of the present invention are contacted to form an antigen-antibody complex, and then the complex is detected or quantitated. Examples of analyte include serum, urine, body fluid, supernatant of culture and the like. As for the detection or quantitation method of the antigen-antibody complex, an ELISA method, a RIA method or the like may be exemplified.

A second embodiment of the present invention relates to a hybridoma that produces the monoclonal antibody of the present invention. The hybridoma of the present invention can be prepared by the method described in the first embodiment of the present invention.

One preferred example of the hybridoma of the present invention is Hybridoma F1024-1-3 (Accession No. P-18061) that produces F1024-1-3 antibody, which is a preferred example of the antibody of the present invention.

A third embodiment of the present invention relates to a peptide comprising consecutive 8 or more amino acids in the region from the positions 269 to 315 of human CD14 described in SEQ ID NO:1. Preferably, it is a peptide comprising consecutive 8 or more amino acids in the region from the positions 285 to 307 of human CD14. Also, preferably, it is a peptide comprising consecutive 10 or more, particularly preferably consecutive 15 or more amino acids.

The peptide of the present invention can be prepared by the method described in the first embodiment of the present invention.

A fourth embodiment of the present invention relates to a method of preparing an antibody characterized by using the peptide according to the third embodiment of the present invention as an immunogen. Preferred examples of the peptide used as an immunogen include the preferred examples of the peptide according to the third embodiment of the present invention.

The particulars of the method of the present invention are the same as those described in the first embodiment of the present invention.

However, since peptides have small molecular weights, they usually have no immunogenicity. Therefore, they are bound to a carrier by utilizing a functional group of the side chain contained in an amino acid thereof to convert it into an immunogen. As the carrier, bovine serum albumin, keyhole limpet hemocyanin (KLH), cycloglobulin, avoalbumin, or the like may be used.

It is preferred that the antibody prepared by the method of the present invention has a function of inhibiting the binding between CD14 and TLR.

A fifth embodiment of the present invention relates to a CD14 mutant polypeptide having a function of inhibiting the binding between CD14 and TLR and being either one of [1] and [2] below:

[1] having an amino acid sequence corresponding to the amino acid sequence described in SEQ ID NO:1 with the N-terminal being any one of amino acids at positions 1 to 6 thereof and the C-terminal being any one of amino acids at positions 246 to 306 thereof;

[2] having an amino acid sequence corresponding to the amino acid sequence described in SEQ ID NO:1 with the N-terminal being any one of amino acids at positions 1 to 6 thereof and the C-terminal being any one of amino acids at positions 269 to 356 thereof, and at least any one of amino acids at positions 269 to 307 thereof being substituted by other amino acid or acids.

Hereinafter, explanation will be made mainly on the amino acid sequence of human CD14 polypeptide and the amino acid sequence of human CD14 will be sometimes termed simply as amino acid sequence of CD14. However, CD14 may be CD14 polypeptides from species other than human as far as it is CD14 of a mammalian other than human and has a portion comprising a function corresponding to the amino acids at positions 269 to 307 of human CD14 being modified similarly to exhibit equivalent effect to that of the modified product of the present invention.

"Modified CD14 polypeptide" includes a polypeptide comprising CD14 polypeptide having deleted a part thereof, a polypeptide comprising CD14 polypeptide having a part of the amino acids thereof substituted, and a polypeptide comprising CD14 polypeptide having added thereto amino acid or acids. It also includes modified polypeptides having some of these modifications in combination.

The polypeptide of the present invention has a function of inhibiting the binding between CD14 and TLR. The particulars of the function of inhibiting the binding between CD14 and TLR are as described in the first embodiment of the present invention.

Further, the polypeptide of the present invention is preferably a polypeptide that suppresses cell activation of TLR expressing cells by 30% or more.

In a more specific assay system, a polypeptide is preferred that suppresses the activation of NF-κB or production of IL-8 in TLR expressing cells in a polypeptide concentration of 10 μg/mL or lower and in the presence of 1 μg/mL of LPS and 0.5 μg/mL of exogenous CD14 by 30% or more. More preferably, it is a polypeptide that suppresses by 40% or more. Further more preferably, it is a polypeptide that suppresses by 50% or more. Particularly preferably, it is a polypeptide that suppresses by 70% or more.

Further, the CD14 mutant polypeptide of the present invention is preferably a polypeptide that by itself does not induce cytokines in endothelial cells in the presence of LPS. In a more specific assay system, it is preferred that this polypeptide does not induce IL-6 production in endothelial cells in the presence of 10 ng/mL LPS at a polypeptide concentration of 300 ng/mL or less.

The term "does not induce" means that IL-6 to be measured in the assay system concerned is below the detection limit. For example, in using a human IL-6 EIA kit (PE Biosystems Corp.) in the measurement of IL-6 production, "below the detection limit" means 50 pg/mL or less.

The CD14 mutant polypeptide of the present invention is preferably a polypeptide that suppresses production of cytokines through exogenous LPS/CD14 in endothelial cells to 60% or less.

In a more specific assay system, a polypeptide is preferred that suppresses production of IL-6 in endothelial cells in a polypeptide concentration of 300 ng/mL or more in the presence of 10 ng/mL of LPS and 300 ng/mL of exogenous CD14 to 60% or less. More preferably, it is a polypeptide that suppresses to 40% or less.

The "LPS/CD14" means a complex of LPS and CD14 bound to each other. In consideration of the activity, among the polypeptides in [1] above, CD14 mutant polypeptides having a C-terminal that corresponds to any one of the amino acids at positions 246 to 285 of CD14 is preferred. From the standpoint of molecular weight, the CD14 low molecular weight form of the present invention described later have a C-terminal that corresponds to any one of the amino acids at positions 246 to 285 of CD14 and have the same function as that of the CD14 mutant polypeptide of the present invention. This constitutes grounds for the observation that CD14 mutant polypeptides having C-termini corresponding to any one of amino acids at positions 246 to 285, which are preferred examples, have the same function. More preferred are polypeptides that have a C-terminal corresponding to the amino acid 246 or 285 of CD14.

Also, in respect of the activity, among the polypeptides in [2] above, those polypeptides are preferred that have C-termini corresponding to any one of amino acids at positions 269 to 356 thereof and have any at least one of amino acids at positions 269 to 286 thereof substituted by other amino acid or acids, i.e., Leu, Ala, Cys or Gly. More preferred are those CD14 mutant polypeptides that have C-termini corresponding to amino acids at positions 284 to 356 thereof and have at least any one of amino acids at positions 284 to 286 thereof substituted by other amino acid or acids, i.e., Leu, Ala, Cys or Gly. Furthermore, those CD14 mutant polypeptides are preferred that have C-termini corresponding to the amino acid at position 307 thereof and at least any one of amino acids at positions 284 to 286 thereof substituted by other amino acid or acid, i.e.. Leu, Ala, Cys or Gly.

From the point of view of removing bacterial cell components by the polypeptide itself, it is preferred that the polypeptide of the present invention binds to bacterial cell components. For example, binding site of CD14 of LPS is at positions 7 to 14 and 57 to 64 thereof and the sequences of the sites are maintained in the polypeptide of the present invention.

In the polypeptide of the present invention, 1 to 6 amino acids at the N-terminal may be deleted, substituted, inserted or added as far as its function is maintained. For example, a polypeptide in which Met is further added to the N-terminal thereof is also included in the polypeptide of the present invention. In addition, in the amino acid sequence in the midway, one or several amino acids may be deleted, substituted, inserted or added.

The polypeptide of the present invention may be subjected to any modification as far as it does not lose its characteristics. The modification includes modification during or after translation of polypeptide, chemical modification and the like which the protein will be possibly subjected to during its production by culturing eucaryotic cells such as animal cells or yeast cells.

These include, for example, addition of methionine or sugar chain and the like. As the sugar addition to proteins, N-glycosylation, O-glycosylation and nonenzymatic sugar addition, and the like are known. The polypeptide of the present invention may or may not have any sugar chain. Furthermore, the number of sugar chains, length of sugar chain or sugar composition and sequence of sugar chain may vary depending on the composition of medium when culturing hosts and the like so that they are not particularly limited. Other natural modifications include binding of lipids and the like.

Also, it is possible to further modify the polypeptide in various manners. The modifiable site includes functional groups in the amino acid residue at the N- or C-terminal or in the side chain. For example, synthetic polymers such as polyethylene glycol, styrene/maleic acid copolymer, dextran, pyran copolymer, and polylysine, natural molecules such as lipids, and polysaccharides, bioactive molecules such as hormones, inorganic substances such as magnetite and the like can be bound to the polypeptide and also a peptide can be bound to the side chain (Proc. Natl. Acad. Sci. USA, 84, 1487-1491, 1981); Biochemistry, 28, 6619-6624, 1989).

Besides, the polypeptides subjected to modification include, for example, an aliphatic ester of a carboxyl group, amide by reaction of a carboxyl group with ammonia or a primary amine or a secondary amine, N-acyl derivatives of free amino acid group in amino acid residues formed from acyl residue of a carboxyl group (for example, alkanoyl or carboxylaroyl group), or O-acyl derivatives of free hydroxyl group formed form acyl residue (for example, hydroxyl group of serine or threonine residue).

The polypeptide of the present invention can be subjected to the modifications as described above by a combination of known methods as described above. Therefore, the polypeptide of the present invention includes such modified polypeptides as far as its characteristics are not lost. Further, it is possible to modify or improve the activity or function of the polypeptide of the present invention or impart or combine other activity or function thereto by such modifications. For example, the activity or function includes stability, solubility, in vivo kinetics, directivity toward specified cell, tissue or organ and the like. Polypeptides having subjected to such modifications are also included in the scope of the present invention.

Furthermore, the polypeptide of the present invention includes those existing in the form of salts. The term "salts" as used herein means both salts of carboxyl groups and acid addition salts to amino groups of the protein molecule. The salts of carboxyl group include inorganic salts such as salts of sodium, calcium, ammonium, Fe(III) or zinc, and organic salts such as salts formed by triethanolamine, arginine or lysine, piperidine, procaine or the like. The acid addition salts include salts with mineral acids, for example, hydrochloric acid or sulfuric acid and salts with organic acids, for example, acetic acid or oxalic acid.

The polypeptide of the present invention can be applied to pharmaceutical preparations. In that case, it is desirable that the aforementioned modifications and the like maintain pharmaceutical acceptability.

The polypeptide of the present invention has a function of inhibiting the binding between CD14 and TLR, that is, it is a binding inhibitor to CD14 and TLR. Because of this, it has a function to suppress human CD14-mediated cell activation so that it can be used in the therapy and prevention of diseases associated with bacterial infection. For example, it is useful in the therapy and prevention of inflammatory cytokines accompanying diseases such as sepsis, in particular symptoms caused by an increase in blood TNF level. More specifically, it is useful in the therapy and prevention of symptoms such as fever, hypotension, leukocytopenia, thrombocytopenia, a shock, and multiple organ insufficiency. Furthermore, the causative agent of cell activation that the polypeptide of the present invention suppresses is not limited to LPS alone. The polypeptide of the present invention also suppresses the cell activation mediated by CD14 that has formed a complex with, for example, LTA, PGN, lipoarabinomannan, mycoplasma or the like. That is, in diseases caused by these substances, the polypeptide of the present invention is useful in the therapy or prevention thereof as described above.

Further, the method of producing the polypeptide of the present invention is not particularly limited. It may be artificially synthesized by using a generally employed chemical synthesizer (Peptide Synthesizer 430A Type, Perkin-Elmer Japan) or the like or may be one obtained by a genetic recombination method. Alternatively, it may be one obtained by subjecting precursor polypeptides separated and purified from these to appropriate chemical treatment, for example, a treatment with an enzyme to cause deletion or fragmentation and or one obtained by chemically binding them or polymerizing them.

Among the aforementioned ones, those produced by genetic engineering are preferred in consideration of production amount, homogeneity of produced protein or purity, for example, absence of human originated other contaminant proteins and so on. Specific examples thereof will be described in detail in the examples herein.

A sixth embodiment of the present invention is a gene encoding the polypeptide according to the fifth embodiment of the present invention.

Since it is generally known that triplet (codons) of DNA of a gene that encodes an amino acid exists in a number of from 1 to 6 depending on the kind of amino acid, the base sequence of DNA of the gene encoding the polypeptide of the present invention is not limited to one kind. Therefore, as far as it is a gene that contains a base sequence encoding the polypeptide of the present invention, the gene is included in the scope of the present invention whatsoever base sequence it may have. Preferred genes of the present invention include a gene comprising DNA having a sequence starting with adenine at position 174 on the 5'-terminal and the 3'-terminal being from thymine at position 722 to adenine at position 1091 of SEQ ID NO:3 in the sequence listing or a gene comprising DNA having a sequence starting with adenine at position 174 on the 5'-terminal and the 3'-terminal being from guanine at position 980 to cytosine at position 1241 of SEQ ID NO:2 in the sequence listing from among the base sequences encoding the polypeptide of the present invention, and having a part of the triplets encoding the amino acids in the sequence from cytosine at position 978 thereof to guanine at position 1094 thereof being substituted by GCT or GCG (codon for Ala), GGT (codon for Gly), CTT (codon for Leu) or TGT (codon for Cys).

The base sequences of DNAs of preferred genes of the present invention are shown in SEQ ID NO:4 to SEQ ID NO:6. Also, the gene of the present invention includes those DNAs encoding polypeptides that hybridize with the DNAs composed of the base sequences shown in SEQ ID NO:4 to SEQ ID NO:6 under stringent conditions and have a function of inhibiting the binding between CD14 and TLR.

The "stringent conditions" means that hybridization is performed in a solution composed of, for example, 50% formaldehyde, standard citric acid solution (SSC) of 50 fold concentration, 50 mM phosphate buffer solution (pH 6.5 to 6.9), 40 μg of salmon sperm DNA, and Denhardt's solution (Funakoshi) of 4 fold concentration at a temperature lower than Tm obtained by the base sequence of DNA by 25°C. In addition, the conditions used in the method of Sambrook, J. et al. (Molecular Cloning: A Laboratory Manual, 2nd ed., Cold Spring Harbor Laboratory, New York, 1989) may be used.

The gene of the present invention may be cDNA, chromosomal DNA and combinations thereof as well as cDNA that contains intron capable of being appropriately spliced. However, the DNA of the present invention is preferably cDNA in view of ease of handling in genetic engineering.

According to the present invention, there are provided RNA corresponding to the novel gene of the present invention, or DNA and RNA containing sequences complementary to the DNA of novel gene of the present invention. The DNA of novel gene of the present invention and DNA and RNA complementary thereto may complementarily bind to each other to form a double strand or triple strand. Also, DNA and RNA containing base sequences that can hybridize with the DNA of the novel gene of the present invention are also provided. Further, the hybrids are also provided. In this case too, they may bind to each other to form a double strand or a triple strand. In the aforementioned cases, as nucleic acid base, universal base such as inosine may be used.

The gene of the present invention may be obtained by any method. For example, it may be one that is chemically synthesized, one that is obtained from an appropriate DNA library or one that is obtained by a PCR (polymerase chain reaction) method by using a DNA containing the DNA of the gene encoding the full length or a part of CD14 as a template. Further, the gene of the present invention may be prepared by annealing or ligating the genes obtained by these methods or fragments thereof as necessary.

The DNA of the gene of the present invention can be chemically synthesized as follows. Specifically, the DNA of the gene of the present invention is divided into fragments each composed of about 20 to 30 bases synthesized as a plurality of fragments by synthesizing these fragments using a DNA chemical synthesizer (for example, 394 Type, manufactured by Applied Biosystems, Inc.), and thereafter optionally phosphorylating the 5'-terminal of each fragment, annealing each fragment and ligating the fragments to obtain the target DNA.

Also, the gene of the present invention can be obtained by a PCR method using a genome library, cDNA library or the like as a template. When using a PCR method, a sense primer and an antisense primer designed based on known base sequences, the base sequence of DNA of the gene encoding the polypeptide of the present invention, and optionally restriction enzyme recognizing sequence and the like in combination are prepared and the gene of the present invention can be obtained by using any optional DNA library or the like according to a known method (cf., Michael AI, et. al. ed., Polymerase Chain Reaction, PCR Protocols, a guide to methods and applications, 1990, Academic Press). Typical examples thereof will be described in Examples herein.

The above-mentioned DNA library may be any library as far as it contains the DNA or part of the gene of the present invention and is not particularly limited. Therefore, a commercially available DNA library may be used. Alternatively, cDNA may be prepared according to the method of Sambrook, J. et al from appropriate cells such as lymphocytes obtained from human peripheral blood, human cell line, or hybridomas, activated with an appropriate activator as necessary, and the obtained cDNA may be used. Note that the transformants containing DNAs encoding polypeptides having a human CD14 signal sequence composed of 19 amino acids described below and a peptide having the region with the N-terminal starting at position 1 and the C-terminal being leucine at position 285 of sequence corresponding to SEQ ID No:1 or a peptide having the region with the N-terminal starting at position 1 and the C-terminal being leucine at position 307 sequence corresponding to SEQ ID No:1 of human CD14, with serine at position 286 thereof being substituted by alanine have been deposited at National Institute of Bioscience and Human-Technology at 1-3, Higashi 1-chome, Tsukuba-shi, Ibaraki-ken, Japan on March 14, 2000 (Accession Nos. P-17777 and P-17778) and transferred from the original deposits to international deposits (Accession Nos. FERM BP-7479 and FERM BP-7480) on March 16, 2001.

The gene of the present invention can be used in preparing the recombinant vector according to the seventh embodiment of the present invention and by transforming a host with the recombinant vector, the polypeptide of the present invention can be produced uniformly and on a large scale.

This makes it possible to provide the polypeptide of the present invention as a main component of a remedy in the field of pharmaceutical preparations or use it as a diagnostic agent. The method of producing the polypeptide of the present invention on a large scale by using DNA of the gene of the present invention will be described in the description with respect to a ninth embodiment of the present invention.

A seventh embodiment of the present invention is a recombinant vector characterized by containing the gene according to the sixth embodiment of the present invention.

The recombinant vector of the present invention may be of any form such as cyclic or linear, or single strand, double strand or chains of a complex of these and may be selected as appropriate depending on the purpose. However, in consideration of ease of handling and ease of incorporation into hosts, it is preferably to be cyclic and from the standpoint of stability and the like, it is preferably of a double strand.

The recombinant vector of the present invention may be a base sequence of DNA of the gene of the present invention but has at least one optional base added to either one of or both of the 5'-terminal and 3'-terminal thereof.

The base added here may be any base as far as it causes no shift in coding frame to DNA of the gene of the present invention. It includes, for example, an adapter sequence, a linker sequence, a base sequence encoding a signal sequence, a base sequence encoding other polypeptide such as β-galactosidase, or a sequence added in order to increase detection sensitivity when preparing a DNA probe or the like.

The recombinant vector of the present invention preferably contains in addition to the gene of the present invention other base sequence as necessary. The "other base sequence" as used herein means a sequence of enhancer, a sequence of promoter, a sequence of ribosome binding site, a base sequence used for amplifying the copy number of DNA, a translation initiator codon, a base sequence encoding a signal peptide, a base sequence encoding other polypeptide, a translation terminator codon, a poly A addition sequence, a splicing sequence, a replication origin, gene sequence serving as a selective marker and the like.

What a base sequence is required depends on the purpose the recombinant vector to be prepared is used for. Preferably, the vector is one that can transform host cells so that the polypeptide of the present invention can be produced on a large scale. Therefore, it is preferred that the recombinant vector contains in addition to the gene of the present invention a translation initiator codon, a terminator codon, a replication origin, and a sequence of a selective marker gene. More preferably, the vector is one that contains a promoter sequence that functions in the host. In particular, the vector that contains a sequence encoding a signal peptide in addition to these sequences is particularly preferred since it can transform host cells so that they can express and secrete polypeptide of the present invention.

By preparing a recombinant vector comprising a DNA having any sequence corresponding to SEQ ID NO:2 in the sequencing list with the 5'-terminal starting with adenine at position 174 and the 3'-terminal being from thymine at position 722 to adenine at position 1091 thereof or any sequence corresponding to SEQ ID NO:2 with the 5'-terminal starting with adenine at position 174 thereof and the 3'-terminal being from guanine at position 980 to cytosine at position 1241 thereof, each having a part of triplet encoding amino acids in the sequence from cytosine at position 978 to guanine at position 1094 thereof being substituted by GCT or GCG(codon for Ala), GGT(codon for Gly), CTT(codon for Leu) or TGT(codon for Cys), connected to downstream of a signal sequence in the appropriate vector encoding a signal peptide and transforming appropriate host cells with this recombinant vector, when culturing the obtained transformant, a CD14 mutant polypeptide comprising a polypeptide having an amino acid sequence described in SEQ ID NO:1 in the sequencing list with the C-terminal being any one of amino acids at positions 183 to 306 thereof or a CD14 mutant polypeptide comprising a polypeptide having an amino acid sequence described in SEQ ID NO:1 with the C-terminal being any one of amino acids at positions 269 to 356 thereof in which at least one of the amino acids at positions 269 to 307 thereof is substituted by other amino acid or acids can be secreted in the supernatant of the culture. The signal sequence to be connected can be selected as appropriate. However, a signal sequence encoding a signal peptide of human CD14 having the sequence of

Met Glu Arg Ala Ser Cys Leu Leu Leu Leu Leu Leu Pro Leu Val His Val Ser Ala (Goyer et al., Nuclic Acid Research, Vol. 16, page 4173, 1988) is preferred.

In addition, appropriate base sequences may be selected and utilized from sequences encoding a signal peptide of TNF or G-CSF, a signal peptide of alkaline phosphatase of Escherichia coli, a signal peptide of yeast PHO1 and a signal peptide of yeast α-factor) and the like depending on the host and conditions to be used.

Also, in a case where Escherichia coli is used and the base sequence is expressed as an inclusion body, it is preferred to add methionine or a peptide containing methionine at the N-terminal thereof.

The selective marker gene may include an ampicillin resistant gene, a kanamycin resistant gene, a neomycin resistant gene, a thymidine kinase gene and the like. Recombinant vectors containing at least two selective markers are preferred because they enable selection of a clone transformed with the target gene when yeast or mammalian cells are used as a host. As for the sequence used in order to amplify the copy number, a sequence of dehydrofolic acid reductase gene (dhfr) and the like can be used.

The promoter sequence that functions in a host includes a trp promoter or lac promoter or a T7 polymerase when the host is Escherichia coli, or a promoter of alcohol oxidase (AOX1), a polyhedrine promoter, a promoter of SV40, a promoter of SRα, a promoter of human elongation factor 1α (EF1α), a promoter of cytomegalovirus (CMV) and the like when the host is eucaryotic cells such as yeast or COS cells.

From the standpoint of hosts, preferred examples of the recombinant vector of the present invention are those that transform eucaryotic cells such as animal cells or yeast so that they can exhibit the polypeptide of the present invention. Therefore, preferred examples of the recombinant vector of the present invention include those vectors that contain at least a translation initiator codon, a terminator codon, and a selective marker gene and in addition thereto, a poly A addition sequence, and further a promoter of SV40, a promoter of EF1α, or a promoter of SRα that functions in animal cells, or a promoter of AOX1 that functions in yeast, as well as a replication origin of SV40 and so on.

The recombinant vector of the present invention can be obtained by a method in which DNA of the gene of the present invention is ligated to other DNA fragment having any optional base sequence, a method in which DNA of the gene of the present invention is introduced in any optional vector (cf., Sambrook J. et al., Molecular Cloning, a Laboratory Manual 2nd ed., Cold Spring Harbor Laboratory, New York, 1989) or the like.

Specifically, it is recommended that DNA and a vector are digested with appropriate restriction enzymes, respectively, and the obtained respective fragments are ligated with a DNA ligase. The vector may be any vector such as a plasmid vector, a phage vector, or a virus vector. A commercially available vector may also be used. Typical examples of the vector include pUC118, pBR322, pSV2-dhfr, pBluescriptII, PHIL-S1, λZapII, λgt10, pAc700, YRP17, pEF-BOS, pEFN-II and the like.

The recombinant vector of the present invention may be used for any purpose. For example, it may be used when the polypeptide of the present invention is produced on an industrial scale or it may be used for amplifying and mass producing a novel DNA of the present invention. Furthermore, the recombinant vector of the present invention can be used in preparing the transformant according to the fourth embodiment of the present invention.

An eighth embodiment of the present invention relates to a transformant characterized by being transformed with the recombinant vector of the present invention. That is, the transformant of the present invention can be obtained by introducing the recombinant vector of the present invention in a cell or microbe that serves as a host.

The transformant of the present invention may be obtained by transforming either one of procaryotic cell and eucaryotic cell. The procaryotic cell includes Escherichia coli, Bacillus subtilis, etc. The eucaryotic cell includes mammalian cells such as COS cell, CHO cell, Hela cell, and Namalwa Cell and in addition thereto insect cells such as Sf cell, and yeast. Among these, transformants obtained by transforming Escherichia coli, mammalian cells and yeast are preferred since they are easy to handle and high expression amounts can be expected.

Among the mammalian cells, it is preferred to use dhfr deficient cell line of CHO cell as a host since the copy number of the gene can be increased. On the other hand, among yeasts, it is preferred to use yeasts belonging to the genus Pichia as a host since it produces a large amount of exogenous protein by secretion or it is preferred to use Schizosaccharomyces pombe as a host since it has an adduct sugar chain similar to that of mammalian species.

In addition, in order to sufficiently exhibit the function of the recombinant vector of the present invention, the vector to be used in preparing the recombinant vector of the present invention must be of a kind suited for host cells. Examples of preferred combination of a vector used in a recombinant vector and a host include a combination of PUC118 and Escherichia coli, a combination of pET and a host E. coli having a T7RNA polymerase expression system (Majerle A., et al.: Protain Expr. Purif., 17:96, 1999), a combination of pEF-BOS and a COS cell or CHO cell, a combination of Yac and yeast, a combination of AcNPV and an Sf cell, and so on (cf. Special Issue of Experimental Medicine, Genetic Engineering Handbook, Yodosha, 1991). Similarly, the promoter contained in the recombinant vector, base sequence encoding a signal peptide, marker gene and the like suited for the host are used.

To obtain a transformant that expresses the polypeptide of the present invention, the recombinant vector must have sequences necessary for expression such as an appropriate promoter and the like in the vector.

In order to obtain a transformant that produces the polypeptide of the present invention by secretion, a host cell may be transformed with the recombinant vector of the present invention suited for the above-mentioned production that contains a base sequence encoding a signal peptide upstream of the gene of the present invention.

As for the method of introducing the recombinant vector of the present invention into a host cell, a method suited for the host and recombinant vector may be selected from known methods including an electroporation method, a protoplast method, an alkali metal method, a calcium phosphate method, a DEAE-dextran method, a microinjection method, a method of infecting by using virus particles, and other known methods (cf. Special Issue of Experimental Medicine, Genetic Engineering Handbook, Yodosha, 1991). Since when the calcium phosphate method or DEAE-dextran method is used, transformation of mammalian cells is performed efficiently, it is preferred to use these methods when the host cell is a mammalian cell.

The transformant of the present invention is preferably a transformant that expresses the polypeptide of the present invent, particularly preferably one that expresses the polypeptide of the present invention and secretes it in the supernatant of culture. This is because by using such a transformant, it becomes easy to produce the polypeptide of the present invention in a large amount.

Although the transformant of the present invention may be used for any purpose, it may be used for the purpose of producing the DNA of the present invention or recombinant DNA molecule of the present invention in large amounts or for the purpose of producing the polypeptide of the present invention on an industrial scale and for other purposes.

A ninth embodiment of the present invention relates to a method of producing the polypeptide of the present invention characterized by comprising the step of culturing the transformant of the present invention.

The method of preparing the transformant of the present invention is as described above. In the production method of the present invention, the transformant of the present invention is cultured and amplification or induction of expression of gene is performed as necessary. Then, culture mixture is recovered and the polypeptide of the present invention is purified by a suitable combination of operations such as concentration, solubilization, dialysis, and various kinds of chromatographies.

The "culture mixture" means a transformant, a medium containing a transformant, culture supernatant, or a lysate of cells. In the production method of the present invention, when the produced polypeptide of the present invention is secreted in the supernatant of cell culture, the polypeptide can be purified from the culture supernatant. On the other hand, when the polypeptide is accumulated in the transformant, the cells are dissolved or spalled by appropriately selecting a method suited for the host cell from a lysozyme treatment, surfactant treatment, freeze thawing, compression, ultrasonication and other methods and then the polypeptide is recovered and purified as a soluble fraction or insoluble fraction by centrifugation, filtration or the like method.

In a case where the host cell is Escherichia coli and the polypeptide of the present invention is accumulated in the periplasm, the polypeptide can be recovered by adopting the method of Willsky et al. (J. Bacteriol., 127, 595-609, 1976).

Culture of the transformant can be performed by a generally accepted technique by referring to various books (cf., for example, "Microbe Experimentation," ed. Incorporated Association the Japanese Biochemical Society, Tokyo Kagaku Dojin Co., Ltd., 1992).

When induction of expression of a gene is performed, a suitable drug selected depending on the promoter incorporated is used. For example, in the case where a trp promoter is incorporated, 3β-indolacrylic acid may be used, while dexamethazone may be used in the case of MMTV promoter or methanol may be used in the case of AOX1 promoter.

Typical examples of amplifying a gene include a method of using methotrexate when a dhfr deficient CHO cell as a host and a vector having dhfr are used and other methods.

The transformant used in the production method is not limited as far as it is the transformant of the present invention. However, preferably it is a transformant that uses any cell selected from mammalian cells such as COS cell and CHO cell, yeast, and E. coli as a host.

Hereinafter, examples of culture and induction of expression when using E. coli, mammalian cells such as CHO cell, or a Pichia yeast as a transformant will be shown.

In the case of E. coli transformed with a recombinant DNA molecule having a trp promoter, the bacterial cells precultured in L-Broth are inoculated in M9-CA medium in an amount of 1/50 and cultured at 37°C. After several hours from the initiation of culture at a point in time when the value of OD550 has reached to 1 to 4 (that is, a logarithmic growth stage), 3β-indoleacrylic acid is added to a final concentration of 10 μg/mL to perform induction of expression. Further, by performing induction for about 1 to 2 days, a culture mixture containing the target protein can be obtained.

In a case where a Pichia yeast transformed with the recombinant vector having an AOX1 promoter is used, the yeast is precultured in BMGY medium for about 2 days, and then after exchanging the medium methanol is added to induce expression. Furthermore, by performing the culture for about 1 to 2 days at 30°C, a culture mixture containing the target protein can be obtained.

The transformant obtained by transforming a mammalian cell such as CHO cell with a recombinant vector having a promoter of EF1α is cultured in DMEM medium containing 10% fetal calf serum. The cells are inoculated in a density of about 1 to 10 $\times$ $10^4$ cells/mL, and cultured under the conditions of 37°C, and 5% carbon dioxide gas/95% air. Usually, after 2 to 3 days, the cells reach a confluent state and at this point in time the medium is exchanged with D-MEM not containing serum. Subsequently, by performing the culture for 2 to 3 days, a culture mixture containing the target protein can be obtained. Note that when the production amount of the target protein is small, it is possible to amplify the gene with methotrexate as described above to increase the production amount.

As for the method of purifying the peptide of the present invention from the culture mixture described above, a suitable method selected as appropriate from among methods that are usually used for purifying polypeptides is used. Specifically, suitable methods are selected from methods usually used including a salting out method, an ultrafiltration method, an isoelectric precipitation method, a gel filtration method, an electrophoresis method, various types of affinity chromatographies, such as an ion exchange chromatography, hydrophobic chromatography and antibody chromatography, a chromatofocusing method, adsorption chromatography, reverse phase chromatography, and the like and combined and optionally an HPLC system or the like may be used to perform purification.

In the production method, the polypeptide of the present invention may be expressed as a fused protein with β-galactosidase of E. coli or other polypeptides. In this case, in any one of steps in the process of purification, an operation is required for treating the fused protein with a chemical substance such as bromocyan or hydroxylamine or an enzyme such as protease to cut out the protein.

In a case where the transformant to be used is E. coli and the protein is to be produced as an inclusion body, which is an insoluble protein, operations of solubilizing, denaturing and refolding the inclusion body may be performed in a suitable step of purification (Thomas E, et al., J. Molecular Biology, 87, 563-577, 1974).

Specifically, first, bacterial cells are spalled and centrifuged and pellets are recovered. Then, a solubilizing buffer solution containing suitable amounts of urea or guanidine hydrochloride, a surfactant, reduced type glutathione, and oxidized glutathione(for example, a buffer solution containing 5 M guanidine hydrochloride, 0.005% Tween 80, 50 mM Tris hydrochloride (pH 8.0) 5 mM EDTA, 2 mM reduced type glutathione, and 0.02 mM oxidized glutathione) is added to the pellets and 2-mercaptoethanol is added to denature the protein and the mixture is dialyzed against a solution comprising the above-mentioned solubilizing buffer solution minus guanidine hydrochloride to refold the protein. When the protein is expressed as a fused protein, after these operations, unnecessary part of the protein is cut off with a chemical substance such as bromocyan or an enzyme such as protease, and then suitable chromatography is performed.

By the production method, the polypeptide of the present invention can be mass-produced uniformly and at high efficiency on an industrial scale.

A tenth embodiment of the present invention relates to a CD14 low molecular weight form having a molecular weight of 36±5 kDa obtainable from plasma.

The CD14 low molecular weight form of the present invention has a molecular weight of about 36±5 kDa.

The CD14 low molecular weight form of the present invention is obtainable from plasma. The term "obtainable from plasma" means molecules that can be actually obtained from plasma but the actual preparation method is not limited. It includes, for example, molecules actually obtained from plasma, molecules obtained from a material other than plasma, or molecules obtained by genetic recombination, as far as they are molecules that can be actually obtained from plasma. For example, it includes those molecules obtained from body fluid such as urine, or milk.

The CD14 low molecular weight form of the present invention can be substantially purified as follows.

By using anti-CD14 antibody that recognizes an amino terminal, all the molecule species polypeptides of soluble type CD14 are bound and eluted by an affinity chromatography method to obtain all the molecule species polypeptides of soluble type CD14 in blood. Then, by using anti-CD14 antibody that has been confirmed to bind to only high molecular weight species out of all the molecule species of soluble type CD14, only high molecular weight species polypeptide of soluble type CD14 in blood can be absorbed and removed to thereby substantially purify CD14 low molecular weight form.

The anti-CD14 antibody that recognizes an amino terminal includes, for example, 3C10 and MEM-18 antibodies. Also, the anti-CD14 antibody that has been confirmed to bind to only high molecular weight species includes F1024-1-3, F1033-3-1, and F1025-3-1 antibodies. The transformants producing F1033-3-1 and F1025-3-1 antibodies have been deposited at National Institute of Bioscience and Human-Technology at 1-3, Higashi 1-chome, Tsukuba-shi, Ibaraki-ken, Japan on February 9, 1999 and March 30, 1999, respectively, (Accession Nos. P-17209 and P-17350, respectively) and transferred from the original deposits to international deposits (Accession Nos. FERM BP-7295 and FERM BP-7296, respectively) on September 11, 2000.

Also, it is possible to perform the purification from all the molecular weight species of CD14 by selecting as appropriate suitable methods from those methods usually used for the purification of polypeptide. Specifically, suitable methods are selected and combined from those methods usually used including a salting out method, an ultrafiltration method, an isoelectric precipitation method, a gel filtration method, an electrophoresis method, an ion exchange chromatography, hydrophobic chromatography, other types of chromatographies, a chromatofocusing method, adsorption chromatography, reverse phase chromatography and the like, and optionally an HPLC system or the like may be used to perform purification.

The CD14 low molecular form of the present invention has a function of inhibiting the binding between CD14 and TLR; that is, it is a binding inhibitor for CD14 and TLR. This indicates that it has a function of suppressing human CD14-mediated cell activation, so that it can be used in the therapy or prevention of diseases associated with bacterial infection. For example, it is useful in the therapy or prevention of symptoms accompanying an increase in concentration of inflammatory cytokines, in particular blood TNF, associated with diseases such as sepsis. More specifically, it is useful in the therapy or prevention of fever, hypotension, leukocytopenia, thrombocytepenia, a shock, and multiple organ insufficiency. Further, the causative agent of cell activities that the antibody of the present invention suppresses is not limited to LPS alone. The antibody of the present invention also suppresses the cell activation mediated by CD14 that has formed a complex with, for example, LTA, PGN, mycoplasma or the like. That is, in diseases caused by these substances, the antibody of the present invention is useful in the therapy or prevention thereof as described above.

As will be apparent from the above-mentioned purification method, the CD14 low molecular form of the present invention has a part of the amino acid sequence of CD14. That is, it has a part that coincides with the amino acid sequence of the polypeptide according to the fifth embodiment of the present invention.

Furthermore, the molecular weight of the CD14 low molecular weight form is between the molecular weight of sCD(1-285) and that of sCD(1-246). From this it follows that in the CD14 low molecular weight form, the N-terminal of the CD14 is in the vicinity of positions 1 to 6 and the C-terminal of the CD14 in the vicinity of positions 246 to 285.

The CD14 low molecular weight of the present invention has a function of inhibiting the binding between CD14 and TLR. Further, the CD14 itself does not induce cytokines in endothelial cells in the presence of LPS, and suppresses production of cytokines in endothelial cells through exogenous LPS/CD14.

An eleventh embodiment of the present invention relates to a binding inhibitor for CD14 and TLR comprising an antibody, antibody fragment, polypeptide or CD14 low molecular weight form.

The binding inhibitor for CD14 and TLR of the present invention contains any one of an antibody, polypeptide and CD14 low molecular weight form that inhibits the binding between CD14 and TLR.

The binding inhibitor for CD14 and TLR of the present invention is not particularly limited as far as it is an agent for inhibiting CD14 to directly bind to TLR or an agent for inhibiting CD14 to bind to TLR through a third substance to form a complex and so on. Also, the mechanism of inhibition is not limited, either.

The binding inhibitor for CD14 and TLR contained in the pharmaceutical composition of the present invention preferably is an agent that has an inhibiting power of suppressing cell activation of TLR expressing cells by 30% or more. More specifically, in assay system, it is preferred that an inhibitor in a concentration of 10 μg/mL or lower suppresses the activation of NF-κB or production of IL-8 in TLR expressing cells in the presence of 1 μg/mL of LPS and 0.5 μg/mL of exogenous CD14 by 30% or more. More preferably, it is an inhibitor that suppresses by 40% or more. Further preferably, it is an inhibitor that suppresses by 50% or more. Particularly preferably, it is an inhibitor that suppresses by 70% or more.

Specific examples of the binding inhibitor for CD14 and TLR of the present invention include the antibody according to the first embodiment of the present invention, antibody fragment thereto, the polypeptide according to the fifth embodiment of the present invention and the CD14 low molecular weight form according to the tenth embodiment of the present invention.

The binding inhibitor for CD14 and TLR of the present invention inhibits signal transduction from CD14 to TLR by inhibiting the binding between CD14 and TLR, and hence signal transduction downstream of TLR is blocked, so that cell activation is suppressed to exhibit a therapeutic effect of sepsis.

That is, the binding inhibitor for CD14 and TLR of the present invention is useful in the therapy or prevention of diseases related to bacteria infection. For example, it is useful in the therapy or prevention of sepsis, articular rheumatism, AIDS, autoimmune diseases, hemolytic anemia, tumors, atopic diseases, allergic diseases, sarcoidosis, malaria, psoriasis, fever, hypotension, ischemic heart disease, leukocytopenia, thrombocytopenia, a shock, and multiple organ insufficiency. In particular, it is useful in the therapy and prevention of symptoms due to an increase in inflammatory cytokines accompanying these diseases, in particular in blood TNF level. Among these diseases, it is useful in the therapy and prevention of gram-negative bacteria infection in which LPS participates, gram-positive bacteria infection in which LTA, peptide glycan or the like participates, or sepsis accompanying mycoplasma. That is, it is useful not only for exhibiting therapeutic effects when symptoms of these diseases have appeared or are in progress but also for exhibiting preventive effects for those patients who contain LPS, LTA, mycoplasma or the like at high levels in blood or those persons infected with bacteria who are expected to come under such a circumstance.

A twelfth embodiment of the present invention relates to a method of screening a medicament for treating sepsis, comprising the step of contacting a cell expressing TLR prepared by a genetic recombination method with CD14 and a test substance.

The "method of screening a medicament for treating sepsis" is a method for studying to see whether or not a test substance is a medicament for treating sepsis or a candidate substance of a medicament for treating sepsis. Also, it is a method of selecting a medicament for treating sepsis or a candidate substance of a medicament for treating sepsis from a plurality of test substances.

The screening method of the present invention comprises the step of contacting a cell expressing TLR prepared by a genetic recombination method with CD14 and a test substance and detecting a change in a specified index to judge whether or not the test substance can be a candidate of a medicament for treating sepsis.

The CD14 source used in the screening method of the present invention includes CD14 expressed in cells, sCD14 existing in blood or sCD14 prepared by a genetic recombination method. The CD14 expressed in cells may be expressed in cells by a genetic recombination method. Further, sCD14 may be a modified one as far as it contains at least the region from the positions 269 to 315 of human CD14. It is preferably full length CD14 in consideration of maintenance of three-dimensional structure of CD14.

Cells that express TLR prepared by a genetic recombination method are not limited particularly as far as they are cells that express TLR prepared by a genetic recombination method. As for the TLR source, cloned TLR described in prior art may be used in a genetic recombination method. It is preferably TLR2 or TLR4.

Alternatively, the CD14 source may exist in the cells that express TLR.

In addition, it is preferred that in the step of contacting the cells expressing TLR prepared by a genetic recombination method with CD14 and a test substance, a CD14 activating substance is added simultaneously. This is because the CD14 activated by the CD14 activating substance readily binds to TLR.

The CD14 activating substance is not particularly limited as far as it binds to CD14 to form a complex and has a function of activating CD14. For example, it includes bacterial cell components such as LPS or LTA, mycoplasma, and the like.

The step of contacting the cells expressing TLR prepared by a genetic recombination method with CD14 and a test substance is not particularly limited as far as the step allows them to be contacted. It includes, for example, directly contacting in a solution, fixing CD14 or cells expressing TLR prepared by a genetic recombination to a plate or the like'and mixing the others in a solution, followed by adding the solution to the plate, or adding in a culture medium of CD14 or the cells expressing TLR the others and so forth.

The order of contacting the cells expressing TLR prepared by a genetic recombination method with CD14 and a test substance is not particularly limited. For example, it includes an order in which these may be contacted simultaneously, an order in which the CD14 activating substance and CD14 are contacted in advance, an order in which the CD14 activating substance and CD14 are contacted with the test substance before the CD14 activating substance and CD14 are contacted with the cells expressing TLR prepared by a genetic recombination method and the like.

The screening method o'f the present invention may include other steps as far as they do not inhibit the screening method. Further, the order of additional steps is not particularly limited.

For example, the step of contacting serum derived from an animal may be included. The serum derived from an animal is not limited as far as it is derived from an animal. Preferred examples of the animal include mammals including humans. Furthermore, humans, rodents, rabbits, oxen and the like are preferred. The binding between CD14 and TLR includes direct binding, binding through other factors or binding neutralized by other factors. This is because there occurs binding between CD14 and TLR to be activated in the presence of such serum.

The order of steps of contacting serum derived from an animal is not particularly limited. It includes simultaneous contacting together with other steps, contacting with TLR in advance, and the like.

The test substance is not particularly limited. Examples thereof include antibodies such as anti-CD14 antibody, polypeptides such as CD14 mutant polypeptide, low molecular weight substances and the like.

By the screening method of the present invention, medicaments for treating sepsis can be selected conveniently. That is, the screening method of the present invention is useful as a method of conveniently screening active ingredients for pharmaceutical compositions for sepsis.

Hereinafter, specific examples of the screening method will be described.

(1) To HEK293 cells or the like caused to constantly express TLR by a genetic recombination method are added given amounts of LPS and soluble type CD14.(1-356) and further a test substance. The cells are cultured for a fixed time period when the amount of cytokines produced in the supernatant is measured to judge a test substance, thus performing screening. Alternatively, a method in which instead of measuring the amount of cytokines, activation of intracellular NF-κB is determined by using reporter gene assay may be used.

(2) A method in which the binding between CD14 and TLR expressing cells prepared by a genetic recombinant method is directly detected by an interaction detecting device utilizing an ELISA method or surface plasmon resonance method may also be used. For example, an apparatus available from BIACORE AB and the like may be exemplified.

Specifically, CD14 is immobilized onto a plate or a chip in a BIACORE apparatus, to which LPS, TLR expressing cells prepared by a genetic recombination method, and a test substance are added. The amount of the TLR expressing cells prepared by a genetic recombination method bound to CD14 is measured by using a labeled antibody or the like in an ELISA method or directly measured when using an apparatus available from BIACORE AB or the like and the test substance is judged and screened.

TLR is a membrane protein. As means for analyzing its function, a method in which an extracellular region is prepared by genetic engineering and use it for a study may be conceived. In this case, however, a study is required as to whether or not the activity is maintained with the extracellular region alone. For this reason, the screening method of the present invention, which utilizes cells capable of expressing it in full length on the membrane is superior to others in that it can circumvent that problem.

The judgment as to whether the test substance is a medicament for treating sepsis is not particularly limited. It includes, for example, directly observing the binding between CD14 and TLR, and judging the test substance is a medicament for treating sepsis or selecting it as a medicament for treating sepsis, if the test substance inhibits the binding, or depending on the degree of inhibition of the binding. Alternatively, it includes measuring activation of CD14 or of TLR expressing cells and making such a judgement or selection.

For example, in the system (1) above, a test substance that suppresses the activation of NF-κB or IL-8 production of TLR expressing cells by 30% or more in a concentration of 10 μg/mL in the presence of 1 μg/mL of LPS and 0.5 μg/mL of CD14(1-356) is judged as a medicament for treating sepsis.

Examples of substances that can be judged'as medicaments for treating sepsis in the screening method of the present invention will be shown below.

As for the polypeptides, such examples include modified CD14s having partly or entirely modified amino acids at positions 269 to 315 of CD14 and peptides containing a part of the modified CD14s having such a modified portion. These function as medicaments for treating sepsis by inhibiting the binding of CD14 to TLR by way of competitive inhibition or the like.

They can be obtained by preparing plasmids of modified CD14s (N-terminal deletions, C-terminal deletions, N-terminal- and C-terminal-deletion products, intermediate-deletion products, amino acid substitution products, amino acid addition products, amino acid modification products, or modified products comprising these in combination, peptides obtained by partly tailoring CD14) by genetic manipulation ("New Cell Engineering Experiments Protocols," Ed. Department of Carcinostatic Research, The Institute of Medical Science, The University of Tokyo, Shujunsha) and transfecting them to COS-1 cells by a conventional method to express CD14 tailored products.

As for the antibody, such examples include antibodies that recognize sCD14(269-315), antibodies that recognize sCD14(1-315) but does not recognize sCD14 (1-269) and so on. These antibodies can be obtained by the method of preparing anitibodies according to the present invention.

Also, a peptide may be designed by drug design as described below.

By conducting the screening method of the present invention using low molecular weight compounds as test substances, medicaments for treating sepsis can be selected.

However, test substances are not particularly limited. Test substances provided at random may also be used. For example, performing drug design in advance as described below is preferred in view of the efficiency of screening.

Drug design can be practiced by obtaining the manner of binding between CD14 and TLR or the manner of inhibiting the binding between the polypeptide or antibody of the present to CD14 and TLR as information on three-dimensional interaction.

Information on three-dimensional interactions can be obtained by X-ray structure analysis or synchrotron radiation structure analysis of co-crystals of CD14 and TLR, co-crystals of the polypeptide of the present invention and CD14 or TLR, or co-crystals of the antibody of the present invention and CD14. In addition, they can be obtained by techniques such as NMR, electromicroscopy, computer simulation and the like.

Examples of practicing drug design with the information on three-dimensional interactions are described below.

The peptide region where CD14 and TLR interact with each other, which is revealed from the information on three-dimensional interactions can serve as an antagonist or agonist by itself. Furthermore, peptides having an increased activity can be searched by using techniques such as phage display, combinatorial synthesis and the like. Also, by supposing active conformation of a peptide from the crystal structure thereof and performing peptide mimic-like synthesis, molecules with a reduced molecular weight can be obtained.

Furthermore, by using the structure of the binding site, a compound that matches the shape or properties of the protein of CD14 or TLR can be screed on a computer by using software such as, for example, DOCK (University of California, San Francisco, CA), FlexX (Tripos, Inc. St. Louis, MO) or the like.

Furthermore, it is possible to predict a three-dimensional pharmacore by using the arrangement of requisite active residues. By using the pharmacore as a search formula, and retrieving compounds data base of already-existing Available Chemicals Directory (ACD) (MDL information systems, Inc., San Leandro, CA) and the like with software such as Catalyst (Molecular Simulations, Inc., Sandiego, CA), Unity (Tripos, Inc., St. Louis, MO) and the like, it is possible to construct a focused library of candidate agonist and antagonist of low molecular weight compounds so that compounds that can exhibit pharmacological actions can be searched efficiently.

As other examples, it is also possible to perform de novo design of compounds by using software, for example, Ludi (Molecular Simulation Inc., Sandiego, CA), LeapFrog (Tripos, Inc., St. Louis, MO) or the like.

Furthermore, by procuring three-dimensional information on the characteristics common in a plurality of compounds, polypeptides of the present invention or antibodies of the present invention obtained by the above drug design or the like and judged as medicaments for treating sepsis or candidate substances of medicaments for treating sepsis it becomes possible to provide three-dimensional pharmacores of substances that have action of inhibiting the binding between CD14 and TLR.

The method of three-dimensionally procuring the characteristics in common can be performed by using software such as Catalyst.

Also, analysis of eutectic crystal structures of the compound having an inhibitory activity thus obtained with CD14, TLR or both enables one to perform drug design more precisely.

The CDR of the antibody of the present invention can be reduced in molecular weight by mimicking it by itself or making drug design of a compound having a function similar to that of neutral antibody.

The compounds thus searched are not limited to polypeptides, low molecular weight compounds or the like. In consideration of having advantages such as peroral absorbability and the like, it is considered that low molecular weight compounds is useful.

The screened low molecular weight compounds are useful as medicaments for treating sepsis. Furthermore, according to the description herein, a method of screening a binding inhibitor, which is a low molecular weight compound, for CD14 and TLR is shown.

The molecular weight of the binding inhibitor for CD14 and TLR, which is low molecular weight compound, is not particularly limited. The low molecular weight compounds generally include compounds having a molecular weight of about 100 and also compounds having a molecular weight of about 10,000 such as antibiotics.

Also, the present invention discloses a method of screening a substance interacting with a region at positions 269 to 315 of human CD14 described in SEQ ID NO:1.

The screening of a substance interacting with the region at positions 269 to 315 of human CD14 refers to a method of studying as to whether or not a test substance interacts with the region at positions 269 to 315 of human CD14. Also, it refers to a method of selecting a substance that interacts with the region at positions 269 to 315 of human CD14 from a plurality-of test substances.

The screening method of the present invention includes the following steps 1 and 2 below.

Step 1. Contacting a polypeptide containing 6 or more amino acids from the region at positions 269 to 315 of human CD14 with a test substance; and

Step 2. Judging if the test substance interacts with the region at positions 269 to 315 of human CD14.

The polypeptide containing 6 or more amino acids from the region at positions 269 to 315 of human CD14 is not particularly limited as far as it includes 6 or more amino acids from the region at positions 269 to 315 of human CD14. For example, the peptide of the present invention is exemplified. Further, it may include an amino acid sequence other than the above amino acid sequence. This is because it suffices to further confirm as to whether or not a polypeptide consisting of amino acid sequence other than that amino acid sequence interacts with amino acid sequence other than the region.

The step of contacting the polypeptide with the test substance is not particularly limited as far as they are contacted therein. For example, they are contacted by adding both the substances in a solution or immobilizing the polypeptide to a plate or the like and adding the test substance to the plate and so on.

The step of judging whether or not the test substance interacts with the region at positions 269 to 315 of human CD14 is not particularly limited as far as a judgment can be made therein if the substance interact with the region at positions 269 to 315 of human CD14. For example, judgment by measuring the binding between the polypeptide and the test substance is exemplified.

By the screening method of the present invention, substances that interact with the region at positions 269 to 315 of human CD14 can be selected conveniently. The region at positions 269 to 315 of human CD14 is a region that the antibody of the present invention recognizes and a region that can interact with other proteins of CD14 as described in the first embodiment of the present invention, and the substance that interacts with that region may serve as a substance that controls the interaction of CD14 and other proteins. For example, in a case where the other protein is TLR, the substance that interacts with the region inhibits the interaction or binding between CD14 and TLR. Thus, the substance that controls the function of CD14 is useful as an active ingredient of a pharmaceutical composition for sepsis. That is, the screening method of the present invention is useful as a method of conveniently screening an active ingredient of a pharmaceutical composition for sepsis.

Hereinafter specific examples of the screening method will be illustrated.

(1) Screening of antibodies

A method of screening an antibody that interacts with the region at positions 269 to 315 of CD14; that is, an antibody that recognizes that region will be described.

Method [1]: First, sCD14 (1-356) is immobilized to a plate, a liquid such as culture supernatant containing a test antibody is mixed with a synthetic peptide that is prepared from 47 amino acids of amino acids at positions 269 to 315 of CD14, and the mixture is added to the plate. The antibody that recognizes the region at positions 269 to 315 of human CD14 is inhibited from binding to the solid phase sCD14(1-356) by the synthetic peptide, and therefore, it is judged that it interacts with the region at positions 269 to 315 of human CD14.

Method [2]: A synthetic peptide having the sequence composed of the region at positions 269 to 315 of CD14 is immobilized to a plate and a liquid such as culture supernatant containing a test antibody is added to the plate. The antibody that recognizes the region at positions 269 to 315 of CD14 binds to the synthetic peptide, and therefore, it is judged that it interacts with the region at positions 269 to 315 of human CD14.

Method [3]: A synthetic peptide having the sequence consisting of amino acids at positions 1 to 268 of CD14 and a synthetic peptide having the sequence consisting of amino acids at positions 1 to 315 of CD14 are immobilized to a plate and a liquid such as culture supernatant containing a test antibody is added to the plate. The antibody that recognizes the region at positions 269 to 315 of CD14 does not bind to the synthetic peptide having the sequence consisting of amino acids at positions 1 to 268 of CD14 but binds to the synthetic peptide having the sequence of amino acids at positions 1 to 315 of CD14, and therefore, it is judged to interact with the region at positions 269 to 315 of human CD14.

Furthermore, as for the antibodies obtained by the methods [1] to [3] above and the like, it can be confirmed whether or not they inhibit the function of CD14 by the method described in Example 2 herein or the like.

(2) Screening of low molecular weight compounds

As the method of screening low molecular weight compounds that interact with the region at positions 269 to 315 of CD14, the following screening methods are exemplified.

It is desirable that the obtained low molecular weight compounds have agonistic activity or antagonistic activity for CD14.

Method [1]: On a chip'of BIACORE 2000(BIACORE, AB.), SCD14(1-268) and sCD14(1-315) are separately immobilized and a test low molecular weight compound is added to the chip. An analyte that binds to CD14(1-315) but does not bind to sCD14(1-268) is judged to interact with the region at positions 269 to 315 of human CD14. This indicates that the low molecular weight compound that binds to sCD14(1-315) has strong affinity for the region at positions 269 to 315 thereof.

Method [2]: sCD14(1-356) containing amino acids at positions 269 to 315 of CD14 is immobilized to a plate and a test low molecular weight compound is added to cause reaction. After completion of the reaction, the plate is once washed to remove the non-bound low molecular compounds, and then, F1024-1-3 antibody is added to cause reaction. Since the test substance that interacts with the region at positions 269 to 315 of human CD14 inhibits the binding of F1024-1-3 to sCD14(1-356), it is judged that the test substance interacts with the region at positions 269 to 315 of CD14.

As for the substances obtained by the screening method of the present invention, it can be confirmed whether or not they inhibit the function of CD14 by the method described in Example 2 or the like. Further, by the screening method of the present invention, it can be confirmed as to whether or not they are medicaments for treating sepsis.

Furthermore, the present invention also discloses a method of screening CD14 mutants having CD14 antagonistic function or CD14 agonistic function.

The screening of CD14 mutants having a CD14 antagonistic function or CD14 agonistic function refers to a method of studying as to whether or not a test substance has a CD14 antagonistic function or CD14 agonistic function.

CD14 has functions of binding to LPS, transducing signals to cells, and activating cells. At present, it is unclear as to whether or not CD14 mutants that is randomly mutagenized have an agonistic effect or antagonistic effect; by the screening method of the present invention, mutants having an antagonistic effect but no agonistic effect or mutants having an agonistic effect but no antagonistic effect can be screened.

The screening method of the present invention comprises Step 1 and Step 2 as described below.

Step 1. Contacting LPS and a test substance, with cells expressing no CD14.
Step 2. Contacting LPS and a test substance, with CD14.

In Step 1, the cells expressing no CD14 include, for example, endothelial cells. By contacting LPS and a test substance with endothelial cells, for example, and measuring the amount of cytokine production, it can be judged as to the presence or absence of agonistic effect.

In Step 2, the CD14 source includes CD14 expressed in cells, sCD14 that exists in blood and the like, or sCD14 prepared by a genetic recombination method. The CD14 expressed in cells may be expressed in cells by a genetic recombination method. In the case of sCD14, it is further contacted with cells that do not express CD14. By measuring the amount of cytokine production from endothelial cells in the same manner as in Step 1, it can be judged as to the presence or absence of antagonistic effect.

In both the steps above, the present invention is not limited to measurement of amount of cytokine production but measurement by a reporter assay by using recombinant cells may be also performed.

In the screening method of the present invention, a test substance having no agonistic effect in Step 1 but having an antagonistic effect in Step 2 serves as a CD14 antagonist and is useful as a medicament for treating sepsis and an active ingredient of a medicament for treating sepsis.

A twelfth embodiment of the present invention relates to an anti-CD14 antibody, a CD14 mutant or low molecular weight compound.

The anti-CD14 antibody, CD14 mutant or low molecular weight compound according to the twelfth embodiment of the present invention are those obtained by the screening method according to the eleventh embodiment of the present invention. The anti-CD14 antibody, CD14 mutant or low molecular weight compound according to the twelfth embodiment of the present invention are those selected as medicaments for treating sepsis by screening, as described in the eleventh embodiment of the present invention, and they are useful as medicaments for treating sepsis or active ingredients of medicaments for treating sepsis.

A thirteenth embodiment of the present invention relates to a pharmaceutical composition for sepsis containing a binding inhibitor for CD14 and TLR as an active ingredient.

The binding inhibitor for CD14 and TLR contained in the pharmaceutical composition of the present invention is not particularly limited as far as it is an agent that inhibits directly binding of CD14 to TLR or an agent that inhibits CD14 from forming a complex or the like by binding to TLR through a third substance. The mechanism of inhibition is not limited either.

It is preferred that the binding inhibitor for CD14 and TLR contained in the pharmaceutical composition of the present invention has a titer at which activation of TLR expressing cells is suppressed by 30% or more. More specifically, in an assay system, the inhibitor with a concentration of 10 μg/mL or less has a titer at which activation of NF-κB or IL-8 production of TLR expressing cells in the presence of 1 μg/mL of LPS and 0.5 μg/mL of exogenous CD14 is suppressed by preferably 30% or more. More preferably, it has a titer at which the activation is suppressed by 40% or more. Further preferably, it has a titer at which the activation is suppressed by 50% or more. Particularly preferably, it has a titer at which the activation by 70% or more.

The molecular form of the binding inhibitor for CD14 and TLR contained in the pharmaceutical composition for sepsis of the present invention as an active ingredient is not particularly limited. It includes, for example, antibody, fragments of antibody, polypeptide, protein and low molecular weight compounds.

As the antibody, fragments of antibody, polypeptide, and protein, the binding inhibitor according to the eleventh embodiment of the present invention is exemplified. The low molecular weight compound is one described in the twelfth embodiment of the present invention.

In the route of sepsis that is caused by cell activation through human CD14, the binding inhibitor for CD14 and TLR inhibits the binding of CD14 with TLR, thereby inhibiting signal transduction that is performed from CD14 to TLR. As a result, it blocks the signal transduction subsequent to TLR, to thereby suppress cell activation, thus exhibiting therapeutic effect for sepsis.

That is, the pharmaceutical composition of the present invention is useful for the therapy and the prevention of diseases associated with bacterial infection. For example, it is useful for sepsis, articular rheumatism, AIDS, autoimmune diseases, hemolytic anemia, tumors, atopic diseases, allergic diseases, sarcoidosis, malaria, psoriasis, fever, hypotension, ischemic heart disease, leukocyto failure, thrombocytopenia, a shock, and multiple organ insufficiency. In particular, it is useful in the therapy and the prevention of symptoms that are caused due to an increase in inflammatory cytokines accompanying these diseases, in particular in blood TNF level. Among these diseases, it is useful in the therapy and the prevention for sepsis of gram-negative bacteria infection with participation of LPS, gram-positive bacteria infection with participation of LTA, peptide glycan or mycoplasma infection. That is, it is useful not only for exhibiting therapeutic effects at the time when symptoms of these diseases have appeared or are in progress but also for exhibiting preventive effects for those patients who contain LPS, LTA, mycoplasma or the like at high levels in blood or those persons infected with bacteria who are suspected to bring into such a circumstance.

Further, as far as it contains the binding inhibitor for CD14 and TLR as an active ingredient various additives that are pharmaceutically acceptable may be included. For example, carriers, excipients, stabilizers, lubricants, colorants, disintegrants, septics, isotonic agents, stabilizers, dispersants, antioxidants, buffers, preservatives, suspending agents, emulsifiers, commonly used suitable solvents (sterilized water, plant oil, etc.), and further dissolution aids that are physiologically acceptable and the like may be selected appropriately.

The pharmaceutical composition of the present invention may contain antibiotics, steroids, various cytokine antibodies, anticoagulation factors or the like. These substances may exhibit additive or synergistic effects together with the antibody of the present invention as the ingredient, thereby to give a more effective pharmaceutical composition.

The dosage in the case of administering the pharmaceutical composition of the present invention is not particularly limited. For example, when the antibody of the present invention is used as the active ingredient, it is preferred that the dosage is 0.1 mg/kg or more. More preferably, the dosage is 1 to 10 mg/kg. In the case where the pharmaceutical composition of the present invention is used as a drug, it is preferred that the preparation form includes suppositories, inhalants, and in particular injections. Also, there are various administration routes applicable. However, parenteral administration is preferred. AS for parenteral administration, injections such as intravenous administration, intraarterial administration, subcutaneous administration, and intramuscular administration are generally used. Other examples include intrarectal administration, percutaneous absorption, permucous absorption and the like. The time or number of administration includes preventive administration single administration, and continuous administration or the like depending on the condition of patient.

Note that no acute toxicity is indicated in a dosage of 10 mg/kg with respect to an animal.

EXAMPLES

Hereinafter, the present invention will be explained in more detail by examples. However, they are indicated as only examples and the present invention should not be limited thereto. In the following description, abbreviations used are based on those abbreviations commonly used in the art. Note that TLR4 will be sometimes described as TOLL4 (To114).

(Example 1)

Preparation of anti-human CD14 antibody

(1) [Preparation of antigen to be administered]

5 mg of purified anti-human CD14 monoclonal antibody 3C10 (purchased from ATCC and prepared and purified by ordinary methods) was bound to Hitrap NHS-Activated resin (Amersham Pharmacia) according to the manual to prepare a 3C10-bound affinity column.

As for soluble type CD14, 100 mL of human serum (purchased from Japan Biotest) was added to the prepared column and continuously washed with phosphate buffer (pH 7.4) (hereinafter, referred to as PBS).

After confirming the reduction of the absorbance at a wavelength of 280 nm, the column was eluted with 0.1 M glycine hydrochloride buffer (pH 3.0) and the eluate was concentrated by ultrafiltration using Diaflow (Grace Japan). After dialysis with PBS, the concentration of protein was calculated with the absorbance at a wavelength of 280 nm (coefficient: 1 O.D.=1 mg/mL). As a result, about 200 μg of purified soluble type CD14 was obtained and a band of about 55 kD was confirmed by SDS-PAGE analysis.

(2) [Preparation of anti-CD14 monoclonal antibody]

To the footpad of a female Wistar rat (purchased form SLC) aged 8 weeks was administered a 1:1 mixture consisting of 100 μg of the purified soluble type CD14 and Freund's complete adjuvant (Difco). After 3 weeks, ilium lymph node was extracted and lymphocytes were aseptically collected.

The obtained lymphocytes were mixed with murine myeloma cell SP2/O-Ag14 (ATCC) in a ratio of 5:1 and cells fusion was performed according to a method described on Ando, Tamie and Chiba, Takeshi: "Introduction to Monoclonal Antibody Experimental Manipulation", page 83, 1991 (Kodansha) with polyethylene glycol 1500 (Sigma). After the cell fusion, the cells were suspended in 10% fetal bovine serum/RPMI1640 containing hypoxanthine, aminopterin and thymidine and inoculated in wells of a 96-well plate (Nunc).

The cells were cultured under the conditions of 5% $CO_2$ at 37°C until a stage where growth of hybridomas was confirmed when the medium was exchanged to the same medium as above but contained no aminopterin.

After I week from the cell fusion, the culture supernatant was sampled and hybridomas producing antibody binding to CD14 were screened by using a plate having immobilized thereto purified soluble type CD14. That is, 1 μg/mL of purified soluble type CD14 was immobilized to a plate (Maxisorp, Nunc) and blocked with 0.1% bovine serum albumin-containing PBS. Then, the culture supernatant was added thereto and reacted at 37°C for 1 hour and thereafter washed with 0.9% physiological saline containing 0.05% Tween-20.

To each well, a peroxidase-labeled anti-rat immunoglobulin antibody (DAKO) was added and reacted at 37°C for 1 hour. After the washing, tetramethylbenzidine color developer solution containing 0.02% hydrogen peroxide was added. After 10 minutes' reaction, the reaction was terminated with 0.5 M sulfuric acid.

The absorbance of the plate was measured at a wavelength of 450 nm and wells having an absorbance of 0.2 or higher were selected as anti-CD14 antibody-producing hybridomas.

The selected hybridomas were cloned by a limiting dilution method (Ando, Tamie and Chiba, Takeshi: "Introduction to Monoclonal Antibody Experimental Manipulation", Kodansha). After 10 days, screening was performed in the same manner as above to obtain 17 clones of anti-CD14 antibody-producing hybridomas.

Then, after the hybridomas were cultured in RPMI1640 containing 10% fetal bovine serum, cells were recovered and production of antibody was performed by culturing the recovered cells in Hybridoma-SFM (Gibco) to obtain supernatant containing monoclonal antibody. After removing cells from the culture supernatant through filter paper, the culture supernatant was purified through Protein G column (Prosep-G, Millipore) to obtain 17 kinds of purified anti-human CD14 antibodies.

(Example 2)

Screening of anti-human CD14 antibody for a medicament for treating sepsis.

(1) [Preparation of a screening system]

[1] Construction of human TLR4 expression plasmid

Since human TLR4 cDNA has a coding region of about 2.5 kb (Genbank Accession No. AF17765), cloning of TLR4 cDNA was performed separately for 1.1 kb on the 5'-flanking region and for 2.3 kb on the 3'-flanking region. cDNA of human TLR4 was cloned by the following method. That is, sense primer 1 (SEQ ID NO:7), sense primer 2 (SEQ ID NO:8), antisense primer 1 (SEQ ID NO:9) and antisense primer 2 (SEQ ID NO:10) were designed from a human TLR4 genome sequence (GenBank: Accession No. AF177765). First, using sense primer 1 and antisense primer 1 and also human lung cDNA (CLONTECH Co.) as a template, PCR reaction was performed with Pyrobest DNA Polymerase(TaKaRa Co., Ltd.) by repeating 30 times the cycle of 98°C for 10 seconds, 55°C for 30 seconds and 72°C for 1 minute. Similarly, using sense primer 2 and antisense primer 2 and also human spleen cDNA (CLONTECH Co.) as a template, PCR was performed. The obtained about 1.1 kb and about 2.3 kb DNA fragments were each phosphorylated at the 5'-terminal with T4 Polynucleotide kinase (TaKaRa Co. Ltd.). On the other hand, a vector was prepared by digesting pBluescriptIISK(+) (STRATGENE Co.) with EcoRV followed by dephosphorylation and the above PCR products were each ligated thereto. Thereafter, using competent cell JM109 (TaKaRa Co., Ltd.), transformation was performed by a conventional method to obtain a plasmid (pT45F) having a fragment of the 5'-flanking region of TLR4 cDNA and a plasmid (pT43F) having a fragment of the 3'-flanking region of TLR4 cDNA. Then, PT45F was double digested with XhoI and EcoRI and pT43F was double digested with EcoRI and HindIII and inserted to the XhoI/HindIII site of pBluescriptIISK(+) (three-way ligation). Transformation of JM109 gave rise to a plasmid pBTLR4 containing full-length human TLR4 cDNA. Confirmation of the inserted sequence of pBTLR4 indicates that it coincided with the exon sequence in human TLR4 genome sequence. Further, in order to express human TLR4 in mammalian cells, human TLR4 cDNA was excised from pBTLR4 with HindIII and inserted into the HindIII site of pcDNA3.1(-) (Invitrogen Co.). Transformation of JM109 gave rise to plasmid pcDNAT4 that causes mammalian cells to express human TLR4.

[2] Establishment of human TLR4 expression transformant cell line

To establish a human TLR 4 expression transformant cell line, the expression plasmid pcDNAT4 prepared in [1] above was transfected to human fetal kidney derived cell line HEK293 by the following method. That is, after mixing 25 μL of FuGENE6 (Roche Diagnostics Co.) and 6.3 μg of PcDNAT4 according to the attached protocol, the mixture was added to HEK293 cells confluently grown in a 75 $cm^2$ Roux flask. On day next, the cells were scraped off from the Roux flask and inoculated again to a 96-well plate in a density of 250 cells/well. Further on day next, G-418 was added to the each well in a final concentration of 1.2 mg/mL and subsequently the medium was exchanged with a fresh medium containing 1.2 mg/mL of G-418 every 3 to 4 days and thus selective culture was performed for 20 days. The obtained 24 cell lines of G-481 resistant clones were studied as to the response to LPS/sCD14. The 24 kinds of clones were each inoculated to a 24-well plate in a density of 2.0 x $10^5$ cells/well. After culturing the cells at 37°C and 5% $CO_2$ for one day, a mixed solution consisting of 50 ng/well of pNFκB-Luc (CLONTECH Co.) and 1 μL/well of FuGENE6 was added to the wells and further the cells were cultured for 24 hours. Then, a mixture of 0.5 μg/mL of sCD14(1-356) (the preparation method was described in Example 8) and 1 μg/mL of LPS was added to stimulate the cells. After 6 hours, the supernatant was removed and the cells were washed with $PBS^-$ and the cells were lysed with 100 μL/well of Passive lysis Buffer(Promega Co.). The luciferase activity in 20 μL of cell extract was measured with Luciferase Assay Substrate (Promega Co.). For the measurement, 1420 ARVOsx multilabel counter (Wallac Co.) was used. As a result, a clone HEKT4-14 showing luciferase activity in the cell extract was obtained. Note that similar experiments were conducted using HEK293 but no luciferase activity was confirmed. The induction of luciferase activity with LPS/sCD14 in HEKT4-14 depended on expression of TLR4 (Fig. 1).

(2) [Screening of human CD14 antibody for a medicament for treating sepsis]

HEKT4-14 cells were suspended in a DMEM medium containing 10% inactivated FBS, inoculated in wells of 24-well plate in a density of 1 × $10^5$ cells/well and cultured for 24 hours under the conditions of 5% $CO_2$ and 37°C. Thereafter, using FuGENE6, 100 ng/well of a reporter gene pNFκB-Luc (CLONTECH Co.) was transfected, followed by further cultivation for 24hr. LPS (E. coli. 055:B5, Difco Co.) in a final concentration of 1 μg/mL, sCD14(1-356) in a final concentration of 0.5 μg/mL, and anti-CD14 antibody 3C10 or anti-CD14 antibody obtained in Example 1 in a final concentration of 1 to 10 μg/mL were added and culture was continued for 6 hours. Thereafter, the cells were lysed with Passive Lysis Buffer (Promega Co.) and the luciferase activity of the lysate was measured using Luciferase Assay Substrate (Promega Co.) according to the attached protocol. As a result, as the antibody having activity, F1024-1-3 antibody was obtained that inhibited activation of NF-κB by about 75% in a system in which 10 μg/mL of antibody was added as shown in Fig. 2.

Isotypes of F1024-1-3 antibody by typing Rat MonoAB ID/SP kit (ZYMED Co.) revealed IgG1/κ.

(Example 3)

Inhibiting activity of F1024-1-3 antibody on IL-6 produciton

Human vascular endothelial cell, HUVEC (Sanko Pure Chemicals Co., Ltd.) were scraped with PBS containing 0.05% trypsin and 0.53 mM EDTA and then suspended in RPMI1640 medium (Asahi Techno Glass Co., Ltd.) containing 2% human serum prepared by removing soluble type CD14 from serum from a healthy person with an anti-CD14 antibody (hereinafter, referred to as 2% CD14w/oHS/RPMI) and inoculated in wells of a 96-well plate in a density of 5 × $10^4$ cells/Well (50 μL/Well), followed by cultivation under the conditions of 37°C and 5% $CO_2$ for 24 hours.

10 μL of RPMI 1640 medium containing 14% of the human serum from which soluble type CD14 had been removed, 10 μL of 120 ng/mL LPS (E. coli 055:B5, Difco Co.), 10 μL of 3.6 μg/mL serum-derived soluble type CD14 and 40 μL of 900 ng/mL F1024-1-3 antibody or 3C10 antibody were added to the cells. The resulting mixture was cultivated for 20 hours and then IL-6 in the culture supernatant was measured by using human IL-6 EIA kit (Applied Biosystems, Inc.).

Measurement of IL-6 was performed according to the protocol attached to the human IL-6 EIA kit. That is, 100 μL of the culture supernatant was transferred to an IL-6 antibody-immobilized plate and incubated at 37°C for 60 minutes. Thereafter, the reaction mixture was removed and the plate was washed 4 times with 400 μL/well Wash Buffer 2 and then 100 μL/Well of anti-human IL-6 antibody was added to the plate, which was incubated at 37°C for 30 minutes.

The reaction mixture was removed and the plate was washed 4 times with 400 μL/Well Wash Buffer 2 and then 100 μL/Well of a solution of peroxidase-labeled streptoavidine was added to the plate, which was incubated at 37°C for 30 minutes.

After washing, 100 μL/Well of a color developing substrate (TMB) was added and the mixture was reacted at room temperature for 15 minutes and then the reaction was stopped by addition of 100 μL/Well of a stop solution. The absorbance at a wavelength of 450 nm was measured and the production amount of IL-6 in the sample was calculated. Note that rat IgG used as control antibody and 3C10 antibody were purified preparations.

The results obtained are shown in Fig. 3. The production amount of IL-6 when no antibody was added was taken as 100%. IL-6 production inhibiting activity was observed in F1024-1-3 antibody and 3C10 antibody. The inhibitory activity was 50% at 0.3 μg/mL in F1024-1-3 antibody in contrast to 10% in 3C10 antibody against the production amount of the control antibody, which indicates that F1024-1-3 antibody is superior to 3C10 antibody in the effect of inhibiting inflammatory cytokine production in endothelial cells.

Also, it is apparent that the substance selected by the screening in Example 2 actually suppresses cytokines in the cells.

(Example 4)

Measurement of suppression of IL-6 production after formation of LPS/CD14 complex by F1024-1-3 antibody

In the same manner as in Example 3, human vascular endothelial cells, HUVEC (Sanko Pure Chemicals Co., Ltd.) were suspended and inoculated in wells of a 96-well plate in a density of $5 \times 10^4$ cells/Well (50 μL/Well), followed by cultivation under the conditions of 37°C and 5% $CO_2$ for 24 hours. 10 μL of RPMI1640 medium containing 14% of the human serum from which soluble type CD14 had been removed, 10 μL of 120 ng/mL LPS (E. coli 055:B5, Difco Co.), and 10 μL of 3.6 μg/mL serum-derived soluble type CD14 were mixed and incubated at 37°C for 1 hour to form LPS/CD14 complex. Thereafter, 40 μL of 900 ng/mL F1024-1-3 antibody or 3C10 antibody was added to the LPS/CD14 mixture. After further 20 hours' cultivation, the IL-6 in the culture supernatant was measured by using human IL-6 EIA kit (Applied Biosystems, Inc.).

Measurement of IL-6 was performed according to the protocol attached to the human IL-6 EIA kit. Note that rat IgG (conAb) used as control antibody and 3C10 antibody were purified preparations.

The results obtained are shown in Fig. 4. The production amount of IL-6 when no antibody was added was taken as 100%. IL-6 production inhibiting activity was observed in F1024-1-3 antibody while in the case of 3C10 antibody this activity was on the same level as the control antibody and after formation of LPS/CD14 complex no effect of suppressing IL-6 production was observed. These indicate that F1024-1-3 antibody has the effect of inhibiting inflammatory cytokine production after formation of LPS/CD14 complex in endothelial cells; therefore, it meets expectation that it has the effect of improving the symptoms of even those patients in whom LPS has already invaded, i.e., those patients who have been judged to suffer from sepsis.

(Example 5)

Test of binding of F1024-1-3 antibody to LPS/CD14 complex

The influence of anti-CD14 antibody on the binding between CD14 and LPS on a cell membrane was analyzed by a flow cytometry method.

Human monocyte cell line, THP-1 was cultured with 40 ng/mL of 1α, 25-dihydroxyvitamin D3 (Funakoshi Co., Ltd.) for 48 hours to induce differentiation and then further incubated in a medium (RPMI1640 containing 10% FBS) containing or not containing 10 μg/mL of anti-CD14 antibody (3C10 or F1024-1-3 antibody) at 37°C for 30 minutes. Thereafter, FITC-labeled LPS (Sigma Co.) was added in a final concentration of 1 ng/mL and incubated at 37°C for 15 minutes. Immediately thereafter, an equivolume of ice-cooled RPMI1640 medium was added and the intensity of fluorescence was measured by using FACSCalibur (BD Co.).

The results obtained are shown in Fig. 5. 'As shown in Fig. 5, specific fluorescence observed as a result of the binding of LPS to CD14 was completely suppressed by 3C10 antibody. In contrast, F1024-1-3 suppressed it only partly, which indicates that F1024-1-3 antibody does not suppress the binding of LPS to CD14.

(Example 6)

Analysis of the recognition region of F1024-1-3 antibody

In order to clarify the region that F1024-1-3 antibody recognizes, experiments on inhibition by peptide, experiments on biding by CD14 C-terminal deletion mutant, and experiments on binding by CD14 amino acid substitution mutant were conducted.

(1) [Preparation of CD14 peptide]

Based on the amino acid sequence of CD14 described in SEQ ID NO:1, four peptides consisting of peptide A (SEQ ID NO:11), peptide B (SEQ ID NO:12), peptide C (SEQ ID NO:13), and peptide D (SEQ ID NO:14), and peptide E (SEQ ID NO:15) as a control were synthesized. That is, peptides were synthesized by using a peptide synthesizer (432A, Applied Biosystems) according to the method of using it, deprotected and cleaved according to conventional methods, and purified by HPLC by using a C18 column (CAPCELL-PAK, Shiseido). The purified fractions were recovered, freeze-dried and weighed and dissolved in distilled water to 10 mg/mL.

(2) Preparation of CD14 mutants with deletion of amino acids from human CD14 (human soluble type CD14 deletion mutants)

This was performed according to Example 8 described hereinbelow. That is, plasmids that cause mammalian cells to express recombinants with deletion of 49, 56, 61, 66, 71, 110, 173, and 204 amino acids from the C-terminal of human CD14 (hereinafter referred to sCD14(1-307), sCD14(1-300), SCD14(1-295), sCD14(1-290), sCD14(1-285), sCD14(1-246), sCD14(1-183), and sCD14(1-152), respectively) (sCD14(1-307) stands for a soluble polypeptide that has amino acids at positions 1 to 307 on the N-terminal of CD14 described in SEQ ID NO:1, hereinafter the same) were constructed and the plasmid were transfected into COS-1 cells, respectively, to obtain various human soluble type.

Also, plasmids that cause mammalian cells to express various types of human soluble type CD14 deletion mutants (hereinafter, deleted portion is indicated by Δ) Δ7-11, Δ57-64, Δ180-234, Δ235-282, and Δ180-282 were constructed in the same manner as the amino acid substitution modified polypeptide expressing plasmids in Example 9-(1). In this case, however, the primers used were sense primer 3 (SEQ ID NO:16), antisense primer 3 (SEQ ID NO:17), sense primer 4 (SEQ ID NO:18), and antisense primer 4 (SEQ ID NO:19) for Δ7-11, sense primer 3, antisense primer 5 (SEQ ID NO:20), sense primer 5 (SEQ ID NO:21), and antisense primer 4 for Δ57-64, sense primer 3, antisense primer 6 (SEQ ID NO:22), sense primer 6 (SEQ ID NO:23), and antisense primer 4 for Δ180-234, sense primer 3, antisense primer 7 (SEQ ID NO:24), sense primer 7 (SEQ ID NO:25), and antisense primer 4 for Δ235-282, and sense primer 3, antisense primer 8 (SEQ ID NO:26), sense primer 8 (SEQ ID NO:27), and antisense primer 4 for Δ180-282, respectively. Then, the obtained plasmids were transfected into COS-1 cells according to Example 9-(2) to obtain human soluble type CD14 deletion mutants.

The expression amount of CD14 deletion mutants was measured by EIA using an anti-human CD14 antibody. That is, anti-CD14 antibody MEM-18 (MONOSAN Co.) diluted 200 folds with 10 mM $NaHCO_3$ buffer solution at pH 8.3 was added to 96-well plate (Maxisorp, Nunc Co.) in an amount of 50 µL/Well and left to stand at 4°C for 24 hours. Thereafter, the plate was washed with deionized water and blocked with $PBS^-$ containing 0.5% BSA (by standing at room temperature for 60 minutes).

Then, the solution in the wells was discarded, 50 µL/Well of the culture supernatant of the transfected COS-1 was added to the wells and the plate was incubated at 25°C for 60 minutes. Thereafter, the plate was washed 3 times with PBS- containing 0.1% of Tween 20, 1.0 µg/mL of HRP-conjugated 3C10 antibody was added to the wells in an amount of 50 µL/Well and the plate was incubated at 25°C for 60 minutes. After washing the plate 5 times with $PBS^-$ containing 0.1% of Tween 20, a color developing substrate (TMB) was added to the wells in an amount of 50 µL/Well and reacted at room temperature for 30 minutes, followed by addition of a stop solution (1N sulfuric acid) in an amount of 50 µL/Well to terminate the reaction.

The absorbance of the reaction mixture at wavelength 450 nm was measured and the production amount of CD14 mutant polypeptide as a sample was calculated. Then, to confirm if the human soluble type CD14 mutants with C-terminal deletion have the objective length, the molecular weight of the CD14 mutant was determined by Western blotting with anti-human CD14 antibodies (3C10 and MEM-18). That is, 30 ng/lane each of CD14 mutants was electrophoressed on SDS-polyacrylamide gradient gel (5-20%, ATTO Co.), the protein was transferred onto PVDF membrane (Japan Millipore Co.) and blocking reaction was performed with 30 mL of PBS containing 0.5% skimmed milk at room temperature for 1 hour, and 10 µg/mL of 3C10 and 100-fold diluted MEM-18 were added, followed by reaction at room temperature for 1 hour. Thereafter, reaction was performed with HRP-conjugated anti-mouse Ig antibody at room temperature for 30 minutes, and detection was performed with an ECL kit (Amersham Pharmacia Biotech Co.). As a result, bands were detected with sizes estimated by calculation for respective CD14 mutant polypeptides.

(3) Preparation of human CD14 amino acid substitution mutants

In tables and this description, human sCD14(1-307) amino acid substitution modified polypeptide obtained by substituting amino acid at position 283 from the N-terminal of sCD14(1-307), Leu, by Ala is described as "sCD14 (1-307)L283A" and other human sCD14(1-307) amino acid substitution modified polypeptides are also described as the same.

According to the method described in Example 8 hereinbelow, plasmids that cause mammalian cells to express human sCD14(1-307) amino acid substitution mutants having introduced therein 1 or 2 amino acid mutations at various sites of human CD14(1-307) were transfected into COS-1 cells to prepare various human CD14 amino acid substitution mutants.

The culture supernatant containing the obtained CD14 mutants was purified as necessary. That is, the supernatant was charged to an affinity column for purification (HiTrap column, (Amersham Pharmacia Biotech Co.)) to which anti-human CD14 antibody (3C10) was bound to carry out selective absorption and the column was eluted with pH gradient. The obtained eluted fractions were immediately neutralized with 1 M HEPES buffer solution at pH 8.0 to make its pH neutral. Each fraction was assayed by an EIA method using HRP-conjugated 3C10 and fractions containing CD14 mutant polypeptides were selected.

(4) [Experiments on biding inhibition by peptides]

1 μg/mL of purified sCD14(1-356) was diluted with carbonate buffer (pH 9.5) and immobilized to a plate (Maxisorp, Nunc) at 37°C for 1 hour. Then, the plate was washed and blocked with 0.5% BSA/PBS. The blocking solution was removed and each peptide prepared in (1) above diluted with PBS to 10 μg/mL was added to the plate. Subsequently, F1024-1-3 antibody labeled with peroxidase by the method of Nakane et al. (J. Histochem. Cytochem., 22: 1084, 1974) was added in an amount of 1 μg/mL and reaction was performed at 37°C for 1 hour.

The plate was washed 5 times with 0.9% of NaCl/0.5% of Tween 20 and color was developed with an $H_2O_2$/TMB solution. Thereafter, the reaction was stopped with a 0.5 M sulfuric acid, and the amount of bound F1024-1-3 antibody was measured.

As a result, as shown in Fig. 6, peptides A and B inhibited F1024-1-3 antibody though weakly, while peptides C and D and irrelevant peptide E used as a control did not inhibit the antibody. This suggests that the region F1024-1-3 antibody recognizes exists between amino acid at position 283 and amino acid 318 at position.

(5) [Experiments on binding antibody to CD14 deletion mutants]

3C10 antibody or 100-fold diluted MEM-18 antibody was immobilized to a plate (Maxisorp, Nunc) in an amount of 10 μg/mL and blocked with 0.5% of BSA/PBS. Then, the blocking solution was removed, each CD14 deletion mutant whose concentration had been measured was added to the plate, and the reaction was performed at room temperature for 1 hour. After washing, the peroxidase-labeled F1024-1-3 antibody or peroxidase-labeled 3C10 antibody was diluted with 10% of RS/0.1% of Tween-20/PBS in a concentration of 1 μg/mL and added to plate to react in the same manner as above at room temperature for 1 hour and half. After washing, color was developed with an $H_2O_2$/TMB solution, then the reaction was stopped with 0.5 M sulfuric acid, and the absorbance of the reaction mixture at a wavelength of 450 nm was measured by using NJ-2100 (Japan Intermed) plate absorbance meter.

Since it has been made clear that 3C10 and MEM-18 antibodies used for immobilization have respective binding sites at amino acids at positions 7 to 11 and amino acids at positions 57 to 64, out of the CD14 deletion mutants used here, CD14(Δ7-11) does not bind to 3C10 while CD14(Δ57-64) does not bind to MEM-18 but binds to other CD14 deletion mutants. Therefore, by analyzing results of 3 types of sandwich ELISA systems, 3C10/F1024-1-3 system, MEM-18/F1024-1-3 system, MEM-18/3C10 system, the binding activity of each antibody can be analyzed.

Analysis of the obtained results indicates that as shown in Fig. 7, F1024-1-3 antibody exhibits binding activity within a range of sCD14(1-356) to sCD14(1-307) and also binds to CD14(Δ7-11) with deletion of the binding site for 3C10 and CD14(Δ57-64) with deletion of the binding site for MEM-18 and further exhibits binding activity to CD14 (Δ180-282) with deletion of amino acids at positions 180 to 282 of sCD14(1-307). From this, it follows F1024-1-3 antibody is different from 3C10, MEM-18 antibodies and has a binding region closer to the C-terminal side of CD14 than amino acid 285 is. Note that the binding activity to sCD14(1-307) was indicated by (+) since it was weaker than the binding activities to other CD14 deletion mutants.

(6) [Experiments on binding by CD14 amino acid substitution mutants]

The binding activity of CD14 amino acid substitution mutants was measured by using measurement systems similar to those described above. As a result, as shown in Fig. 8, the binding activity of F1024-1-3 antibody was lost by substituting amino acid 294, Pro, by Ala while such a phenomenon was not observed in the case of 3C10 and MEM-18 antibodies. F1024-1-3 antibody's loss of binding activity by point mutation at amino acid 294 of CD14, Proline is attributable to the change in three-dimensional structure of CD14. Therefore, it is evident that F1024-1-3 antibody recognizes a three-dimensional structure that can be generated when the amino acid 294 is Pro.

(7) [Experiments on binding by peptide mapping]

For the purpose of more specifically analyzing epitopes that F1024-1-3 antibody recognizes, 46 kinds of 10-amino acid residue peptides having an amino acid sequence shifted by 2 amino acids toward the C-terminal based on the amino acid sequence between amino acids at positions 246 to 345 of CD14 described in SEQ ID NO:1 by using custom SPOTs (GENOSYS) were synthesized on a membrane. Then, based on the protocol, the membrane was blocked and reacted with F1024-1-3 antibody as a primary antibody, and after washing, reacted with β-galactosidase-labeled anti-rat IgG $F(ab')_2$ antibody (American Qualex Antibodies) as a secondary antibody. After washing, a color developing solution was added and emergence of blue spots was observed. As a result, no spot attributable to F1024-1-3 was detected and determination of epitope of the antibody by peptide mapping was unsuccessful. This suggests that F1024-1-3 antibody does not recognize linear peptide epitopes composed of 10 amino acids but recognizes epitopes caused by three-dimensional structure.

From these, it has been made clear that the binding region of F1024-1-3 antibody recognize binding region existing between amino acids at positions 285 to 315 of CD14 described in SEQ ID NO:1 and recognizes the three-dimensional structure that can be generated by the amino acid 294 when it is Pro. That is, it has been made clear that the antibody recognizes the epitope existing between amino acids at positions 285 to 315 of the three-dimensional structure that can be generated by the amino acid 294 of CD14 when it is Pro.

(Example 7)

Analysis of the range of epitope of anti-CD14 antibody

The range of recognition site on CD14 to be recognized by an anti-CD14 body, which is necessary for the anti-CD14 antibody to have the same function as F1024-1-3 antibody, was analyzed centered on the periphery of the epitope of F1024-1-3 antibody as analyzed in Example 6.

Profile search for human CD14 was performed on BLOCKS database, which is a database prepared by arranging data in a multiple alignment so that motives were aligned, calculating similarity scores again including these of residues therearound, and extracting only those regions having no insertion or deletion and having a high degree of conservation. Further, analysis was made by various types of secondary structure prediction methods (Lev, GOR IV, PREDATOR).

As a result, based on the profile search of BLOCKS database, a region having similarity to the binding region of IL-1 (Accession No. BL005253C) to its receptor was identified as 39 amino acid residues 269 to 307. The amino acid residues (residue number) presumed to have high degree of conservation include Q(271), V(272), P(273), L(276), K(279), L(283), L(285), S(286), C(287), P(294), E(298), L(299), P(300), E(301), N(304), L(305) and T(306).

Furthermore, the C-terminal side sequence from the position 287 of this region did not have so high homology to LRR (PDB-ID:1A4Y:A).

Various types of secondary structure prediction methods (Lev, GORIV, PREDATOR) indicated that no coincident prediction results as to both α-helix structure and β-sheet structure can be obtained, that generally it is difficult to assume a helix structure or a sheet structure when proline exists in large amounts, and that the amino acid 304, asparagine, coincides with the sugar chain binding motif in the motif database and is a sugar chain bindable site. From this it has revealed that this region is exposed on the surface of the protein.

From these analyses, the region is capable of forming a loop under physiological conditions and is a site capable of interaction with other proteins.

That is, from the epitope of F1024-1-3 antibody in Example 6 and the above-mentioned analyses, the range of epitope of anti-CD14 antibody that can inhibit the binding between human CD14 and TLR is the region at positions 269 to 315 of human CD14.

(Example 8)

Preparation of CD14 mutant polypeptides with deletion of amino acid on the C-terminal side of human CD14 (human soluble type CD14 C-terminal deletion modified polypeptides)

(1) [Construction of full-length sCD14 expression plasmid (pM1656)

First, plasmid pM1650 that expresses mCD14 in mammalian cells was constructed. An about 1.4 kb DNA fragment containing human CD14 cDNA was cleaved from plasmid pUCH14P-4 described in WO98/39438 with Xba I and HindIII and inserted into the Xba I/HindIII site of pcDNA 3.1(-) (Invitrogen Co.), which is a mammalian cell expression vector. Thereafter, E. coli Competent Cells (JM109 cells, TaKaRa Co.) were transformed therewith according to the attached protocol and the resulting colonies were confirmed by PCR to obtain target mCD14-expressing plasmid (pM1650).

Next, in order to have human CD14 expressed as a soluble type protein, a recombinant-expressing plasmid pM1656 with substitution of Asn at position 326 and Gly at position 328 from the N-terminal (cf. SEQ ID NO:1), which are sites necessary for GPI anchoring, by Gln and Val, respectively. That is, by using sense primer 9 (SEQ ID NO:28) and antisense primer 9 (SEQ ID NO:29) and also using the above-mentioned pM1650 as a template, PCR reaction was performed by repeating 30 times the cycle consisting of 94°C for 30 seconds, 55°C for 30 seconds, and 72°C for 1 minute with TaKaRa Ex Taq (TaKaRa Co.). The amplified DNA fragment was subjected to double digestion with XhoI and ApaL I, and inserted into the XhoI/ApaL I site of pM1650. After the transformation of JM109 cells, the resulting colonies were confirmed by PCR to obtain the target sCD14-expressing plasmid (pM1656).

(2) [Construction of human soluble type CD14 C-terminal deletion modified polypeptide-expressing plasmids (pM1658 to pM1662 and pM1674 to pM1676)]

Plasmids pM1658, pM1674, pM1675, pM1676, pM1659, PM1660, PM1662 and PM1661 that express recombinants with deletion of amino acid residue at positions 49, 56, 61, 66, 71, 110, 173, and 204, respectively, from the C-terminal of CD14 (hereinafter, referred to as sCD14(1-307), sCD14(1-300), sCD14(1-295), sCD14(1-290), sCD14 (1-285), sCD14(1-246), sCD14(1-183), and s-CD14(1-152), respectively) in mammalian cells were constructed by the following method.

First, by using sense primer 9 and antisense primers 10, 11, 12, 13, 14, 15, 16 and 17 (SEQ ID NOs:30, 31, 32, 33, 34, 35, 36 and 37, respectively), and also using plasmid pM1656 prepared in (1) above as a template, PCR reaction was performed with Pyrobest DNA Polymerase (TaKaRa Co.) by repeating 30 times the cycle consisting of 98°C for 10 seconds, 55°C for 30 seconds, and 72°C for 1 minute.

Then, the amplified DNA fragment was subjected to double digestion with XhoI and HindIII, and the products were separated and purified by 1% agarose gel electrophoresis. Also, pM1656 was digested with XhoI and HindIII and purified in the same manner as above, and the obtained an about 5.8 kb DNA fragment and the above-mentioned PCR fragment were ligated. After the transformation of JM109 cells, the resulting colonies were confirmed by PCR to obtain the target CD14 mutant polypeptide-expressing plasmids (pM1658, pM1674, pM1675, pM1676, pM1659, pM1660, pM1662 and pM1661).

(3) [Expression in COS-1 cells]

Plasmids pM1656, pM1658 to pM1662 and pM1674 to pM1676 prepared in (1) and (2) were transfected into COS-1 cells by the following method to have sCD14(1-356), sCD14(1-307), sCD14(1-300), sCD14(1-295), sCD14 (1-290), sCD14(1-285), SCD14(1-246), SCD14(1-183) and SCD14(1-152) expressed therein. That is, 50 μL of FuGENE6 (Roche Diagnostics Co.) was mixed with 12.5 μg of each of the above-mentioned plasmid DNAs according to the attached protocol, and the mixtures were each added to COS-1 cells semi-confluently grown in 150 $cm^2$ flask. After culturing the cells under the conditions of 5% $CO_2$ and 37°C for 72 hours, the supernatants were recovered to obtain the target CD14 mutant polypeptides.

The expression amount of CD14 mutant polypeptides was measured by EIA with anti-human CD14 antibody. That is, anti-CD14 antibody MEM-18 (MONSANT Co.) diluted 200 folds with 10 mM $NaHCO_3$ buffer solution at pH 8.3 was added to a well plate (Maxisorp, Nunc Co.) in an amount of 50 μL/well and allowed to stand at 4°C for 24 hours. Thereafter, the plate was washed with deionized water and blocked with $PBS^-$ containing 0.5% BSA (by standing at room temperature for 60 minutes).

Then, the solutions in the wells were removed, 50 μL/well of culture supernatant of the transfected COS-1 was added to the wells and the plate was incubated at 25°C for 60 minutes. After washing the plate 3 times with $PBS^-$ containing 0.1% Tween 20, 1.0 μg/mL of HRP-conjugated 3C10 antibody was added to the wells in an amount of 50 μL/well and the plate was incubated at 25°C for 60 minutes. After washing the plate 5 times with $PBS^-$ containing 0.1% Tween 20, a color developing substrate (TMB) was added to the wells in an amount of 100 μL/well and reacted at room temperature for 30 minutes, followed by addition of a stop solution (1N hydrochloric acid) in an amount of 100 μL/well to terminate the reaction.

The absorbance at a wavelength of 450 nm was measured and the production amount of CD14 mutant polypeptide in a sample was calculated.

(4) [Purification of human soluble type CD14 C-terminal deletion modified polypeptides]

The culture supernatant containing the CD14 mutant polypeptides obtained in (3) above were subjected to an affinity column for purification (HiTrap column, Amersham Pharmacia Biotech Co.) to which anti-human CD14 antibody (3C10) was bound to carry out selective absorption and the column was eluted with pH gradient. The obtained eluted fractions were immediately neutralized with 1 M HEPES buffer solution at pH 8.0 to make its pH neutral. Each fraction was assayed by an EIA method by using HRP-conjugated 3C10 and fractions containing CD14 mutant polypeptides were selected.

(5) [Detection of human soluble type CD14 C-terminal deletion modified polypeptides]

The molecular weight of the CD14 mutant polypeptides was determined by Western blotting with anti-human CD14 antibodies (3C10 and MEM-18). That is, 30 ng/lane each of CD14 mutants was electrophoresed on SDS-polyacrylamide gradient gel (5 to 20%, ATTO Co.). After the protein was transferred onto a PVDF membrane (Japan Millipore Co.), blocking reaction was performed with 30 mL of $PBS^-$ containing 0.5% skimmed milk at room temperature for 1 hour. Then, 10 µg/mL of 3C10 and 100-fold diluted MEM-18 were added and reaction was performed at room temperature for 1 hour. This was further reacted with HRP-conjugated anti-mouse Ig antibody at room temperature for 30 minutes, and thereafter detection was performed with an ECL kit (Amersham Pharmacia Biotech Co.). As a result, bands were detected with sizes estimated by calculation for respective modified polypeptides (Fig. 9).

(Example 9)

Preparation of human CD14 amino acid substitution modified polypeptides

(1) [Construction of CD14 mutant polypeptide with

substitution of amino acids of sCD14(1-307) (human sCD14(1-307) amino acid substitution modified polypeptides)-expression plasmids]

In tables and in this description, human sCD14(1-307) amino acid substitution modified polypeptide obtained by substituting amino acid 283 from the N-terminal of sCD14(1-307), Leu, by Ala is described as "sCD14(1-307)L283A", and other human sCD14(1-307) amino acid substitution modified polypeptides are also described in this manner.

In order to prepare human sCD14(1-307) amino acid substitution modified polypeptides having introduced therein 1 or 2 amino acid mutations at various sites of human CD14(1-307), plasmids that express the polypeptides in mammalian cells were prepared by the following method.

Plasmid pM1673 that expresses sCD14(1-307)K279A was prepared as follows. That is, by using sense primer 3 and antisense primer 18 (SEQ ID NO:38) or by using sense primer 10 (SEQ ID NO:39) and antisense primer 4, and also using the plasmid pM1685 prepared in Example 8-(2) as a template, PCR reaction was performed by repeating 30 times the cycle consisting of 98°C for 10 seconds, 55°C for 30 seconds, and 72°C for 1 minute with Pyrobest DNA Polymerase (TaKaRa Co.).

As for the antisense primer 18 and sense primer 10, the sequence GCT (AGC in the case of antisense primer) encoding Ala was used in place of the sequence encoding Lys. DNA fragments amplified by the PCR were separated and recovered by 1% agarose gel electrophoresis and the terminals of the DNA fragments were blunt-ended with Klenow Fragment (TaKaRa Co.).

Then, by using a mixture of these fragments as a template and also using sense primer 3 and antisense primer 4, PCR reaction was performed again under the same conditions as described above. The DNA fragments amplified by the second PCR were subjected to double digestion with XhoI and HindIII and the product was ligated to an about 5.8 kb DNA fragment obtained by digestion of pM1656 with XhoI and HindIII. After the transformation of JM109 cells, the resulting colonies were confirmed by PCR to obtain the target CD14 mutant polypeptide-expressing plasmid (pM1673).

Similarly, plasmids (pM1663, pM1677, pM1664, pM1678, pM1665, pM1666, pM1667, pM1669, pM1670, pM1671 or pM1672) expressing sCD14(1-307)V282A, sCD14(1-307)L283A, sCD14(1-307)D284A, sCD14(1-307)L285A, sCD14(1-307)S286A, sCD14(1-307)C287A, sCD14(1-307)R289A, sCD14(1-307)P294A, sCD14(1-307)P296A, sCD14(1-307)P294/296A or sCD14(1-307)P300A obtained by substituting Val at position 282, Leu at position 283, Asp at position 284, Leu at position 285, Ser at position 286, Cys at position 287, Arg at position 289, Pro at position 294, Pro at position 296, Pro's at positions 294 and 296, or Pro at position 300 from the N-terminal each by Ala were prepared in the same manner as pM1673 by using antisense primers 19, 20, 21, 22, 23, 24, 25, 26, 27, 28 and 29 (SEQ ID NOs:40, 41, 42, 43, 44, 45, 46, 47, 48, 49 and 50) and sense primers 11, 12, 13, 14, 15, 16, 17, 18, 19, 20 and 21 (SEQ ID NOs:51, 52, 53, 54, 55, 56, 57, 58, 59, 60 and 61) with changing the codon sequence of the amino acid to which substitution was to be introduced to the codon sequence GCT or GCG encoding Ala (the sequence AGC or CGC in the case of antisense primers).

In addition, plasmid that expresses sCD14(1-307)R289D (pM1668) was similarly constructed. In this case, in order to substitute Arg by Asp, sense primer 22 (SEQ ID NO:62) and antisense primer 30 (SEQ ID NO:63) with the codon sequence (AGA) encoding Arg being changed to the codon sequence GAT (ATC in the case of antisense primer) encoding Asp were used.

Furthermore, plasmids that express mutants with Ser at position 286 thereof being substituted by Cys, Gly, Thr or Leu, respectively, were prepared. The same construction method as that for constructing sCD14(1-307)S286A expressing plasmid was used except that sense primer 23 (SEQ ID NO:64) and antisense primer 31 (SEQ ID NO:65) were used in the case of Cys substitution. Construction of other plasmids was performed by using sense primer 24 (SEQ ID NO:66) and antisense primer 32 (SEQ ID NO:67) in the case of Gly substitution, sense primer 25 (SEQ ID NO:68) and antisense primer 33 (SEQ ID NO:69) in the case of Thr substitution, and sense primer 26 (SEQ ID NO: 70) and antisense primer 34 (SEQ ID NO:71) in the case of Leu substitution.

(2) [Production and purification of human sCD14(1-307) amino acid substitution modified polypeptides]

Production of human sCD14(1-307) amino acid substitution modified polypeptides in COS-1 cells and measurement of expression amount in the culture supernatant were performed in the same manner as in Example 8-(3).

Expression amounts of respective CD14 mutant polypeptides are shown in Table 1 and Table 2.

Table 1

| Expression amount of human sCD14(1-307) amino acid substitution modified polypeptides in COS cells | |
|---|---|
| Name | Expression amount in supernatant'of COS-1 cell culture (μg/mL) |
| sCD14(1-356) | 3.38 |
| sCD14(1-307) | 2.99 |
| sCD14(1-307)L283A | 0.44 |
| sCD14(1-307)D284A | 0.46 |
| sCD14(1-307)L285A | 0.11 |
| sCD14(1-307)S286A | 0.80 |
| sCD14(1-307)C287A | 2.44 |
| sCD14(1-307)N288A | 0.11 |
| sCD14(1-307)R289A | 0.51 |
| sCD14(1-307)R289D | 0.96 |
| sCD14(1-307)P294A | 0.69 |
| sCD14(1-307)P296A | 0.60 |
| sCD14 (1-307) P294/296A | 0.67 |
| sCD14(1-307)P300A | Not detected |

Table 2

| Expression amount of human sCD14(1-307) amino acid substitution modified polypeptides in COS cells | |
|---|---|
| Name | Expression in COS cells (μg/mL) |
| sCD14(1-307)S286A | 0.80 |
| sCD14(1-307)S286C | 4.36 |
| sCD14(1-307)S286G | 0.178 |
| sCD14(1-307)S286T | 6.05 |
| sCD14(1-307)S286L | 0.076 |

In the case of sCD14 (1-307)P300A, expression in the culture supernatant was not confirmed. In addition, sCD14(1-307) modified polypeptides other than sCD14(1-307)S286L with a low expression amount were purified from the culture supernatant of COS-1 transfected with each plasmid in the same manner as in Example 8-(4).

3) Detection of human sCD14(1-307) amino acid substitution modified polypeptides

Detection of each CD14 mutant polypeptide was performed by Western Blotting in the same manner as the detection of soluble type CD14 C-terminal deletion modified polypeptides. All the human sCD14(1-307) amino acid substitution modified polypeptides showed the same molecular weight as that of sCD14(1-307).

(Example 10)

IL-6 production inhibitory activity of CD14 mutant polypeptides

This was performed in the same manner as described in Example 3 except that 40 µL of 900 ng/mL CD14 mutant polypeptide was added in place of 40 µL of 900 ng/mL F1024-1-3 antibody.
The results obtained are shown in Fig. 10, Fig. 11 and Fig. 12.
sCD14(1-285), sCD14(1-246) and sCD14(1-183) exhibited IL-6 production inhibitory activities of 67.5%, 45.4%, and 42.6%, respectively. The CD14 mutant polypeptide that exhibited the highest inhibitory activity was sCD14 (1-285).
Also, sCD14(1-307)D284A and sCD14(1-307)S286A exhibited high inhibitory activities as high as 81.3% and 79.6%, respectively. In the case of sCD14(1-307)C287A, low activity as low as 24.4% was observed.
Furthermore, as for the inhibitory activity of the mutants with substitution of Ser at position 286 thereof, sCD14 (1-307)S286G was the highest and sCD14(1-307)S286C exhibited substantially equivalent inhibitory activity as that of sCD14(1-307)S286A.

(Example 11)

IL-6 production inductive activity of CD14 mutant polypeptides

Whether or not CD14 mutant polypeptides exhibit IL-6 production inductive activity to HUVEC cells was observed by the following method. That is, in the same manner as in Example 10, HUVEC cells were inoculated in wells of a 96-well plate and cultured for 24 hours. Thereafter, 20 µL of RPMI1640 medium containing 7% of the human serum from which soluble type CD14 had been removed, 10 µL of 120 ng/mL LPS (E. coli 055:B5, Difco Co.), and 40 µL of 900 ng/mL CD14 mutant polypeptide were added to the wells. The resulting mixture was further cultivated for 20 hours and then IL-6 amount in the culture supernatant was measured by using human IL-6 EIA kit (PE Biosystems, Inc.).
The results obtained are shown in Table 3. Induction of IL-6 production was observed in sCD14(1-356), sCD14 (1-307) and sCD14(1-152) that showed no IL-6 inhibitory activity IL-6 production and sCD14(1-183) and sCD14(1-307) C287A that showed inhibitory activity of the IL-6 production in Example 10.
In the case of sCD14(1-246), sCD14(1-285), sCD14(1-307)D284A and sCD14(1-307)S286A that showed inhibitory activity in Example 10, production of IL-6 was at or below detection limit of measurement (50 pg/mL). That is, it has been elucidated that sCD14(1-246), sCD14(1-285), sCD14(1-307)D284A, sCD14(1-307)S286A, and sCD14 (1-307)S286C do not induce IL-6 production in the presence of LPS but inhibits IL-6 production through a complex of serum-derived soluble type CD14 with LPS.

Table 3

| Amount of IL-6 production in a final concentration of 300 ng/mL (pg/mL) | |
|---|---|
| sCD14(1-356) | 216 |
| sCD14(1-307) | 203 |
| sCD14(1-285) | Not detected |
| sCD14(1-246) | Not detected |
| sCD14(1-183) | 86.7 |
| sCD14(1-152) | 149 |
| sCD14(1-307)D284A | Not detected |
| sCD14(1-307)S286A | Not detected |
| sCD14(1-307)S286C | Not detected |
| sCD14(1-307)C287A | 76.0 |
| Serum-derived soluble type CD14 low molecular weight form | Not detected |

(Example 12)

TNFα production inhibitory activity of CD14 mutant polypeptides in peripheral blood macrophage and monocyte

Peripheral blood macrophages and monocytes were isolated from peripheral blood of a healthy person by the following method. First, 200 mL of heparinized human peripheral blood was diluted with $PBS^-$ two folds and 28 mL aliquot of the dilution was overlaid on to 21 mL of Ficoll Paque (Amersham Pharmacia'Biotech Co.). After centrifugation at 1,500 rpm for 30 minutes, the monocyte fractions were recovered. Then, macrophages and monocytes were isolated by using a MACS system (Daiich Pure Chemicals Co., Ltd.). That is, monocytes recovered by density gradient centrifugation were reacted with magnetic beads coated with anti-CD14 antibody (Daiichi Pure Chemicals Co., Ltd.) and then CD14-positive cells were positively selected by using separation column RS+ (Daiichi Pure Chemicals Co., Ltd.).

The obtained peripheral blood macrophages and monocytes were floated in 2% CD14w/oHS/RPMI and inoculated in wells of a 96-well plate in a density of $0.5 \times 10^5$. cells/well (50 μL/well) and cultured under the conditions of 37°C and 5% of $CO_2$ for 24 hours.

20 μL of RPMI1640 medium containing 7% of the human serum from which soluble type CD14 had been removed, 10 μL of 120 ng/mL LPS (E. coli 055:B5, Difco Co.), and 40 μL of CD14 mutant polypeptide of varied concentration were added to the wells and culture was continued for additional 4 hours. Then, TNFα amount in the culture supernatant was measured by using an hTNF-α ELISA SYSTEM (Amersham Pharmacia Biotech Co.). The measurement method was in accordance with the protocol attached to the kit. That is, 50 μL of the appropriately diluted culture supernatant was transferred to the reaction plate and 50 μL of a solution of biotinated antibody was added thereto, followed by standing the plate at room temperature for 2 hours. Then the reaction mixture was removed and each well was washed 4 times with 400 μL/well of washing solution. Then, 100 μL of an appropriate diluted solution of peroxidase-labeled streptoavidin was added to each well and the plate was allowed to stand at room temperature for additional 30 minutes. After washing, 100 μL of a color developing substrate was added and reacted at room temperature for 15 minutes. The reaction was stopped by addition of 100 μL of a stop solution and absorbance at a wavelength of 450 nm was measured, followed by calculation of the amount of TNFα.

As a result, TNFα production inhibitory activity was observed for human soluble type CD14 C-terminal deletion modified polypeptides sCD14(1-285), sCD14(1-246) and sCD14(1-183) and sCD14(1-307) amino acid substitution modified polypeptides sCD14(1-307)D284A and sCD14(1-307)S286A (Fig. 13).

(Example 13)

Experiments on inhibition of NF-κB activation by CD14 mutant polypeptides in human TLR4 expression transformant cell line

In the same manner as in Example 2(2), experiments on inhibition of NF-κB activation in human TLR4 expression transformant cell line by using sCD14(1-307)S286C. As a result, sCD14(1-307)S286C exhibited addition concentration-dependent suppressive activity (Fig. 14).

(Example 14)

Analysis of inhibition mechanism by using a protein interaction analyzer

(1) [Preparation of monoclonal antibody which is specific to CD14 protein having a molecular weight of 49 kDa]

[1] Preparation of a peptide specific to a CD14 protein having a high molecular weight of 49 kDa

The sequence consisting of amino acids at positions 316 to 328 described in SEQ ID NO:1 was selected as a CD14-specific peptide having a high molecular weight of 49 kDa for use in immunization (hereinafter referred to as peptide 13).

Note that cystein was inserted at the C-terminal in order to bind the selected peptide to a carrier protein through an SH group at the C-terminal. The synthesis of peptide was performed by using ABI432A peptide synthesizer (Applied). The peptide was cut out from the resin by a conventional method and the peptide was purified by using C18 reverse phase HPLC (CAPCELL-Pak, Shiseido).

[2] Preparation of a peptide carrier antigen by using the synthetic peptide

The peptide prepared in [1] was dissolved in distilled water to 10 mg/mL, and the solution was mixed with maleimidated keyhole limpet hemocyanin (KLH, PIERCE) in equal proportions. After 2 hours of reaction at room temperature, the reaction mixture was desalted through NAP-10 column (Pharmacia) to obtain peptide 13 carrier antigen (hereinafter, referred to as peptide 13-KLH). As for the concentration of the protein, the value obtained by dividing the volume of KLH used by the volume of the solution was used.

[3] Preparation of monoclonal antibody which is specific to CD14 protein having a molecular weight of 49 kDa

Cell fusion was performed in the same manner as in Example 1(1) by using 100 μg of peptide 13-KLH as an immunogen. Hybridomas were selected in HAT medium (GIBCO) and after 1 week, screening of hybridomas producing antibodies that react with a recombinant human CD14 protein was performed.

First, the purified recombinant human CD14 protein was diluted with 0.01 M carbonate buffer solution (pH 9.5) to 1 μg/mL, and 50 μL aliquot was added to each well of Immuno-plate (Maxisorb, NUNC). After 1 hour of reaction at 37°C, the wells were washed 5 times with deionized water and 100 μL of PBS containing 0.5% BSA was added to each well to effect blocking. Then, the supernatants sampled from the cultures of the selected hybridomas were added in wells, respectively, and reacted at 37°C for 1 hour. Then, the wells were washed 3 times with physiological saline containing 0.05% Tween 20. Peroxidase-labeled anti-rat immunoglobulin antibody (DAKO) diluted 1,000 folds with PBS containing 10% rabbit serum was added to each well in an amount of 50 μL. After 1 hour's reaction at 37°C, the wells were similarly washed 5 times and tetramethylbenzidine solution containing 0.01% hydrogen peroxide was added to each well. After 10 minutes of reaction at room temperature, the reaction was stopped by a 0.5 M sulfuric acid solution and the absorbance was measured at 450 nm by using a plate spectrophotometer (NJ-2100, Japan Intermed). As a result, the well containing the hybridoma that reacted with CD14 protein having a high molecular weight (F1025-4-1) was selected and cloning was performed by a limiting dilution method.

The selected hybridoma was cultured in 10% FCS/RPMI-1640 medium (GIBCO) and then cultured in Hybridoma-SFM medium (GIBCO) to produce an antibody, which was purified by using Prosep-G column (Bioprocessing). The subtype of the purified F1025-4-1 antibody revealed to be rat IgG1/κ.

[4] Preparation of HRP-labeled antibody

To 0.5 mg of a peroxidase (Toyobo) solution in distilled water was added a solution of 100 mM periodic acid in distilled water and the mixture was allowed to react at 25°C for 20 minutes. After completion of the reaction, 1.5% of ethylene glycol was added and after 10 minutes of reaction at 25°C, the reaction mixture was dialyzed against a 1 mM acetate buffer solution (pH 4.4). The purified F1025-4-1 antibody was dialyzed against a 10 mM carbonate buffer solution (pH 9.5) and 0.5 mg of peroxidase activated by addition of 0.5 mg of 1 M carbonate buffer solution (pH 9.5) was mixed with each antibody in equal proportions and the mixture was allowed to react at 25°C for 2 hours. 4 mg/mL sodium borohydride was added to the reaction mixture and 2 hours of reaction was performed at 4°C. The reaction mixture was dialyzed against PBS to obtain a peroxidase-labeled antibody. The amount of liquid was measured and the concentration of antibody was calculated from the amount of antibody used.

(2) [Analysis of mechanism of inhibition by F1024-1-3 antibody]

[1] Expression of recombinant TLR4

COS-1 cells (ATCC: CRL1150) were inoculated in a density of $3 \times 10^5$ cells/75-cm$^2$ of flask and cultured for 24 hours under the conditions of 37°C and 5% $CO_2$. On the day next, the plasmid pCDNAT4 described in Example 2 (1) was mixed with the cells in proportions of 6.25 μg DNA: 25 μL FuGENE6 according to the protocol described in FuGENE6 (Roche) and the mixture was added to a flask containing 15 mL of 1% FBS/DMEM (Sigma, high glucose) and cultured under the conditions of 37°C and 5% $CO_2$ for 48 hours. COS-1 cells manipulated in the same manner as above except that no plasmid was contained were used as negative control. Supernatants of cultures of COS-1 cells expressing TLR4 molecules on the cell membrane thereof (hereinafter, referred to as TLR4-COS) and COS-1 cells expressing no TLR4 molecule on the cell membrane thereof were discarded and the cells were washed twice with $PBS^-$ (Sigma). Then, 5 mL of a 1% EDTA/$PBS^-$ solution was added thereto and lightly stirred. Thereafter, the cells were scraped off from the culture flask by using cell scraper (COSTAR) and recovered in a 50 mL centrifuge tube. The flask was further washed with 5 mL of $PBS^-$ and added in a 50-mL centrifuge tube, which was centrifuged at 1,000 rpm for 10 minutes to settle the cells, and then the supernatant was discarded. Further, the cells were washed twice with $PBS^-$. Then, the washed cells were filtered through a 40-μm mesh cell strainer (FALCON) and the number of cells was counted. The cells were centrifuged once again and diluted with $PBS^-$ to $5 \times 10^5$ cells/mL and stored at 4°C.

[2] preparation of anti-FITC antibody-immobilized chip

In order to immobilize anti-FITC monoclonal antibody (OEM concept Co.) to cells on a chip for use in the analysis of BIACORE3000 (BIACORE Co.), the cells were activated with a NHS/EDC solution (BIACORE Co.) for 7 minutes according to the manual provided by BIACORE Co. and then an antibody diluted with a pH 6.0 acetate buffer solution to 50 μg/mL was added to the cells by a manual injection method to immobilize the antibody, followed by blocking with ethanolamine. As the reference, non-treated cells were used.

[3] Preparation of FITC-LPS/CD14 complex

FITC-LPS (Sigma, Serotype0111:B4) diluted with PBS-( pH 7.4) to 6 μg/mL and 300 μg/mL of recombinant sCD14(1-356) prepared in Example 8 were mixed in the proportion of 1:1 and the mixture was allowed to stand at 37°C for 30 minutes to form a complex. The formation of a complex was confirmed by an ELISA system using an anti-FITC antibody-immobilized plate and the peroxidase-labeled anti-CD14 antibody (F1025-4-1) prepared in (1) above. That is, the anti-FITC antibody was immobilized to the plate in a concentration of 10 μg/mL and blocked with 0.5% bovine serum albumin/PBS-. Then, the prepared FITC-LPS/CD14 complex, FITC-LPS, and sCD14 were added to antibody-immobilized wells and allowed to react at 37°C for 1 hour. After washing each well, the peroxidase-labeled anti-CD14 antibody diluted to 1 μg/mL was added to each well, followed by reaction. After washing, the product was reacted with a TMB color developing substrate solution (BioFix, Funakoshi), and at a stage where an appropriate color developing was obtained, the reaction was stopped with sulfuric acid and the absorbance was measured at 450 nm. As a result, FITC-LPS and sCD14 alone resulted no increase in absorbance while only when FITC-LPS/CD14 complex was present, the absorbance increased, which confirmed formation of a complex.

[4] Analysis of inhibition mechanism

The prepared FITC-LPS/CD14 complex was immobilized on the chip and then F1024-1-3 antibody diluted with HBS-EP buffer solution to 130 μg/mL was injected to bind the antibody to the immobilized CD14. Then, TLR4-COS cells and COS cells as control were injected and binding amounts of TLR4-COS cells and COS cells were measured. The results obtained are shown in Fig. 15, in which the heights of the bar charts indicate binding amounts of complexes onto the cells on the tip. That is, an increase in response was observed by immobilizing of LPS/CD14 complex to the cells on the tip. Next, since no response was observed in COS cells, no binding was confirmed. On the other hand, in TOLL4-COS cells, an increase in response (200RU) was observed and hence binding was confirmed. When LPS/CD14 was reacted with an antibody, an increase in response was similarly observed and binding was confirmed. Further, when COS cells and TOLL4-COS cells were allowed to react, increases in response were observed in both cases (136 RU in TOLL4-COS cells and 115 RU in'COS cells). Since the increase in response in COS cells was attributable to nonspecific binding between the cell and antibody, the inhibition rate was 90%. This indicates that binding of F1024-1-3 antibody to CD14 inhibited the binding between TLR4 and CD14, so that it has become evident that the suppression mechanism of F1024-1-3 antibody involves inhibition of binding of TLR4 to CD14. Inhibition rate was calculated by [(Response of TLR4-COS(TOLL4-COS) cells without F1024-1-3 antibody) - {(Response of TOLL4-COS cells when F1024-1-3 antibody is bound) - (Response of COS cells when F1024-1-3 antibody is bound)}]/(Response of TOLL4-COS cells without F1024-1-3 antibody) × 100 (%).

(2) [Analysis of mechanism of inhibition of CD14 mutant polypeptide]

[1] Preparation of CD14 immobilized chip

In order to immobilize sCD14(1-356) to cells on a chip for use in the analysis of BIACORE3000, the cells were activated with a NHS/EDC solution (BIACORE) for 14 minutes according to the manual provided by BIACORE Co. and then a sCD14(1-356) diluted with a pH 4.0 acetate buffer solution to 200 μg/mL was added to the cells by a manual injection method to immobilize the antibody, followed by blocking with ethanolamine. As the reference, non-treated cells were used.

[2] Formation of CD14/LPS/TLR4 complex

The prepared chip was set in BIACORE3000 and sensorgram was started at a flow rate of 10 μL/min by using an HBS-EP buffer solution (BIACORE) according to the manual provided by BIACORE Co. First, 5 μL each of 10 μg/mL sCD14(1-307)(S286C), 1.7 × $10^5$ cells/mL COS cells, 1.7 × $10^5$ cells/mL Toll4-COS cells were injected but no binding to sCD14( 1-356) was observed in the three cases. Then, 30 μL of LPS (Sigma, Serotype 055:B5) was injected to sCD14( 1-356) cells at a flow rate of 1 μL/mL to bind it to the cells, and subsequently, injection of 5 μL of Toll4-COS cells adjusted to 1.7 × $10^5$ cells/mL with PBS- containing 1% human serum (from which soluble type CD14 had been removed by the method described in Example 3) in order to form LPS/CD14/TLR4 complex led to confirmation of the binding (Fig. 16).

[3] Analysis of inhibition mechanism

After dissociation of the complex with 2M thiocyanate solution, LPS was bound again under the same conditions. Then, 1.7 × $10^5$ cells/mL To114-COS cell and 33 μg/mL sCD14(1-307) ( S286C) were mixed and allowed to react at room temperature for 20 minutes. After completion of the injection of LPS, 5 μL of the mixed solution was injected at a flow rate of 10 μL/min. As a result, binding of To114-COS cell to sCD14( 1-356)/LPS complex was suppressed by substantially 100% in the presence of sCD14(1-307) ( S286C) (Fig. 16).

(Example 15)

Experiment on binding of LPS

sCD14(1-356), sCD14(1-307) or sCD14(1-307)S286A (each in a final concentration of 20 μg/mL) and LPS (final concentration of 100 μg/mL) were mixed and incubated at 37°C for 16 hours. After 5 hours of electrophoresis of the reaction mixtures on 7.5% polyacrylamide gel (ATTO Co.) without addition of SDS at a constant voltage of 300 V for 5 hours, the proteins were transferred to a PVDF membrane.

Thereafter, Western Blotting was performed in the same manner as in Example 8 to detect bands of sCD14. In this case, however, the anti-CD14 antibody used was rat serum obtained by administering sCD14 purified from serum of healthy person as an antigen to a rat, that is, rat anti-human CD14 antiserum and the secondary antibody used was HRP-conjugated anti-rat Ig antibody (DAKO Co.).

As a result, shift of molecule weights was observed in all sCD14(1-356), sCD14(1-307) and sCD14(1-307) S286A. Thus, it was confirmed that these bind to LPS (Fig. 17).

(Example 16)

Preparation of human blood-derived soluble type CD14 low molecular weight form polypeptide

(1) [Purification of human blood-derived soluble type CD14 low molecular weight form polypeptide]

First, human serum-derived soluble type CD14 purified by the method shown in Example 8-(4) was applied to a column for affinity purification binding of an F1024-1-3 antibody (HiTrap column, Amersham Pharmacia Biotech Co.) to remove soluble type CD14 of high molecular weight species. The obtained non-absorbed fraction was concentrated by a freeze-drying method and the concentration thereof was determined by an EIA method using anti-CD14 antibody described in Example 8. As a result, the ratio of serum-derived soluble type CD14 low molecular weight form in the whole soluble type CD14 in the serum was below 5%.

(2) [Detection of human serum-derived soluble type CD14 low molecular weight form polypeptide]

The molecular weight of each fraction of human serum-derived soluble type CD14 obtained in the method shown in [1] was confirmed by Westernblotting using anti-human CD14 antibodies (3C10 and MEM-18). That is, the human serum-derived soluble type CD14 fractions were analyzed by the method shown in Example 8(5). The results of analysis are shown in Fig. 18.

As a result, bands of human serum-derived soluble type CD14 were detected at positions of 49kDa and 55kDa (Lane1) and a band of human serum-derived soluble type CD14 low molecular weight form polypeptide was detected at a position of 36 kDa (Lane2).

Further, the CD14 low molecular weight form polypeptide was confirmed to be located between sCD14(1-285) and sCD14(1-246) out of the CD14 mutant polypeptides prepared in Example 8 (Fig. 9). Further, their binding to 3C10 antibody indicates that the amino acids at position 7 et seq. of CD14 are maintained therein. From these it can be seen that CD14 low molecular weight form corresponds to CD14 with its N-terminal being between amino acids at positions 1 to 6 and C-terminal between amino acids at positions 246 to 285 thereof.

Note that the reason that no molecule of 36 kDa was found in human serum-derived soluble type CD14 (Lane1) was due to the fact that the molecule of 36 kDa contained in human serum-derived soluble type CD14 is in minute amounts in comparison with the molecules of 49 kDa and 55 kDa.

Further, 100 ng/lane of the obtained serum-derived soluble type CD14 low molecular weight form polypeptide was electrophoresed on SDS-polyacrylamide gradient gel (5-20%, ATTO Co.) and staining of protein with silver was performed by using 2D-silver staining reagent II "Daiichi" kit (Daiich Pure Chemicals Co., Ltd.). As a result, human serum-derived soluble type CD14 low molecular weight form polypeptide was detected as a slightly broad single band at a position of 36±5 kDa, which confirmed that it was substantially purified.

(Example 17)

Cytotoxic inhibitory activity of CD14 low molecular weight form

Human vascular endothelial cell, HUVEC (Sanko Pure Chemicals Co., Ltd.) were scraped off by the method shown in Example 3 and suspended in 2% CD14w/oHS/RPMI and inoculated in wells of a 96-well plate in a density of $5 \times 10^4$ cells/well (50 µL/well), followed by cultivation under the conditions of 37°C and 5% $CO_2$ for 24 hours.

10 µL of 36 µg/mL LPS (E. coli 055:B5, Difco Co.), 50 µL of 7.2 µg/mL recombinant soluble type CD14 (sCD14 (1-356)) with or without 9.6 µg/mL human serum-derived soluble CD14 low molecular weight form polypeptide, and 10 µL of RPMI1640 medium containing 14% human serum from which soluble type CD14 had been removed and 144 µg/mL Cycloheximide (Sigma Co.) were added to HUBEC culture and the mixture was cultured for 18 hours.

Thereafter, 12 µL of MTT labeled reagent (Roche Diagnostics Co.) was added and after additional 4 hours of culture, 120 µL of solubilizing solution (Roche Diagnostics Co.) was added. The obtained mixture was placed in the dark overnight and then absorbance at 600 nm was measured to determine the degree of cytotoxicity.

The results obtained are shown in Fig. 19. sCD14(1-356) alone showed 53% cytotoxic activity under the above conditions, which was completely inhibited by addition of CD14 low molecular weight form polypeptide.

(Example 18)

IL-6 production inhibitory activity of CD14 low molecular weight form

This was performed in the same manner as described in Example 3 except that 40 µL of 900 ng/mL CD14 low molecular weight form was added instead of addition of 40 µL of 900 ng/mL F1024-1-3 antibody.

The results obtained are shown in Fig. 10. The CD14 low molecular weight form had an IL-6 production inhibitory activity of 86.7%.

(Example 19)

IL-6 production inductive activity of CD14 low molecular weight form

This was performed in the same manner as described in Example 11 except that 40 µL of 900 ng/mL CD14 low molecular weight form was added instead of addition of 40 µL of 900 ng/mL CD14 mutant polypeptide.

The results obtained are shown in Table 3. The IL-6 production of CD14 low molecular weight form was at or below the detection limit of measurement (50 pg/mL).

(Example 20)

Tests on inhibition of cytokine production in human TLR4 expression transformant cell line

HEKT4-14 obtained in Example 2(1) was inoculated in wells of a 24-well plate in a density of $0.8 \times 10^5$ cells/well and was stimulated with LPS/sCD14 in the presence or absence of F1024-1-3 or sCD14(1-307)S286C in the same manner as in Example 2(2). After 20 hours, the supernatant of culture was recovered and the amount of produced IL-8 was confirmed by EIA. As a result, both F1024-1-3 and sCD14(1-307)S286C showed suppressive activity, in does-dependent manner(Fig. 20).

(Example 21)

IL-6 production inhibitory activity induced by gram-positive bacterial cell component

Human kidney-derived cell line U-373MG (ATCC) was scraped wit $PBS^-$ containing 0.05% Trypsin and 0.53 mM EDTA, then suspended in RPMI1640 medium (Asahi Techno Glass Co., Ltd.), and inoculated in wells of a 96-well plate in a density of $3 \times 10^4$ cells/well (100 µL/well), followed by culture under the conditions of 37°C and 5% $CO_2$ for

24 hours. Then, 70 μL of RPMI1640 medium, 10 μL of 1 μg/mL (w/v) Staphylococcus aureus cell suspension (Sigma Co.), 10 μL of 5 μg/mL sCD14(1-356) and 10 μL of 10 μg/mL CD14 mutant polypeptide or 10 μL of F1024-1-3 antibody were added to each well. After additional 20 hours of culture, IL-6 in the supernatant of culture was measured by using human IL-6 EIA kit (IL-6 Eli-pair: GIBCO BRL Co.). As a control, sCD14(1-356) was added instead of CD14 mutant polypeptide or F1024-1-3 antibody.

Measurement of IL-6 was performed according to the protocol attached to the human IL-6 EIA kit. That is, 50 μL of the culture supernatant diluted with 1% (w/v) BSA/PBS(-) 4 folds was transferred to an IL-6 antibody immobilized plate and 50 μL of biotinated anti-human IL-6 antibody was added thereto. After 60 minutes of incubation at 37°C, the reaction mixture was discarded and the wells were washed 3 times with 400 μL/well 0.05% (v/v) Tween-20/PBS(-). 100 μL/well of a peroxidase-labeled streptoavidin solution was added to wells and further incubated at 37°C for 20 minutes. After washing, 100 μL/well of a color developing substrate (TMB) was added and allowed to react at room temperature for 15 minutes. Then 100 μL/well of a stop solution (1 M HCl). was added to terminate the reaction. The absorbance at a wavelength of 450 nm was measured and the amount of produced IL-6 in the sample was calculated.

The results obtained are shown in Fig. 21. sCD14(1-307)S286C and F1024-1-3 antibody had IL-6 production inhibitory activity of 48.1% and 55.2%, respectively.

The results indicate that CD14 mutant polypeptide and F1024-1-3 suppress cytokine production induced by gram-positive bacteria cell component.

(Example 22)

Confirmation of cross reactivity of F1024-1-3 antibody

For the purpose of usefulness of F1024-1-3 antibody in a sepsis animal model, cross reactivity of F1024-1-3 antibody with various animal-derived CD14s was studied. First, human sCD14(1-356) was immobilized to wells of a plate (Maxisorp, Nunc) in an amount of 50 ng/well and blocked with 0.5% BSA/PBS. Sera of dog (beagle), monkey (crab-eating macaque, rhesus monkey), rabbit (New Zealand white), human (positive control), and rat (negative control) diluted with PBS were each mixed with 1 μg/mL peroxidase-labeled F1024-1-3 antibody and the mixtures were each added to wells of a plate from which the blocking solution had been removed. The plate was incubated at 37°C for 1 hour and washed with a washing solution 5 times. Thereafter, a tetramethylbenzidine color developing solution containing 0.02% hydrogen peroxide was added to the wells and after 10 minutes of reaction, the reaction was terminated with 0.5 M sulfuric acid. The absorbance of the plate was measured at a wavelength of 450 nm. The results obtained with 4-fold diluted sera are shown in Fig. 22.

As a result, inhibition ratios of binding of F1024-1-3 antibody to human CD14 were 52% for rabbit, 30% for dog, 44% for rhesus monkey, 57% for crab-eating macaque, and 83% for human, so that F1024-1-3 antibody showed cross reactivity with CD14s of rabbit, dog, and monkey.

Next, binding of F1024-1-3 antibody to rabbit CD14 was studied by using a flow cytometric method.

From an ear artery of a male rabbit (New Zealand white, Kitayama Labes) weighing 2.2 kg, 1 mL of rabbit whole blood was collected by using a syringe wetted with heparin. To 100 μL of this was added 10 mL of a Tris/$NH_4Cl$ solution to cause hemolysis and the remaining cell fractions were recovered by centrifugation. The collected cells were blocked with 5% bovine serum/0.1% EDTA/PBS- and centrifuged again to recover cells.

Then, the cells were suspended in 1 mL of 5% bovine serum/0.1% EDTA/PBS- and F1024-1-3 antibody, F1024-1-3 antibody preincubated with 275 ng of human sCD14(1-356), and rat IgG were added each in a final concentration of 1 μg/mL, the mixtures were allowed to react at 4°C for 1 hour. Each cell suspension was washed 3 times with 0.25% bovine serum/PBS- and suspended again in 1000-fold diluted FITC-labeled anti-rat antibody (DAKO) and allowed to react at 4°C for 30 minutes. Again, the cells were washed, FACS analysis was performed by using FACS Calibur (BD Co.), and the intensity of fluorescence of the suspension was measured.

As shown in Fig. 23, rabbit monocytes were stained with F1024-1-3 antibody and this staining was inhibited by pretreatment with human CD14. Further, no staining occurred with control antibody (rat IgG). Therefore, it was confirmed that F1024-1-3 antibody binds to rabbit CD14 on monocytes.

(Example 23)

Effectiveness of F1024-1-3 antibody in an LPS-loaded rabbit sepsis model

For the purpose of confirming effectiveness of F1024-1-3 antibody in a sepsis model, an LPS-loaded rabbit sepsis model was prepared and effectiveness of F1024-1-3 antibody therein was studies by two kinds of protocols of pre-administration and post-administration of antibody.

(1) [Pre-administration effect of F1024-1-3 antibody]

[1] Pre-administration of LPS

The LPS-loaded rabbit sepsis model was prepared by administering 5 μg/kg of LPS (Salmonella minnesota Re595, Sigma Co.) to a New Zealand white rabbit (2.1-2.4 kg, Kitayama Labes) through ear vein after 0 (immediately), 5 and 24 hours, respectively, in conformance with the method of Schimke et al. (Proc. Natl. Acad. Sci. USA, 95:13875, 1998). The protocol for pre-administration group was to administer 2.5 mg/kg of F1024-1-3 antibody through the ear vein after -1 (before 1 hour), 4, and 23 hours, respectively. The control group was administered with saline instead of the antibody.

First, the rabbits were grouped by body weight and 5 rabbits were selected for each group. Preliminary blood collection was performed at -1 hour (before 1 hour of the administration). Then, blood collection was performed after 1, 3, 5, 7, 23, 25, 28 and 48 hours, respectively, from the administration and body weight, number of leukocytes, serum GPT value, serum creatinine value, and serum TNFα value were measured. Note that the number of leukocytes was counted by using Sysmex F-280 (Toa Medical Electronics) and serum GPT value and serum creatinine value were measured by using DRI-CHEM5000 (FUJI FILM).

Further, TNFα was obtained in conformance with the method of David et al. (Journal of Immunological Methods, 68: 167, 1984). That is, L929 cells (ATCC) were scraped with 0.25% trypsin/PBS-, resuspended to $5.5 \times 10^5$ cells/mL, and inoculated to wells of a plate in a density of $5.5 \times 10^4$ cells/well. After culturing overnight under the conditions of 37°C and 5% $CO_2$, the supernatant was discarded, 100 μL of a TNF standard preparation (human TNF, Mochida) or diluted analyte was added, subsequently actinomycin D (Sigma) was added to a final concentration of 5 μg/mL, and the cells were cultured under the conditions of 37°C and 5% $CO_2$ for 20 hours.

The amount of TNF was determined as follows. That is, after removing 100 μL of the supernatant of culture in each well of the plate, adding 10 μL of a WST-1 solution (Dojin Chemical), incubating the mixture at 37°C for 40 to 90 minutes, and then measuring the absorbance at 450 nm. The TNF amount in the serum was indicated as the relative index based on absorbance the standard TNF. Further, the effectiveness a of F1024-1-3 antibody was judged by survival rate after 48 hours.

Summary is shown in Table 4. As a result, the group administered with F1024-1-3 antibody had a survival rate after 48 hours of 100% (5/5) while the group administered with saline had a survival rate of 40% (2/5), which indicates that the survival rate is significantly improved by administration of F1024-1-3 antibody. In addition, as for the value of each parameter after 28 hours, the F1024-1-3 antibody-administered group showed values closer to the pre-values than the saline-administered group.

Furthermore, it has become evident that as shown in Fig. 24, administration of F1024-1-3 antibody suppressed the production amount of TNF in serum and F1024-1-3 antibody suppressed production of inflammatory cytokines in vivo like in vitro.

Table 4 shows improvement in mortality and improvements in various parameters by preadministration of F1024-1-3 antibody in an LPS-loaded rabbit sepsis model.

Table 4

| | Pre-value (n=10) | F1024-1-3 antibody-administered group | Saline-administered group |
|---|---|---|---|
| Survival rate after 48 hours (%) | - | 100% | 40% |
| Parameter after 28 hours | | | |
| Number of leucocytes ($10^3$/$mm^3$) | 6.6 +/- 0.7 | 8.2 +/- 2.7 | 2.1 |
| GPT (U/L) | 27 +/- 8 | 82 +/- 42 | 231 |
| Creatinine (mg/dL) | 0.8 +/- 0.04 | 0.78 +/- 0.04 | 1.4 |
| Body weight after 48 hours (kg) | 2.4 +/- 0.04 | 2.35 +/- 0.02 | 2.07 |

[2] Pre-administration of LTA/PepG

Further, for the purpose of confirming the effect of F1024-1-3 antibody on gram-positive bacteria, tests were conducted in the same manner as in [1] above. Instead of LPS, LTA/PepG was administered and the effect of F1024-1-3 antibody to improve leukocytopenia was studied. That is, F1024-1-3 antibody (2.5 mg/kg) or saline was administered to New Zealand white (2.1 to 2.4 kg, Kitayama Labes), and after 1 hour from administration, LTA (Sigma) and PepG (Fluka) in 160 μg/mL were administered through ear vein.

The time immediately before the administration of LTA/PepG was defined as 0 hour and blood was collected every hour to count the number of leukocytes therein. As a result, as shown in Fig. 25, a decrease in the number of leukocytes was observed in the saline-administered group while no decrease in the number of leukocyte was observed in the F1024-1-3 antibody-administered group. This confirms that F1024-1-3 antibody is effective to leukocytopenia due to LTA/PepG and its effectiveness to sepsis caused by gram-positive bacteria was confirmed.

(2) [Post-administration effect of F1024-1-3 antibody]

(1) According to the method in [1] above, an LPS-loaded rabbit sepsis model was prepared. The protocol for post-administration group was as follows. That is, 2.5 mg/kg of F1024-1-3 antibody was administered through the ear vein after 4 and 23 hours from the administration. The control group was administered with saline instead of the antibody.

First, preliminary blood collection from rabbits grouped in the same manner as above was performed on 5 animals per group at -1 hour. Then, LPS was administered according to the protocol and blood collection was conducted after 1, 3, 5, 7, 24, 26, 28 and 48 hours from the administration, and body weight, number of leukocytes, serum GPT value, and serum creatinine value were measured in the same manner as above.

A summary is shown in Table 5. As a result, the group administered with F1024-1-3 antibody had a survival rate after 48 hours of 100% (5/5) while the group administered with saline had a survival rate of 80% (4/5), which indicates that no significant difference in survival rate was observed between these groups. However, as for various parameters after 28 hours, the F1024-1-3-administered group had values close to normal values. On the other hand, the saline-administered group had extraordinary values indicative of damages of cells. Further, as shown in Fig. 26, the blood creatinine level in serum did not fluctuate greatly during observation of the progress, so that it was confirmed that F1024-1-3 antibody had an effect of suppressing damages of tissue caused by LPS loading.

Table 5 shows improvement of various parameters by post-administration of F1024-1-3 antibody in an LPS-loaded rabbit sepsis model.

Table 5

| Pre-value F1024-1-3 Saline-(n=10) antibody- administered administered group group | | | |
|---|---|---|---|
| Survival rate after 48 hours (%) | - | 100% | 80% |
| Parameter after 28 hours | | | |
| Number of leukocytes ($10^3/mm^3$) | 6.6 +/- 0.4 | 5.3 +/- 0.7 | 1.6 +/- 0.3 |
| GPT (U/L) | 27 +/- 8 | 41 +/- 22 | 22 +/- 13 |
| Creatinine (mg/dL) | 0.8 +/- 0.04 | 0.82 +/- 0.09 | 2.04 +/- 1.07 |
| Body weight after 48 hours (kg) | 2.13 +/- 0.08 | 1.98 +/- 0.1 | 1.92 +/- 0.06 |

(Example 24)

Effectiveness of CD14 mutant polypeptide in mouse lethal LPS-shock model

(1) [Preparation of sCD14(1-356) and sCD14(1-307)S286C for use in vivo tests]

In order to administer sCD14(1-356) and sCD14(1-307)S286C to an animal disease model to confirm their pharmacological effects, 17.6 mg of sCD14(1-356) was prepared from 1.15 liters of the supernatant of culture by the method shown in Example 8, and 11.2 mg of sCD14(1-307)S286C was prepared from 7.8 liters of the supernatant of culture by the method shown in Example 9. The purity of each polypeptide was confirmed by silver staining so that it is of a single band. That is, 100 ng/lane of each polypeptide was electrophoresed on SDS-polyacrylamide gradient gel (5 to 20%, ATTO Co.) and staining of protein with silver was performed by using 2D-silver staining reagent II "Daiichi" kit (Daiichi Pure Chemicals Co., Ltd.). As a result, only the band having a calculated size was stained for each polypeptide.

(2) [Effect of sCD14(1-307)S286C in mouse lethal LPS-shock model]

Balb/c male mice of 6 weeks age (Charles River Japan, Inc.) were grouped into 5 groups (each group consisting of 10 animals) based on body weight. Then, 3 to 10 mg/kg of sCD14(1-307)S286C, 10 mg/kg of sCD14(1-356), and 10 mg/kg of saline (for negative control, Otsuka Pharmaceutical Co., Ltd.) or Solu-Medrol (for positive control, Pharmacia Upjohn Co.) were administered through tail vein as a single dose.

After 2 minutes from the administration of a test drug, 10 μg/kg of LPS (Salmonella minnesota Re595, SIGMA Co.) and 700 mg/kg of galactosamine (D-Galactosamine Hydrochloride, Wako Pure Chemical Industry Co., Ltd.) were administered through tail vein to induce shock. Thereafter, survival rate was judged up to 24 hours with a lapse of time.

The results obtained are shown in Fig. 27. In the physiological saline-administered group, which is a negative control group, all the animals were dead up to 8 hours after the induction of shock. Furthermore, in the group administered with 10 mg/kg of sCD14(1-356), all the animals were dead up to 12 hours after the induction of shock. On the other hand, as for the sCD14(1-307)S286C-administered group, 8 animals in the 10 mg/kg-administered group survived even after 24 hours while survival of 6 animals in the 3 mg/kg-administered groups was confirmed. These results confirm that sCD14(1-307)S286C has a dose-dependent survival rate improving effect.

(Example 25)

Analysis of N-terminal amino acid sequence of CD14 mutant polypeptide

N-terminal amino acid sequence analysis of sCD14(1-307)S286C, which is one of CD14 mutant polypeptides, was practiced by using Protein sequencer Procise™ 494cLC Protein sequencing system (Applied Biosystems Japan Co., Ltd.). As a result, one sequence composed with 5 amino acid residue, i.e., Thr Thr Pro Glu Pro were confirmed as the N-terminal amino acid sequence. From the fact that no other amino acid sequence was confirmed, it can be seen that the N-terminal of sCD14(1-307)S286C is the amino acid at the position 1.

(Example 26)

Expression of sCD14(1-285) using Escherichia coli

From the sCK14 (1-285) expression plasmid pM1659 prepared in Example 8-(2), a coding region of CD14 was cleaved with NcoI and HindIII. Then, the DNA fragment was inserted into plasmid pTrc99A (Amersham Pharmacia Biotech Co.) for expression in Escherichia coli, which has a Trp promoter, by the NcoI and HindIII sites, and JM109 competent cell was transformed with the plasmid by a conventional method to obtain plasmid pTrc1659. By using the obtained plasmid as a template and also using sense primer 27 (SEQ ID NO:72) and antisense primer 35 (SEQ ID NO:73), PCR reaction was performed with Pyrobest DNA Polymerase (TaKaRa Co., Ltd.) by repeating 30 times the cycle consisting of 98°C for 10 seconds, 55°C for 30 seconds, and 72°C for 1 minute. Then the PCR product was digested with XhoI. In addition, pTrc1659 was digested with NcoI and the termini thereof were blunt-ended with Klenow fragment (TaKaRa Co., Ltd.), followed by digestion with XhoI and ligation with the aforementioned PCR product. By a conventional manner, JM109 competent cells were transformed with the ligation product to obtain sCD14(1-285) Escherichia coli expression plasmid pTrc1659-2. JM109 containing pTrc1659-2 was cultured in an LB medium (BIO 101 Co.) containing ampicillin (D[-]-α-Aminobenzylpenicillin; Sigma Co.) in a final concentration of 50 μg/mL until the absorbance thereof at 600 nm reached 1, and then, IPTG (Isopropyl β-D-Thiogalactopyranoside; Sigma Co.) in a final concentration of 1 mM was added to the medium, to perform additional 3 hours of culture. The cells recovered by centrifugation were suspended in TE buffer (Wako Pure Chemical Industry Co., Ltd.) containing 0.5% Triton-X100 (Sigma Co.) and fractured by freeze thawing and ultrasonication, followed by solubilization of CD14 the polypeptide. Measurement of the expression amount of polypeptide by the method shown in Example 8-(5) indicates that the polypeptide was obtained in an expression amount of about 8 μg/mL of culture solution of Escherichia coli.

INDUSTRIAL APPLICABILITY

According to the present invention, there are provided a substance having a function of inhibiting the binding between CD14 and TLR, which is capable of controlling signal transduction into cells even the already-formed CD14/LPS, a method of preparing the same, a method of screening a medicament for treating sepsis, and a pharmaceutical composition for sepsis.

## Claims

1. An anti-CD14 antibody, which specifically recognizes an epitope comprising a part of the region at positions 269 to 315 of human CD14 described in SEQ ID NO:1 and has a function of inhibiting the binding between CD14 and Toll Like Receptor (hereinafter referred to as TLR).

2. An antibody according to claim 1, which is a monoclonal antibody.

3. A fragment of an antibody according to claim 2, which is Fab, Fab' or $(Fab')_2$.

4. F1024-1-3 Monoclonal antibody produced by hybridoma F1024-1-3 (Accession No. P-18061).

5. A hybridoma producing an antibody or fragment of antibody according to any one of claims 2 to 4.

6. A peptide comprising 8 or more consecutive amino acids out of the region at positions 269 to 315 of human CD14 described in SEQ ID No:1.

7. A method of preparing an antibody, comprising using a peptide according to claim 6 as an immunogen.

8. A CD14 mutant polypeptide having a function of inhibiting the binding between CD14 and TLR, and comprising an amino acid sequence selected from the group consisting of [1] and [2]. below.

   [1] having an amino acid sequence corresponding to the amino acid sequence described in SEQ ID NO:1 with its N-terminal being any one of amino acids at positions 1 to 6 thereof and its C-terminal being any one of amino acids at positions 246 to 306 thereof.
   [2] having an amino acid sequence corresponding to the amino acid sequence described in SEQ ID NO:1 with its N-terminal being any one of amino acids at positions 1 to 6 thereof and its C-terminal being any one of amino acids at positions 269 to 356 thereof and with any one or more out of amino acids at positions 269 to 307 thereof being substituted by one or more other amino acids.

9. A CD14 mutant polypeptide according to claim 8, wherein the C-terminal in claim 8 [1] is any one of amino acids at positions 246 to 285 thereof.

**10.** A CD14 mutant polypeptide according to claim 8, wherein the C-terminal in claim 8 [2] is any one of amino acids at positions 269 to 356 thereof and any one or more of amino acids at positions 269 to 307 thereof are substituted by Leu, Ala, Cys or Gly.

**11.** A CD14 mutant polypeptide according to any one of claims 8 to 10, wherein one or more amino acids are deleted, substituted, inserted or added and said polypeptide has a function of inhibiting the binding between CD14 and TLR.

**12.** A gene encoding a CD14 mutant polypeptide according to any one of claims 8 to 11.

**13.** A gene comprising a DNA of [1] or [2] below.

[1] DNA described in any one of SEQ ID NOs:4 to 6,
[2] DNA encoding a polypeptide, wherein said DNA hybridizes with DNA described in any one of SEQ ID NOs: 4 to 6 under stringent conditions, and said polypeptide has a function of inhibiting the binding between CD14 and TLR.

**14.** A recombinant vector containing a gene according to claim 12 or 13.

**15.** A transformant containing a recombinant vector according to claim 14.

**16.** A method of producing a CD14 mutant polypeptide according to any one of claims 8 to 11, **characterized by** comprising the step of culturing a transformant according to claim 15.

**17.** A CD14 low molecular weight form having a molecular weight of 36±5 kDa obtainable from plasma.

**18.** A binding inhibitor for inhibiting the binding between CD14 and TLR, comprising an anti-CD14 antibody, an antibody fragment, a polypeptide or a CD14 low molecular weight form.

**19.** A method of screening a medicament for treating sepsis, comprising the steps of contacting a cell expressing TLR prepared by a genetic engineering method with CD14 and a test substance, detecting a change in a specified index, and judging whether or not the test substance can be a candidate of a medicament for treating sepsis.

**20.** An anti-CD14 antibody, CD14 mutant or low molecular weight compound obtained by a screening method according to claim 18 or 19.

**21.** A pharmaceutical composition for sepsis comprising a binding inhibitor for inhibiting the binding between CD14 and TLR as an active ingredient.

FIG.1

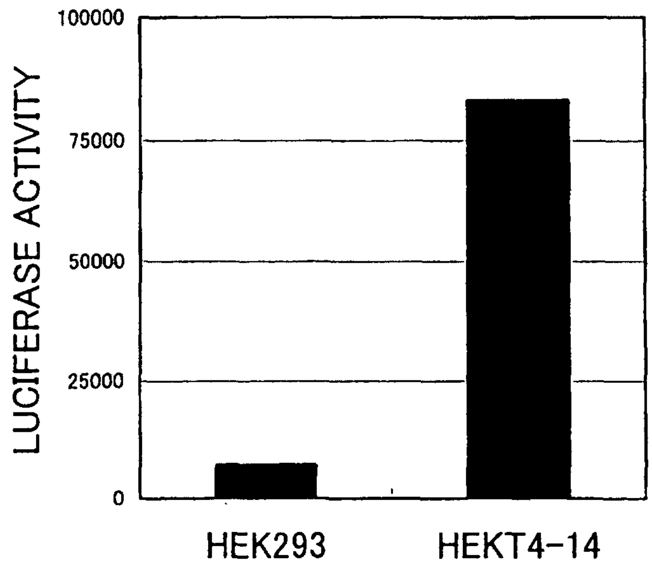
100000
75000
50000
25000
0
LUCIFERASE ACTIVITY
HEK293
HEKT4-14

FIG.2

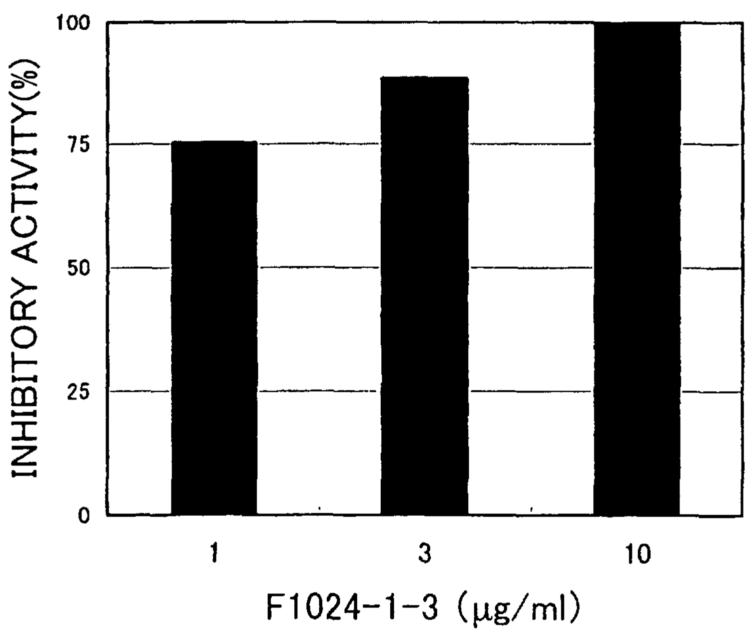
100
75
50
25
0
INHIBITORY ACTIVITY(%)
1
3
10
F1024-1-3 (μg/ml)

FIG. 3

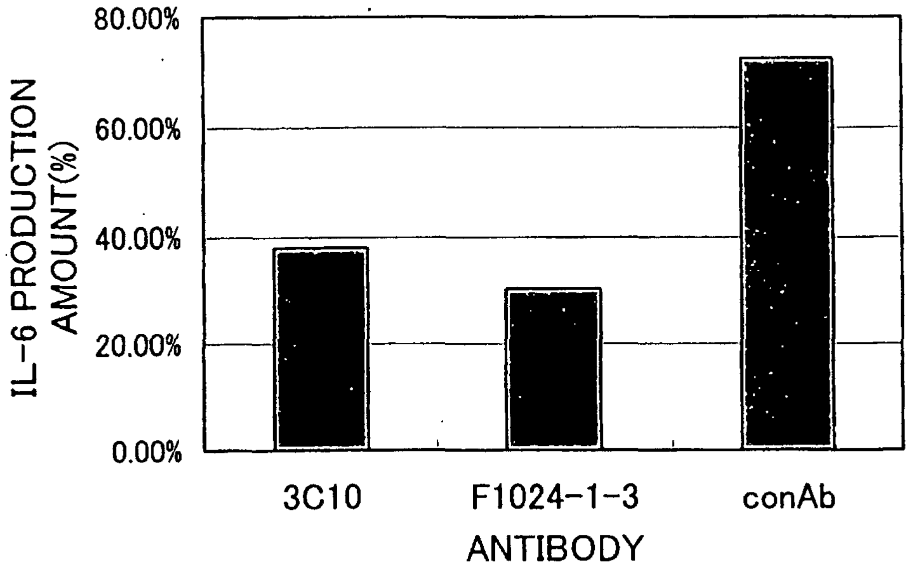
80.00%
60.00%
40.00%
20.00%
0.00%
IL-6 PRODUCTION AMOUNT(%)
3C10
F1024-1-3
conAb
ANTIBODY

FIG. 4

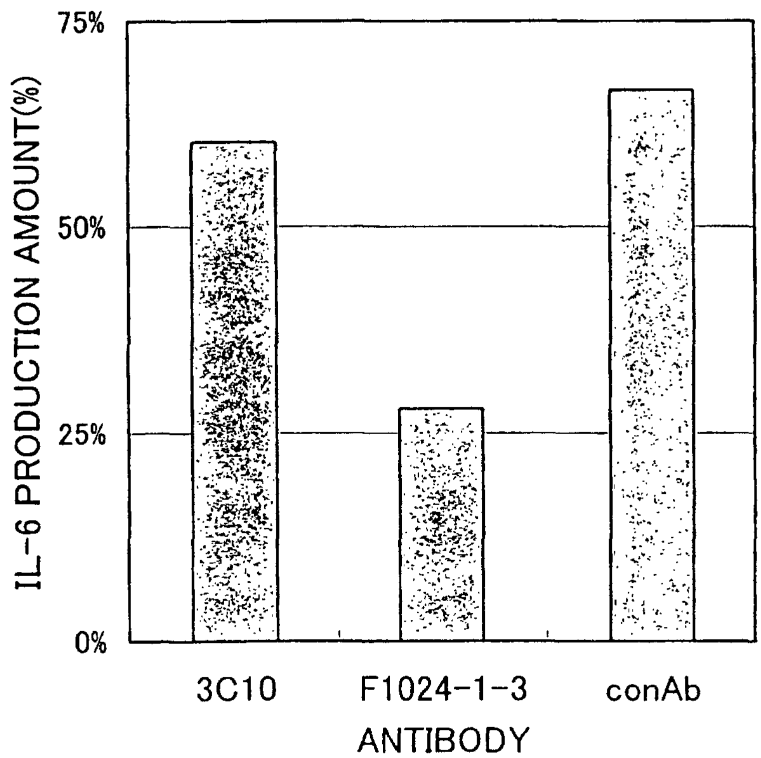
75%
50%
25%
0%
IL-6 PRODUCTION AMOUNT(%)
3C10
F1024-1-3
conAb
ANTIBODY

FIG.5

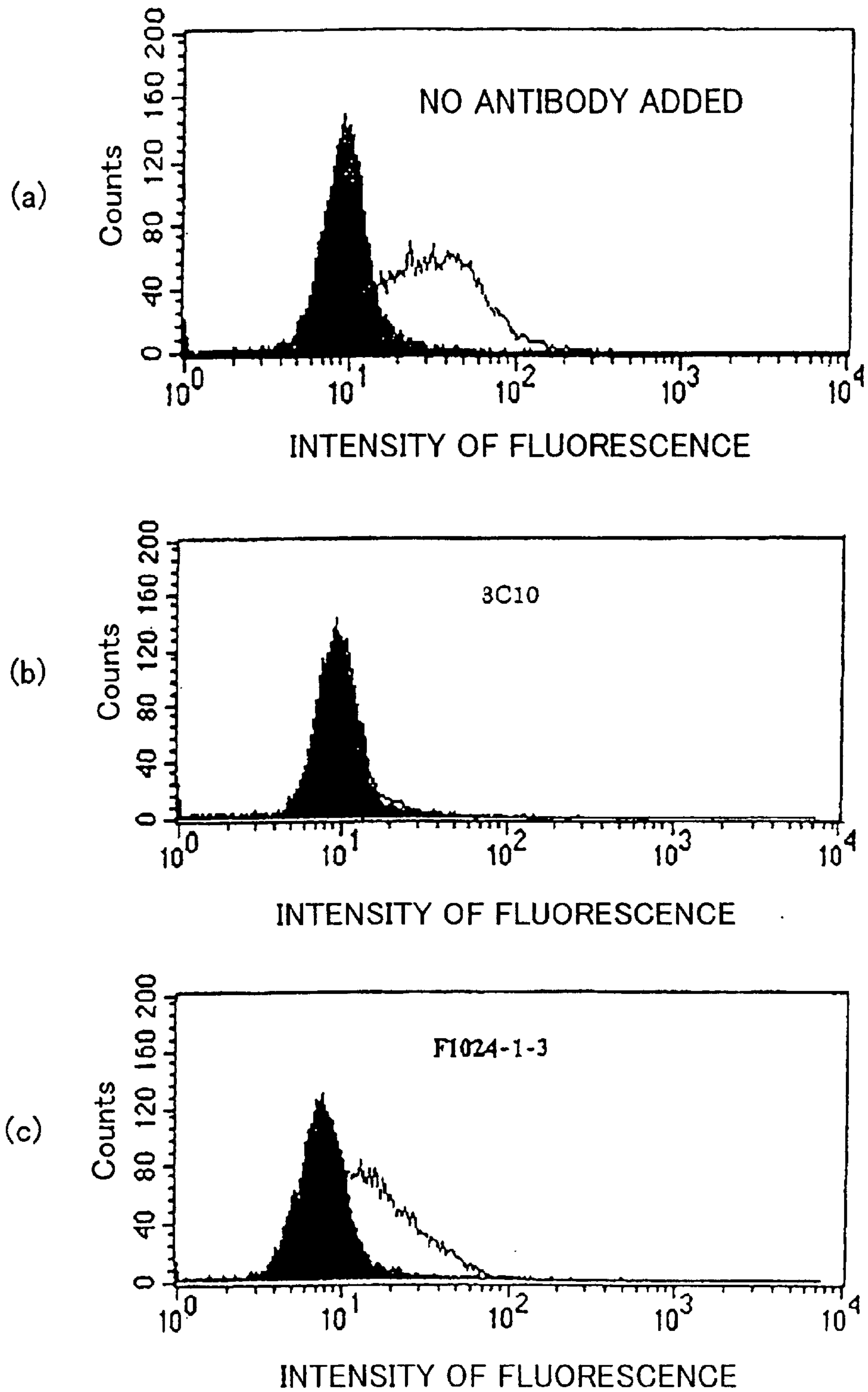
(a)
Counts
0 40 80 120 160 200
NO ANTIBODY ADDED
$10^0$ $10^1$ $10^2$ $10^3$ $10^4$
INTENSITY OF FLUORESCENCE
(b)
Counts
0 40 80 120 160 200
3C10
$10^0$ $10^1$ $10^2$ $10^3$ $10^4$
INTENSITY OF FLUORESCENCE
(c)
Counts
0 40 80 120 160 200
F1024-1-3
$10^0$ $10^1$ $10^2$ $10^3$ $10^4$
INTENSITY OF FLUORESCENCE

# FIG.6

| PEPTIDE | INHIBITORY ACTIVITY |
|---|---|
| A | + |
| B | + |
| C | − |
| D | − |
| E (CONTROL) | − |

283 301 307 315 318 329 331 343

LDLSCNRLNRAPQPDELPEVDNLTLDGNPFLVPGTALPHEGSMNSGVVPACARSTLSVGVSGTL

A B C D

# FIG.7

| sCD14 DELETION TAILORED MUTANT | | | BINDING ACTIVITY | | |
|---|---|---|---|---|---|
| TYPE | POSITION OF DELETION | FEATURE | F1024-1-3 | 3C10 | MEM-18 |
| 1-356 | − | − | + | + | + |
| 1-328 | C-TERMINUS | − | + | + | + |
| 1-315 | C-TERMINUS | − | + | + | + |
| 1-307 | C-TERMINUS | − | (+) | + | + |
| 1-285 | C-TERMINUS | − | − | + | + |
| 1-246 | C-TERMINUS | − | − | + | + |
| 1-183 | C-TERMINUS | − | − | + | + |
| 1-152 | C-TERMINUS | − | − | + | + |
| 1-307 | Δ7-11 | 3C10 BINDING REGION | + | − | + |
| 1-307 | Δ57-64 | MEM-18 BINDING REGION | + | + | − |
| 1-307 | Δ180-234 | − | + | + | + |
| 1-307 | Δ235-282 | − | + | + | + |
| 1-307 | Δ180-282 | − | + | + | + |

# FIG.8

| sCD14 AMINO ACID SUBSTITUTION TAILORED MUTANT | | BINDING ACTIVITY | | |
|---|---|---|---|---|
| TYPE | POSITION OF SUBSTITUTION | F1024-1-3 | 3C10 | MEM-18 |
| 1-307 | − | + | + | + |
| 1-307 | D284A | + | + | + |
| 1-307 | S286A | + | + | + |
| 1-307 | S286K | + | + | + |
| 1-307 | C287A | + | + | + |
| 1-307 | R289A | + | + | + |
| 1-307 | P294A | − | + | + |

FIG.9

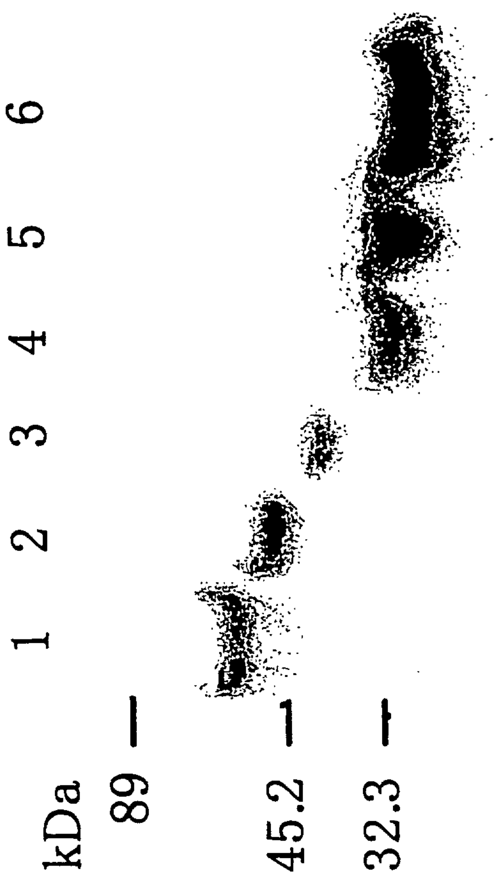

FIG. 10

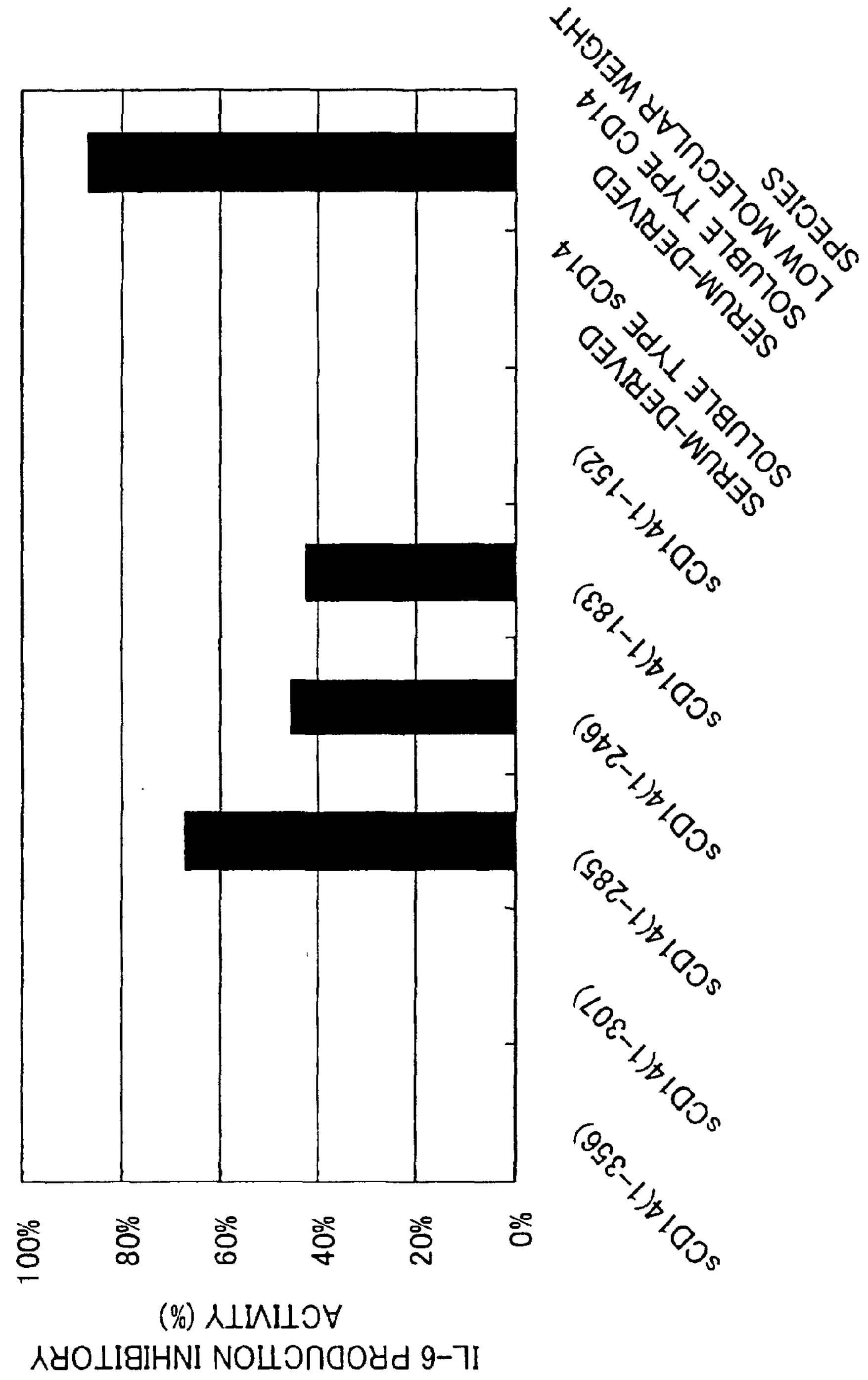
IL-6 PRODUCTION INHIBITORY ACTIVITY (%)
100%
80%
60%
40%
20%
0%
sCD14(1-356)
sCD14(1-307)
sCD14(1-285)
sCD14(1-246)
sCD14(1-183)
sCD14(1-152)
SERUM-DERVED SOLUBLE TYPE sCD14
SERUM-DERVED SOLUBLE TYPE CD14 LOW MOLECULAR WEIGHT SPECIES

FIG.11

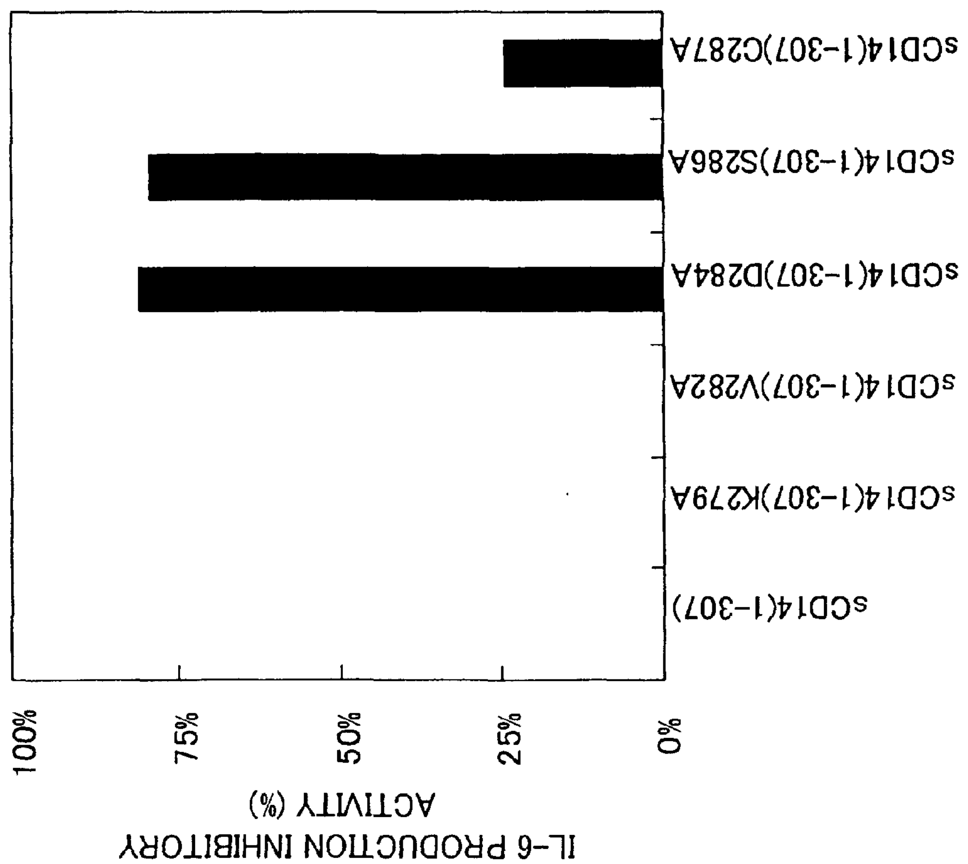
sCD14(1-307)
sCD14(1-307)K279A
sCD14(1-307)V282A
sCD14(1-307)D284A
sCD14(1-307)S286A
sCD14(1-307)C287A
100%
75%
50%
25%
0%
IL-6 PRODUCTION INHIBITORY ACTIVITY (%)

FIG.12

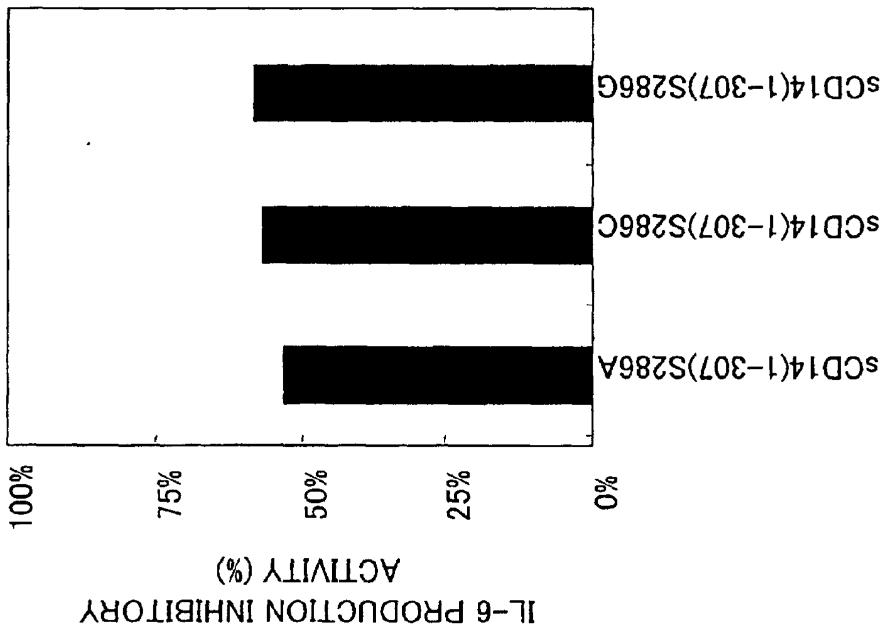
sCD14(1-307)S286G
sCD14(1-307)S286C
sCD14(1-307)S286A
100%
75%
50%
25%
0%
IL-6 PRODUCTION INHIBITORY ACTIVITY (%)

FIG.13

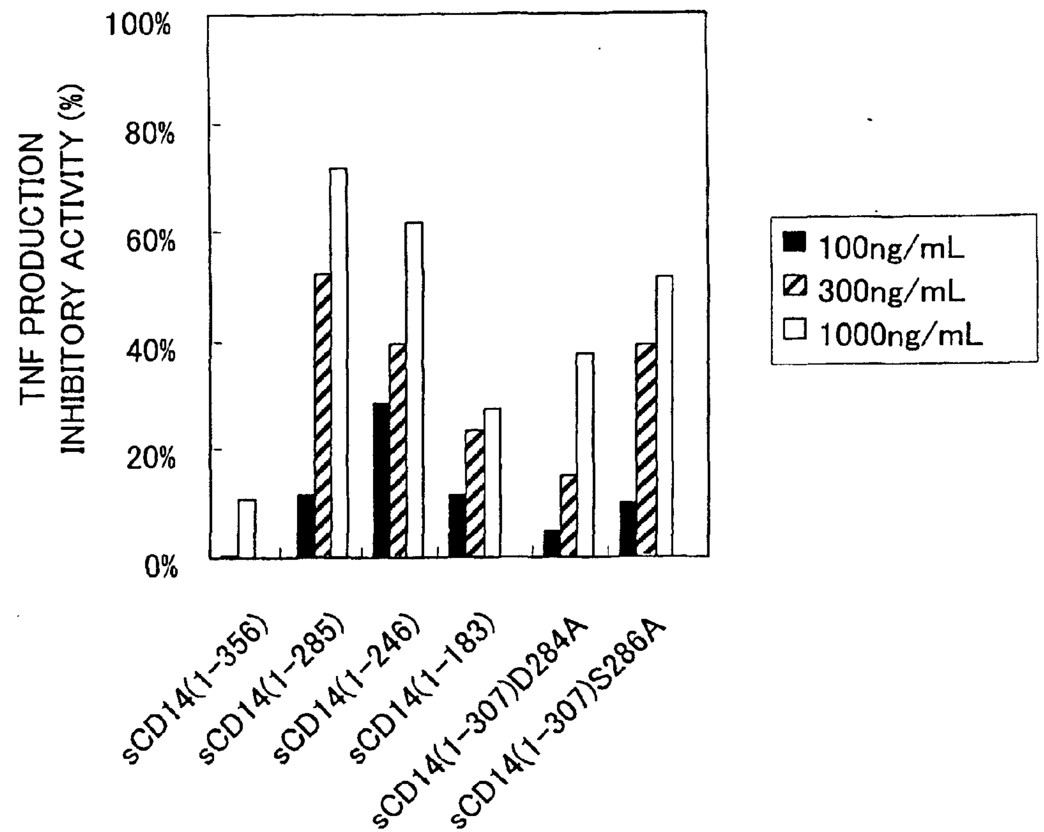
TNF PRODUCTION
INHIBITORY ACTIVITY (%)
100%
80%
60%
40%
20%
0%
sCD14(1-356)
sCD14(1-285)
sCD14(1-246)
sCD14(1-183)
sCD14(1-307)D284A
sCD14(1-307)S286A
100ng/mL
300ng/mL
1000ng/mL

FIG.14

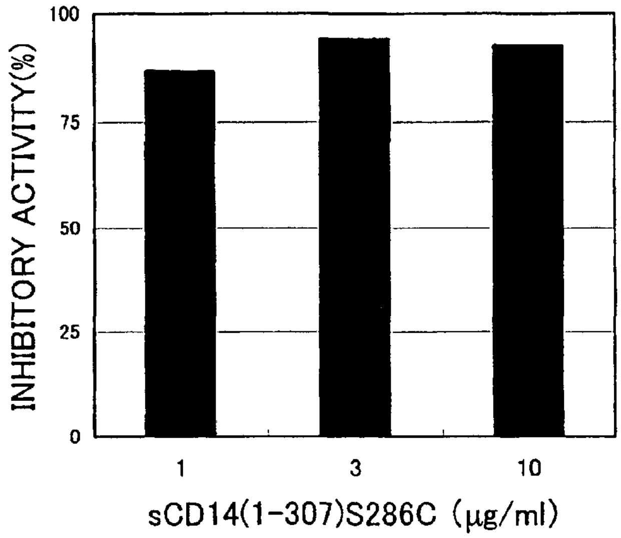
INHIBITORY ACTIVITY(%)
100
75
50
25
0
1
3
10
sCD14(1-307)S286C (μg/ml)

FIG.15

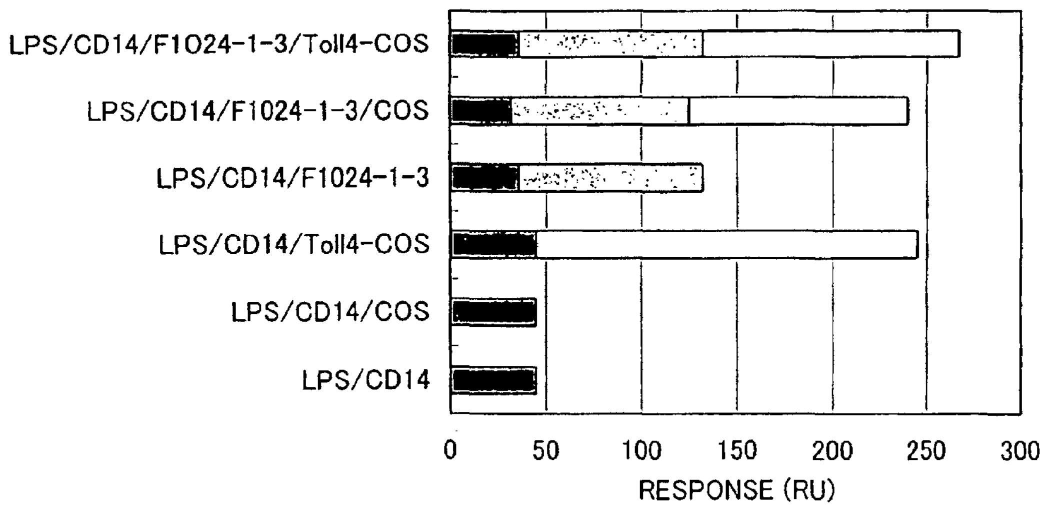
LPS/CD14/F1024-1-3/Toll4-COS
LPS/CD14/F1024-1-3/COS
LPS/CD14/F1024-1-3
LPS/CD14/Toll4-COS
LPS/CD14/COS
LPS/CD14
0
50
100
150
200
250
300
RESPONSE (RU)

FIG.16

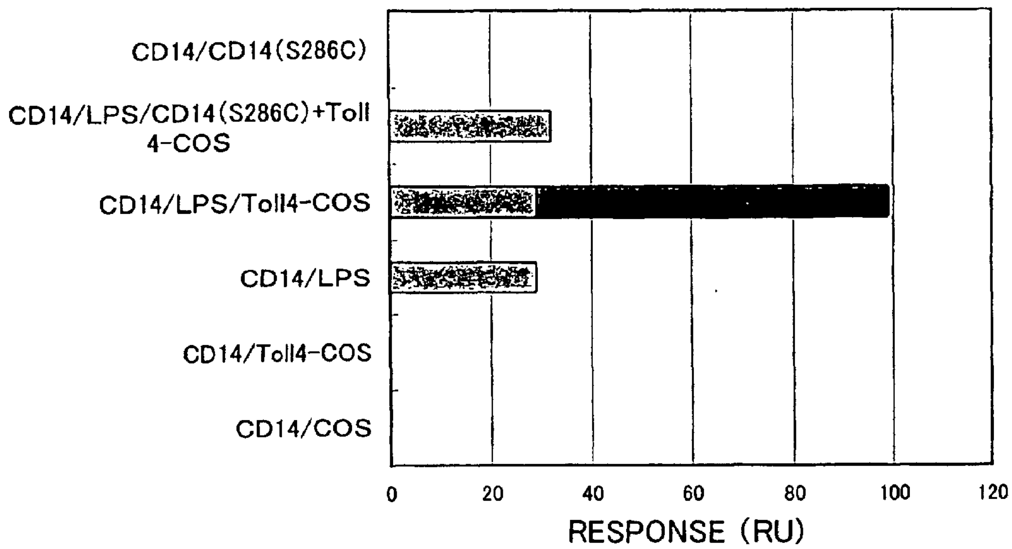


* CD14(S286C) STANDS FOR CD14(1-307) (S286C).

FIG.17

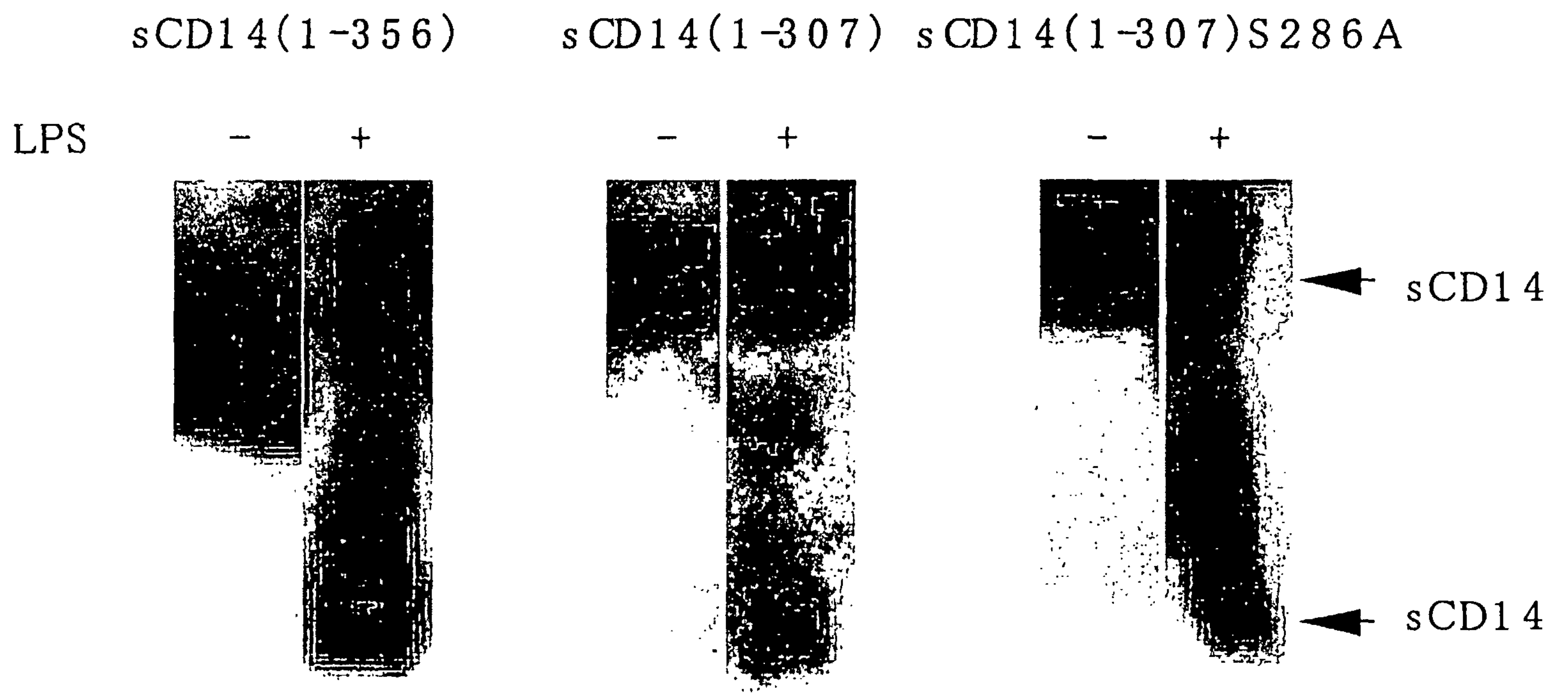

FIG.18

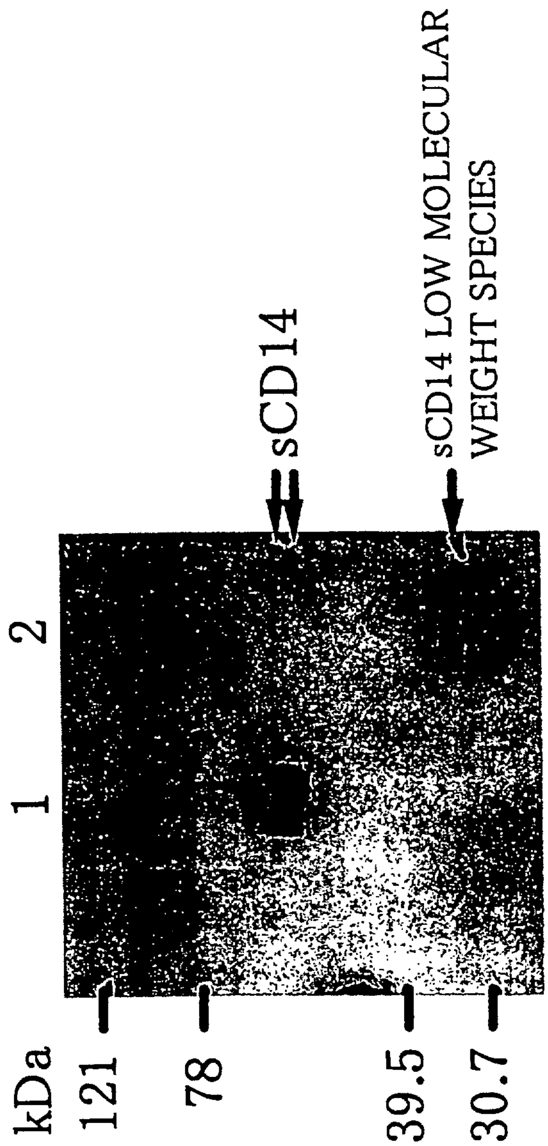
1
2
kDa
121
78
39.5
30.7
sCD14
sCD14 LOW MOLECULAR
WEIGHT SPECIES

FIG.19

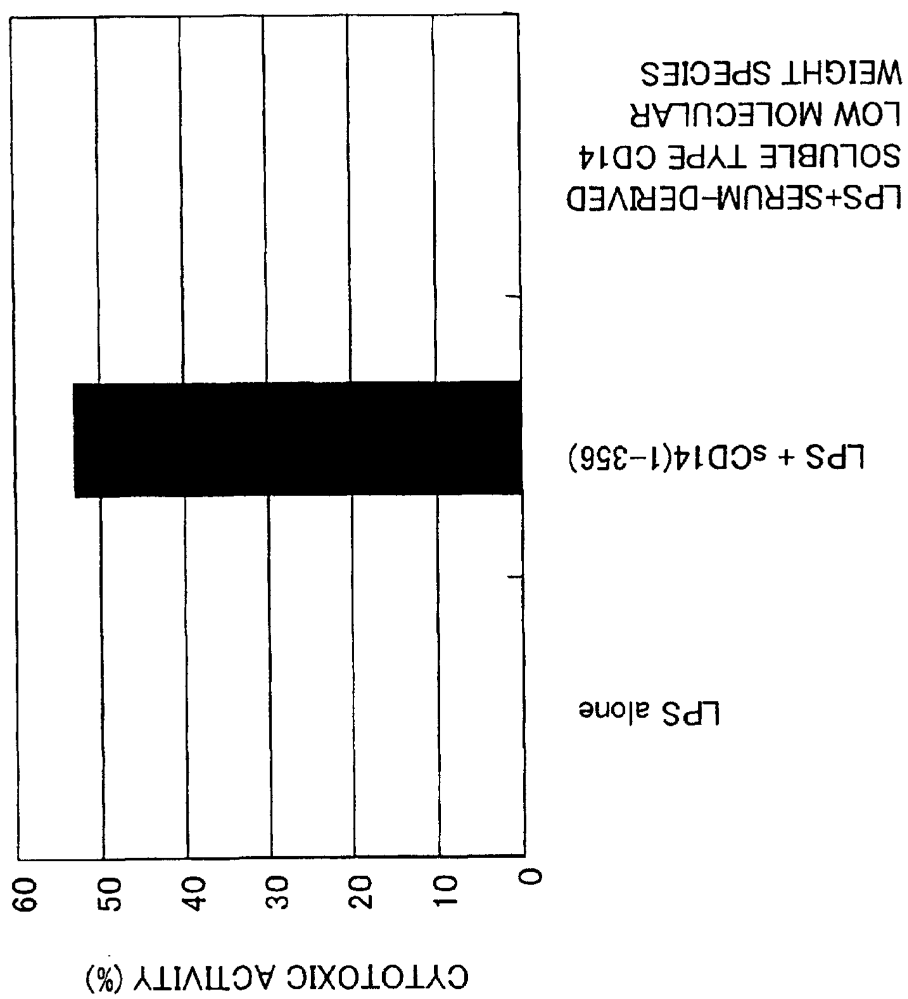
CYTOTOXIC ACTIVITY (%)
0
10
20
30
40
50
60
LPS alone
LPS + sCD14(1-356)
LPS+SERUM-DERIVED
SOLUBLE TYPE CD14
LOW MOLECULAR
WEIGHT SPECIES

FIG.20

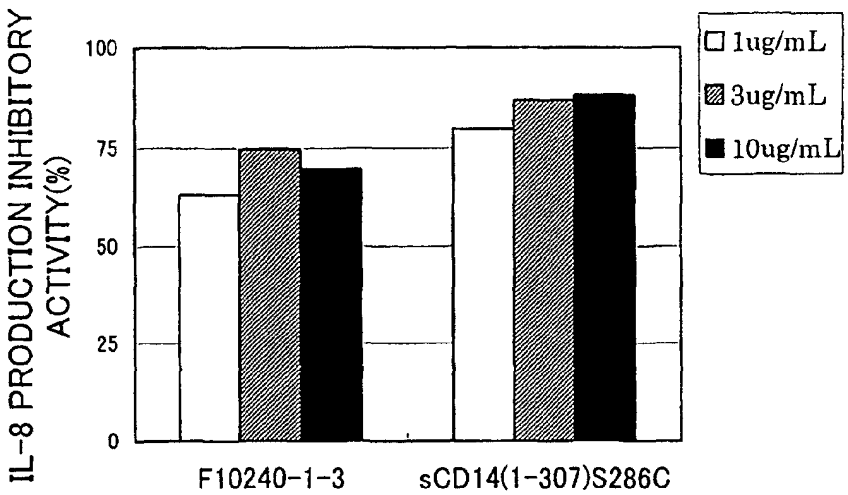
IL-8 PRODUCTION INHIBITORY ACTIVITY(%)
100
75
50
25
0
F10240-1-3
sCD14(1-307)S286C
1ug/mL
3ug/mL
10ug/mL

FIG.21

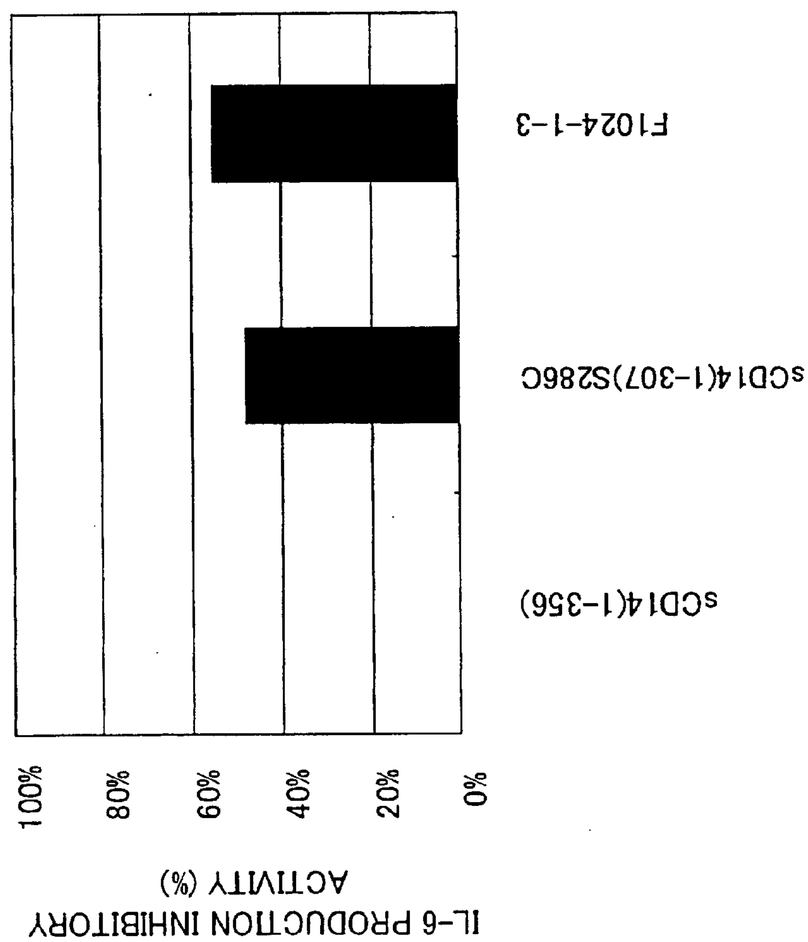
IL-6 PRODUCTION INHIBITORY ACTIVITY (%)
100%
80%
60%
40%
20%
0%
sCD14(1-356)
sCD14(1-307)S286C
F1024-1-3

FIG.22

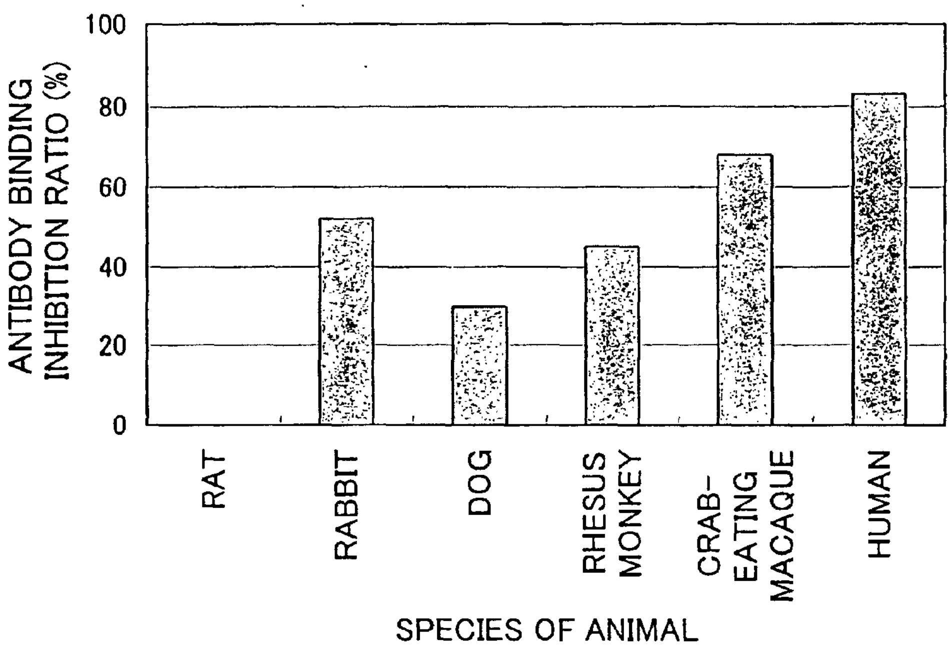
ANTIBODY BINDING INHIBITION RATIO (%)
100
80
60
40
20
0
RAT
RABBIT
DOG
RHESUS MONKEY
CRAB-EATING MACAQUE
HUMAN
SPECIES OF ANIMAL

FIG.23

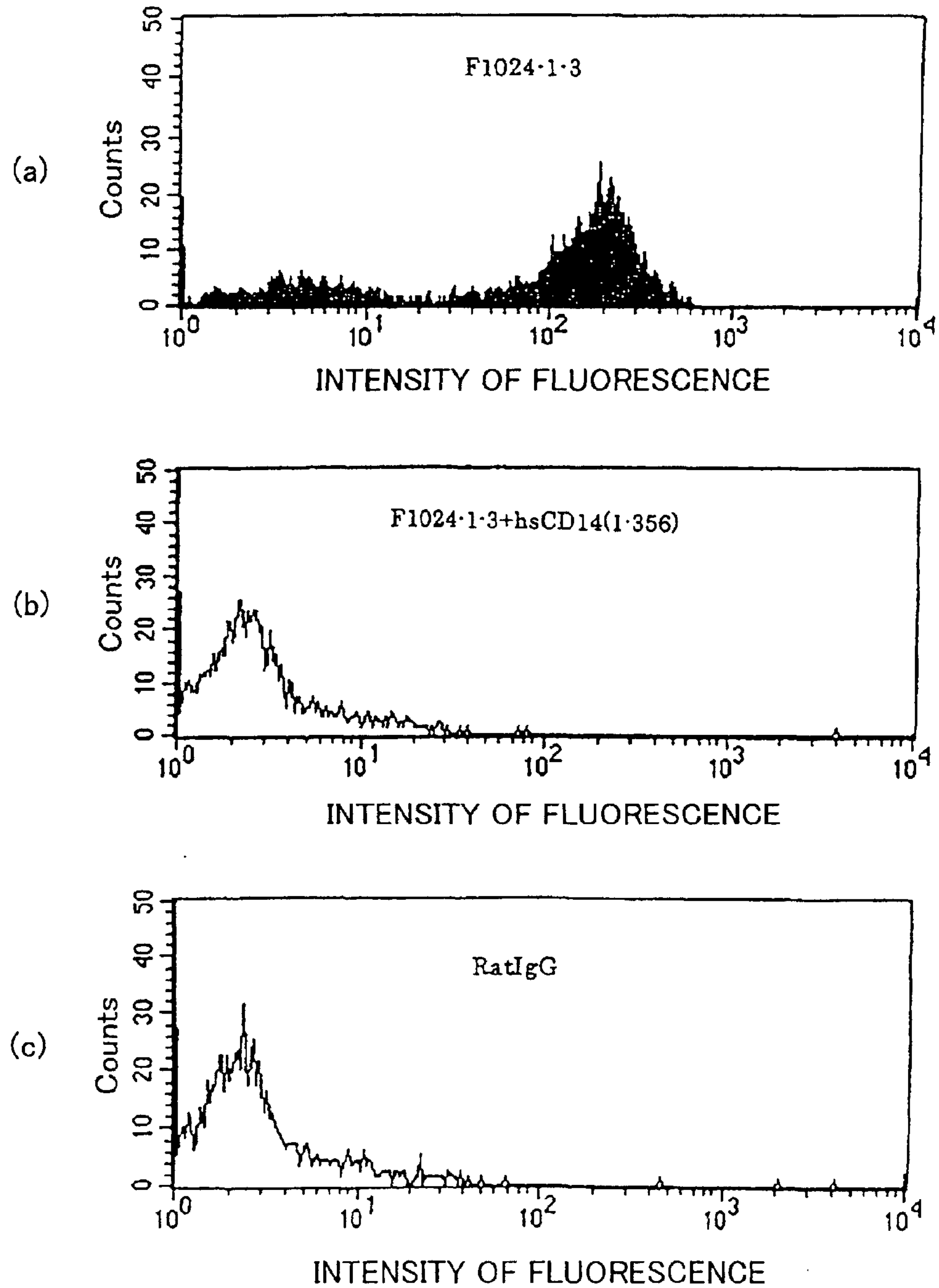
(a)
F1024·1·3
Counts
INTENSITY OF FLUORESCENCE
(b)
F1024·1·3+hsCD14(1·356)
Counts
INTENSITY OF FLUORESCENCE
(c)
RatIgG
Counts
INTENSITY OF FLUORESCENCE

FIG.24

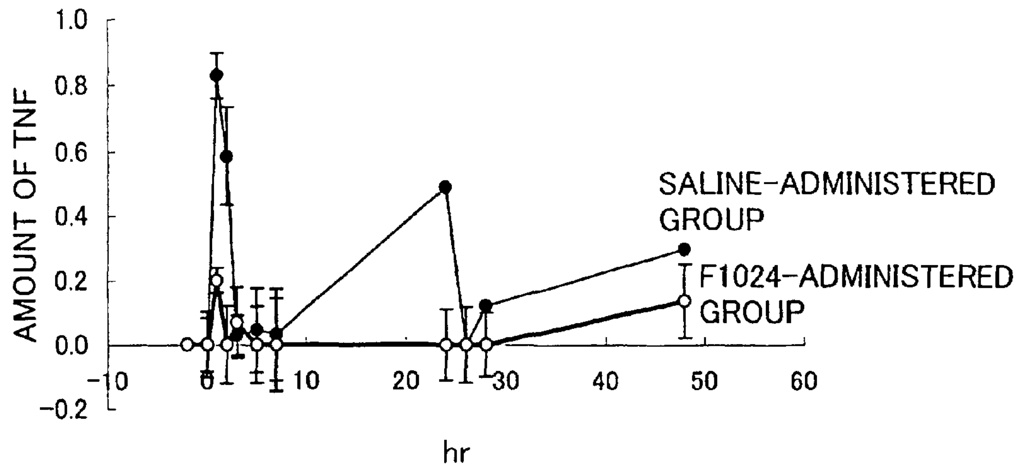
1.0
0.8
0.6
0.4
0.2
0.0
-0.2
AMOUNT OF TNF
-10
0
10
20
30
40
50
60
hr
SALINE-ADMINISTERED GROUP
F1024-ADMINISTERED GROUP

FIG.25

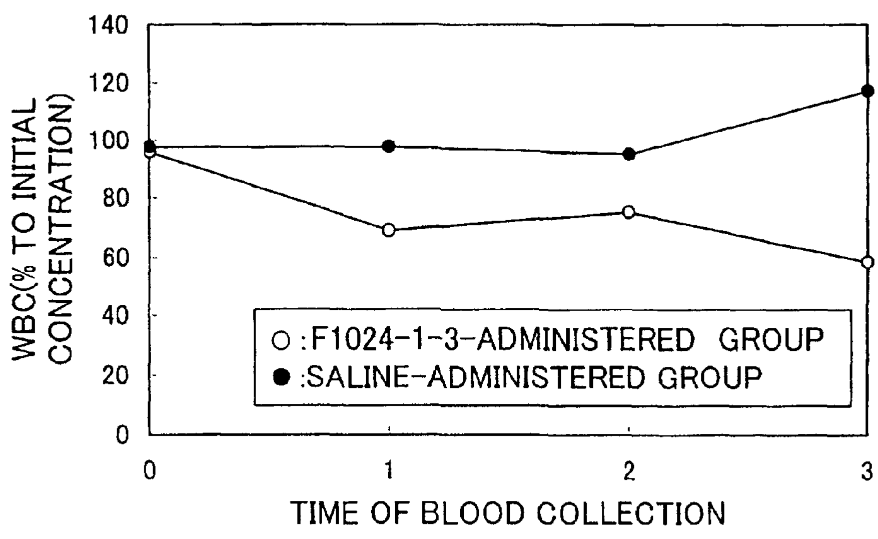
140
120
100
80
60
40
20
0
WBC(% TO INITIAL CONCENTRATION)
0
1
2
3
TIME OF BLOOD COLLECTION
○ :F1024-1-3-ADMINISTERED GROUP
● :SALINE-ADMINISTERED GROUP

FIG.26

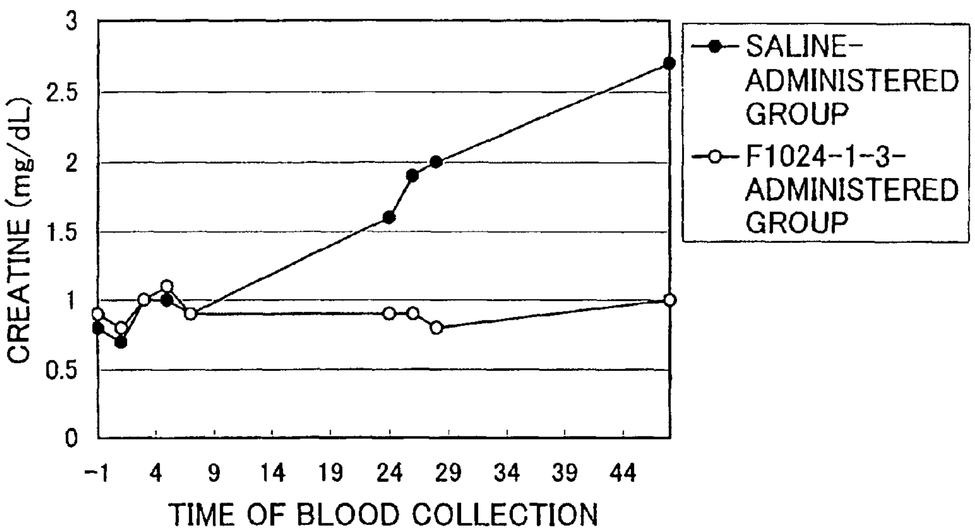
3
2.5
2
1.5
1
0.5
0
CREATINE (mg/dL)
-1
4
9
14
19
24
29
34
39
44
TIME OF BLOOD COLLECTION
SALINE-ADMINISTERED GROUP
F1024-1-3-ADMINISTERED GROUP

FIG.27

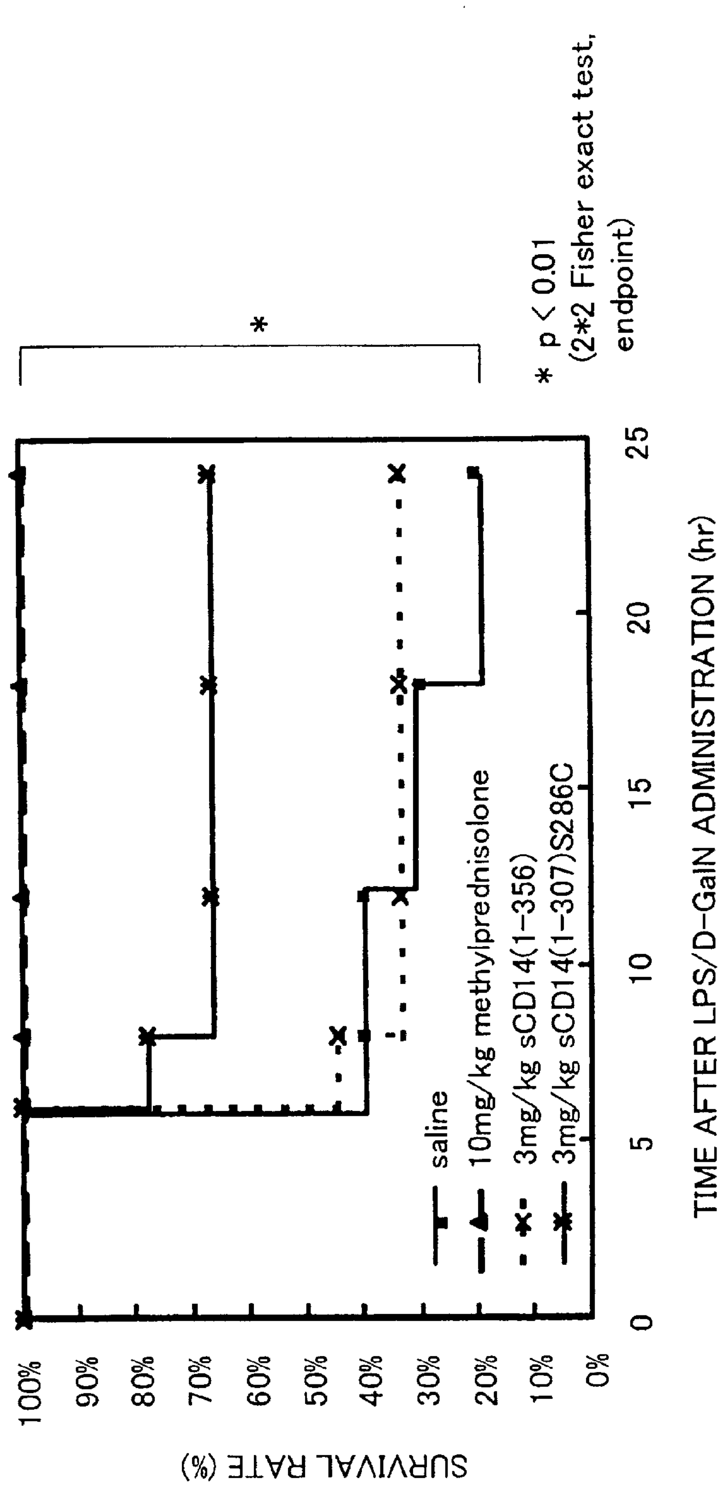
SURVIVAL RATE (%)
100%
90%
80%
70%
60%
50%
40%
30%
20%
10%
0%
0
5
10
15
20
25
TIME AFTER LPS/D-GalN ADMINISTRATION (hr)
saline
10mg/kg methylprednisolone
3mg/kg sCD14(1-356)
3mg/kg sCD14(1-307)S286C
*
* p < 0.01
(2*2 Fisher exact test,
endpoint)

## INTERNATIONAL SEARCH REPORT

International application No.
PCT/JP01/02869

A. CLASSIFICATION OF SUBJECT MATTER
Int.Cl[7] C12N15/00, C07K7/08, 14/705, 16/28, A61K45/00, A61P31/04, 43/00, G01N33/15, 33/50, 33/53, 33/577, A61K38/02, 39/395,

According to International Patent Classification (IPC) or to both national classification and IPC

B. FIELDS SEARCHED

Minimum documentation searched (classification system followed by classification symbols)
Int.Cl[7] C12N15/00, C07K7/08, 14/705, 16/28, A61K45/00, A61P31/04, 43/00, G01N33/15, 33/50, 33/53, 33/577, A61K38/02, 39/395,

Documentation searched other than minimum documentation to the extent that such documents are included in the fields searched

Electronic data base consulted during the international search (name of data base and, where practicable, search terms used)
CA (STN), REGISTRY (STN), MEDLINE (STN),
Genbank/EMBL/DDBJ/PIR/SwissProt/Genseq,
WPI (DIALOG), BIOSIS (DIALOG)

C. DOCUMENTS CONSIDERED TO BE RELEVANT

| Category* | Citation of document, with indication, where appropriate, of the relevant passages | Relevant to claim No. |
|---|---|---|
| P,A | JP, 2000-86699, A (Seikagauk Corporation), 28 March, 2000 (28.03.00), Claims; working examples, etc. & CA, 2264509, A & US, 6245897, B | 1-7,18-19,21 |
| P,A | Shunji Sugawara et al., "Proteolysis of Human Monocyte CD14 Cysteine Proteinases (Gingipains) from Porphyromonas gingivalis Leading to Lipopolysaccharide Hyporesponsiveness", J. Immunol., Vol.1651(1), pages 411 to 418, (2000) | 1-7 |
| A | JP, 10-505839, A (The Scripps Research Institute), Claims; working examples, etc. & WO, 98/2311, A1 & EP, 956957, A | 1-7,18-19,21 |
| A | Trude H. Flo et al., "Human Toll-Like Receptor 2 Mediates Monocyte Activation by Listeria monocytegenes, But Not by Group B Streptococci or Lipopolysaccharide", J. Immunol., Vol.164(4), pages 2064 to 2069, (2000) | 1-7 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 June, 2001 (26.06.01) | 10 July, 2001 (10.07.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/02869

C (Continuation). DOCUMENTS CONSIDERED TO BE RELEVANT

| Category* | Citation of document, with indication, where appropriate, of the relevant passages | Relevant to claim No. |
|---|---|---|
| A | WO, 99/61468, A1 (Gemma Biotechnology Ltd.), Claims; working examples, etc. & EP, 1080111, A2 & AU, 9940258, A & BR, 9910725, A | 8-11 |
| A | US, 5705398, A (The Scripps Research Institute), 06 June, 1999 (06.06.99), Claims, etc. (Family: none) | 6,8-13, 18-19,21 |
| A | US, 5543303, A (Sanna M. Goyert), 06 August, 1996 (06.08.96), Claims, etc. (Family: none) | 6,8-19,21 |

Form PCT/ISA/210 (continuation of second sheet) (July 1992)

**INTERNATIONAL SEARCH REPORT**

International application No.
PCT/JP01/02869

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 20
because they relate to parts of the international application that do not comply with the prescribed requirements to such an extent that no meaningful international search can be carried out, specifically:

Even though the contents of the description are taken into consideration, it is completely unclear what particular inhibitors or compounds are involved and what inhibitors or compounds are not involved in the scope of "anti-CD14 antibody, a CD14 modification or a low-molecular weight compound obtained by a screening method" as set forth in the above claim. Thus, the above claim is described in an extremely unclear manner. Such being the case, no meaningful international search can be practiced on this claim.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)